# EUROPEAN PATENT APPLICATION

(11) **EP 4 803 528 A1**
(43) Date of publication of application: **09.09.2026**
(21) Application number: 25305299.7
(22) Date of filing: 06.03.2025
(51) Int. Cl.: C07H 15/26, C07H 15/222, A61P 7/00, A61P 11/00, A61P 21/00, A61K 31/7008

(54) **AMINOGLYCOSIDE-RELATED READTHROUGH COMPOUNDS AND USES THEREOF**

(71) Applicant: Urania Therapeutics, 67540 Ostwald (FR)
(72) Inventor: The designation of the inventor has not yet been filed
(74) Representative: Plasseraud IP

(57) **Abstract**

The present disclosure relates to a compound of formula (I), which are aminoglycoside-related compounds. The present disclosure also relates to pharmaceutical compositions containing the same. Such compounds are useful in the treatment of genetic disorders associated with a premature stop codon mutation and/or protein truncation phenotype.

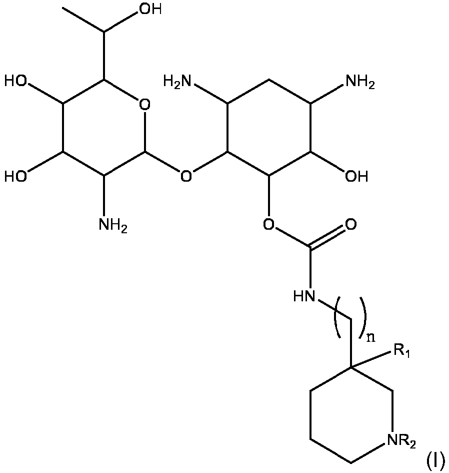

## Description

### Technical field

The present disclosure relates to a compound of formula (I), which are aminoglycoside-related compounds. The present disclosure also relates to pharmaceutical compositions containing the same. Such compounds are useful in the treatment of genetic disorders associated with a premature stop codon mutation and/or protein truncation phenotype.

### Background

Many human genetic disorders result from nonsense mutations, where one of the three stop codons (UAA, UAG or UGA) replaces an amino acid-coding codon, leading to premature termination of the translation and eventually to truncated inactive proteins.

Currently, hundreds of such nonsense mutations are known, and several were shown to account for certain cases of fatal diseases, including, for example, cystic fibrosis (CF), Duchenne muscular dystrophy (DMD), ataxia-telangiectasia, Hurler syndrome, hemophilia A, hemophilia B, Tay-Sachs, Rett Syndrome, Usher syndrome, severe epidermolysis bullosa and more. For many of those diseases there is presently no effective treatment.

Some aminoglycoside compounds have been shown to have therapeutic value in the treatment of several genetic diseases because of their ability to induce ribosomes to readthrough premature stop codons generated by nonsense mutations, partially restoring the synthesis of full-length, functional proteins.

Because aminoglycosides are too toxic to be used on the long term for the treatment of monogenic diseases caused by nonsense mutations, there is a need to develop non-aminoglycoside compounds with the same mechanism of action but non-toxic and displaying improved readthrough properties.

### Summary

The inventors surprisingly found that compounds of formula (I) exhibit valuable pharmaceutical properties. In particular, they are active in readthrough of premature stop codons, and therefore, can be useful in the treatment of genetic disorders associated with a premature stop codon mutation and/or protein truncation phenotype, as shown in the examples.

The presently disclosed aminoglycoside-related compounds are characterized by a core structure based on 3 rings, in which ring 3 is a piperidine. In particular, ring 3 has been carefully chosen using structure-based design to obtain compounds having improved readthrough activity. The fact that the commonly present saccharide ring 3 is replaced by piperidine makes it possible to obtain compounds having low toxicity in mammalian cells and low antimicrobial activity, as well as improved bioavailability and/or cell permeability, and/or improved readthrough activity.

Consequently, in a first aspect, the present disclosure relates to a compound of formula (I): 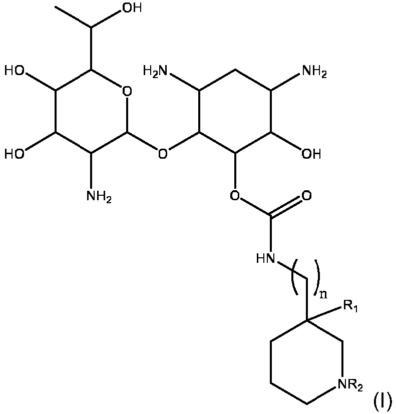 wherein
R1 is H or an alkyl
R2 is selected from alkyl, heteroalkyl, cycloalkyl, heterocyclyl, aryl, heteroaryl and any combinations thereof,
n is an integer from 1 to 3, preferably n is 1 or 2
R', R" and R‴ are independently selected from H and alkyl,
   and pharmaceutically acceptable salts thereof.

In another aspect, the disclosure relates to a pharmaceutical composition comprising a compound of the present disclosure and a pharmaceutically acceptable carrier.

In another aspect, the disclosure relates to a compound of the present disclosure for use as a drug.

In another aspect, the disclosure relates to a compound of the present disclosure for use in treating genetic disorders associated with a premature stop codon mutation and/or protein truncation phenotype.

### Detailed description

### Definitions

As used herein, the terms "alkyl", by itself or as part of another substituent, refer to a linear or branched alkyl functional group having 1 to 12 carbon atoms, more preferably 1 to 6 carbon atoms. Suitable alkyl groups include methyl, ethyl, n-propyl, i-propyl, n-butyl, i-butyl, s-butyl and t-butyl, pentyl and its isomers (e.g. n-pentyl, iso-pentyl), and hexyl and its isomers (e.g. n-hexyl, iso-hexyl).

As used herein, the terms "cycloalkyl" refer to a saturated or unsaturated cyclic group having 3 to 12 carbon atoms, preferably 3 to 6. The cycloalkyl can have a single ring or multiple rings fused together. The cycloalkyl can also include spirocyclic rings. Suitable cycloalkyl groups include cyclopropyl, cyclobutyl, cyclopentyl and cyclohexyl.

As used herein, the term "halogen" refers to a fluoro (-F), chloro (-CI), bromo (-Br), or iodo (-I) group.

As used herein, the terms "haloalkyl" refer to a C₁-C₆ alkyl as defined herein that is substituted by one or more halogen group as defined herein. Suitable C₁-C₆ haloalkyl groups include trifluoromethyl and dichloromethyl.

As used herein, the terms "heteroalkyl", refer to a straight or branched hydrocarbon chain consisting of 1 to 20 carbon atoms, preferably 1 to 12 carbon atoms, and more preferably 1 to 6 carbon atoms ,and at least one heteroatom, preferably from one to three, heteroatoms selected from the group consisting of O, N, Si and S (preferably from O, N and S°, and wherein the nitrogen and sulfur atoms may optionally be oxidized (for example: a sulfoxide or a sulfone) and the nitrogen heteroatom may optionally be quaternized. The heteroatom(s) O, N and S may be placed at any interior position of the heteroalkyl group or at the position at which the alkyl group is attached to the remainder of the molecule.

As used herein, the terms "alkoxy" refer to a -O-alkyl group, wherein the alkyl group is a C₁-C₆ alkyl as defined herein. Suitable C₁-C₆ alkoxy groups include methoxy, ethoxy, propoxy.

As used herein, the terms "haloalkoxy" refer to a C₁-C₆ alkoxy group as defined herein, that is substituted by one or more halogen group as defined herein. Suitable haloalkoxy include trifluoromethoxy.

As used herein, the terms "alkylene", used alone or as part of another substituent, refer to a divalent saturated, straight-chained or branched hydrocarbon group having 1 to 12 carbon atoms, preferably 1 to 6.

As used herein, the terms "heteroalkylene", refer to a divalent heteroalkyl as defined above. For heteroalkylene groups, heteroatoms can also occupy either or both of the chain termini.

As used herein, the terms "aryl " refer to a polyunsaturated, aromatic hydrocarbyl group having a single ring or multiple aromatic rings fused together, containing 6 to 10 ring atoms, wherein at least one ring is aromatic. The aromatic ring may optionally include one to two additional rings (cycloalkyl, heterocyclyl or heteroaryl as defined herein) fused thereto. Suitable aryl groups include phenyl, naphtyl and phenyl ring fused to a heterocyclyl, like benzopyranyl, benzodioxolyl, benzodioxanyl and the like.

As used herein, the terms "heteroaryl" refer to a polyunsaturated, aromatic ring system having a single ring or multiple aromatic rings fused together or linked covalently, containing 5 to 10 atoms, wherein at least one ring is aromatic and at least one ring atom is a heteroatom selected from N, O and S. The nitrogen and sulfur heteroatoms may optionally be oxidized and the nitrogen heteroatoms may optionally be quaternized. Such rings may be fused to an aryl, cycloalkyl or heterocyclyl ring. Non-limiting examples of such heteroaryl, include: furanyl, thiophenyl, pyrrolyl, pyrazolyl, imidazolyl, oxazolyl, isoxazolyl, thiazolyl, isothiazolyl, triazolyl, oxadiazolyl, thiadiazolyl, tetrazolyl, oxatriazolyl, thiatriazolyl, pyridinyl, pyrimidyl, pyrazinyl, pyridazinyl, oxazinyl, dioxinyl, thiazinyl, triazinyl, indolyl, isoindolyl, benzofuranyl, isobenzofuranyl, benzothiophenyl, isobenzothiophenyl, indazolyl, benzimidazolyl, benzoxazolyl, purinyl, benzothiadiazolyl, quinolinyl, isoquinolinyl, cinnolinyl, quinazolinyl and quinoxalinyl.

As used herein, the terms "heterocyclyl" refer to a saturated or unsaturated cyclic group having 3 to 10 ring atoms, wherein at least one ring atom is a heteroatom selected from N, O and S. The nitrogen and sulfur heteroatoms may optionally be oxidized and the nitrogen heteroatoms may optionally be quaternized. The heterocycle can include fused or bridged rings as well as spirocyclic rings. Examples of heterocycle include, but are not limited to, tetrahydropyridyl, piperidinyl, morpholinyl, tetrahydrofuranyl, tetrahydrothienyl, piperazinyl, 1-azepanyl, imidazolinyl, 1,4-dioxanyl and the like.

In some embodiments, the substituents can be optionally substituted. As used herein, the terms "optionally substituted" can refer to groups that can be substituted with one or more of the substituents independently selected from: C₁₋C₂₀ alkyl, C₁₋C₂₀ heteroalkyl, aryl having 6 to 10 ring atoms, C₃-C₈ cycloalkyl, heterocycloalkyl having 3 to 10 ring atoms, heteroaryl having 5 to 10 ring atoms, C₁₋C₂₀ haloalkyl,-X, -R', -O⁻, -OR', =O, -SR', -S⁻, -NR'₂, -NR'₃, =NR', -CX₃, -CN, -OCN, -SCN, -NCS, -NO, -NO₂, =N₂, -NRC(=O)R', -C(=O)R', -C(=O)NR'₂, -SO₃⁻, -SO₃H, -S(=O)₂R', -OS(=O)₂OR', -S(=O)₂NR', -S(=O)R', -C(=O)R', -C(=S)R', -CO₂R', , - C(=S)OR', C(=O)SR', C(=S)SR', C(=O)NR'₂, C(=S)NR'₂, and C(=NR')NR'₂, where each X is independently a halogen: -F, -Cl, -Br, or -I; and each R' is independently -H, -C₁-C₂₀ alkyl, -C₆-C₁₀ aryl, C₃-C₈ cycloalkyl, heterocycloalkyl having 3 to 10 ring atoms, or heteroaryl having 5 to 10 ring atoms.

Various embodiments of the disclosure are described herein. It will be recognized that features specified in each embodiment may be combined with other specified features to provide further embodiments.

The present disclosure encompasses the compounds of the present disclosure, their tautomers, enantiomers, diastereomers, racemates or mixtures thereof, and their hydrates, esters, solvates or pharmaceutically acceptable salts.

The terms "pharmaceutically acceptable salts" refers to salts that retain the biological effectiveness and properties of the compounds of this disclosure and, which typically are not biologically or otherwise undesirable. In many cases, the compounds of the disclosure are capable of forming acidic and/or basic salts due to the presence of amino and/or carboxyl groups or similar groups. Pharmaceutically acceptable acid addition salts can be formed with organic acids and/or inorganic acids. Pharmaceutically acceptable base addition salts can be formed with organic bases and/or inorganic bases.

In many cases, the compounds of the disclosure are capable of forming esters by virtue of the presence of carboxyl groups. Esters include C₁-C₆ alkyl esters.

Any formula given herein is also intended to represent unlabeled as well as isotopically labeled forms of the compounds, like deuterium labeled compounds or ¹⁴C-labeled compounds.

As used herein, the term "treating" includes reversing, alleviating, inhibiting the progression of, preventing or reducing the likelihood of the disease, disorder, or condition to which such term applies, or one or more symptoms or manifestations of such disease, disorder or condition. The term "preventing" refers to stopping a disease, disorder, condition, or symptom or manifestation of such, or worsening of the severity of such, from occurring. Accordingly, the presently disclosed compounds can be administered prophylactically to prevent or reduce the incidence or recurrence of the disease, disorder, or condition.

As used herein, the terms "therapeutically efficient amount" of a compound refer to an amount of the compound that will elicit the biological or medical response of a subject, for example, ameliorate the symptoms, alleviate conditions, slow or delay disease progression, or prevent a disease.

### Compound of formula (I)

The present disclosure first relates to a compound of formula (I) 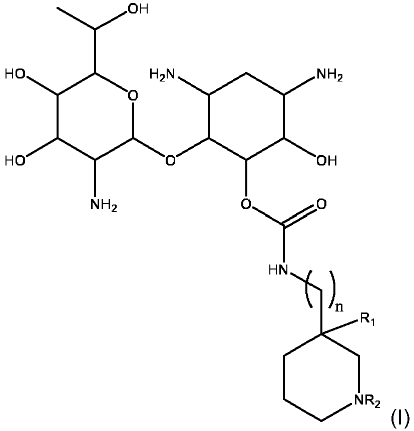 Wherein
R1 is H or an alkyl
R2 is selected from alkyl, heteroalkyl, cycloalkyl, heterocyclyl, aryl, heteroaryl and any combinations thereof, n is an integer from 1 to 3, preferably n is 1 or 2
R', R" and R‴ are independently selected from H and alkyl,
and pharmaceutically acceptable salts thereof.

According to an embodiment, the compound is of formula (II) 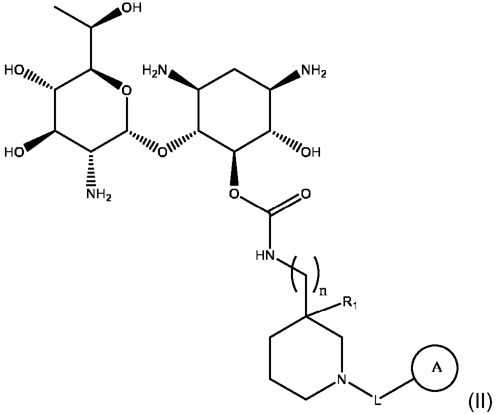 Wherein
L is a bond or an alkylene,
R1 is H or CH₃,
Ring A is selected from cycloalkyl, and heterocyclyl.
According to an embodiment, the compound is of formula (III) 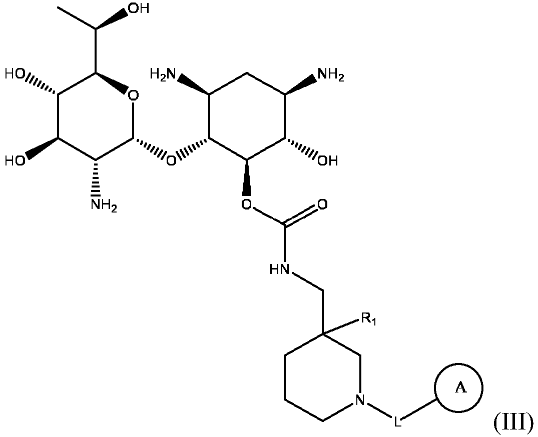 According to an embodiment, R2 is -R4-NH-R5,
R4 is an alkylene,
R5 is selected from H, alkyl, heteroalkyl, cycloalkyl, heterocyclyl, aryl, heteroaryl and any combinations thereof.
According to an embodiment, the compound is selected from 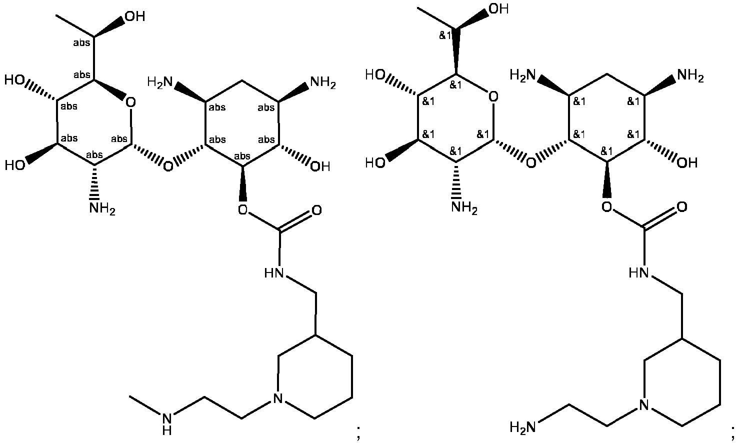 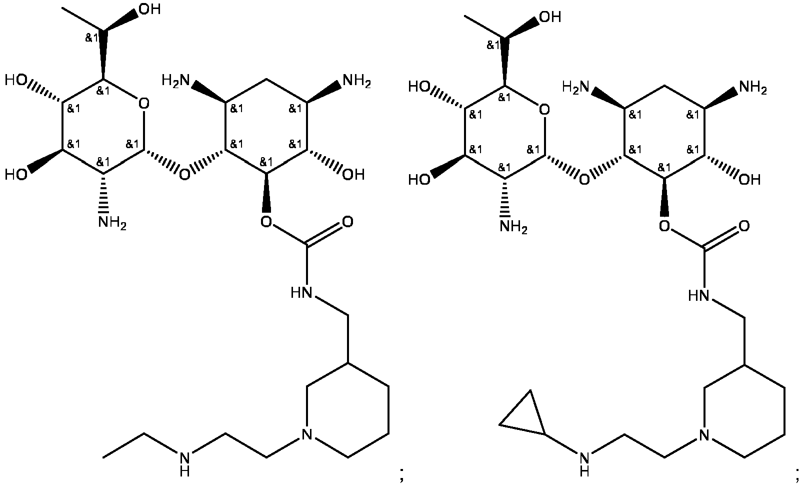 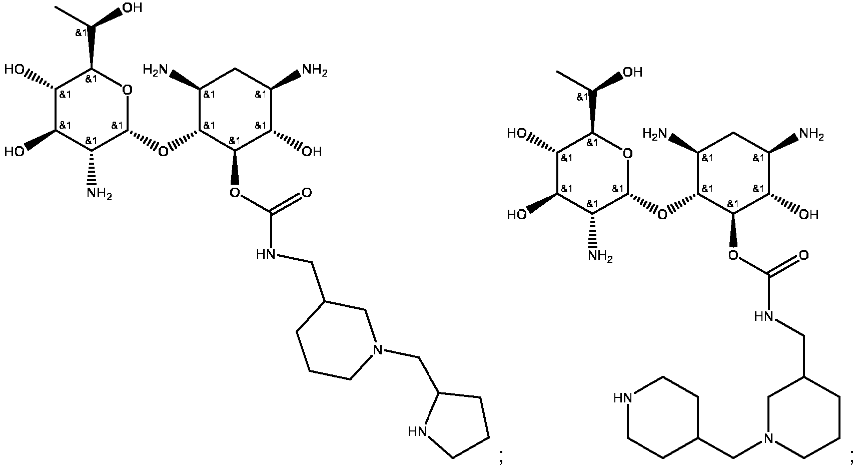 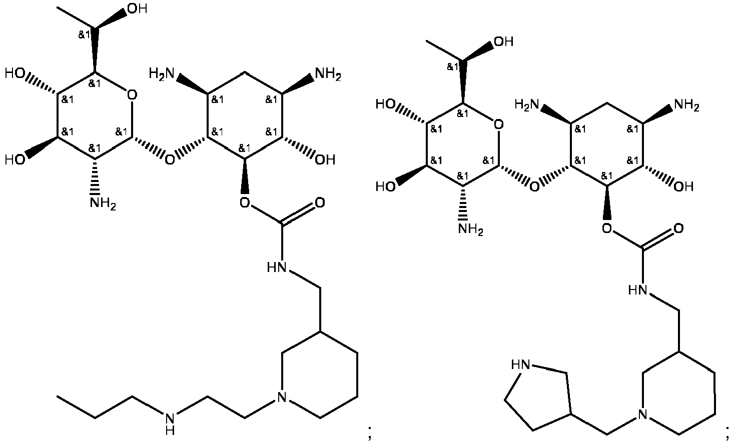 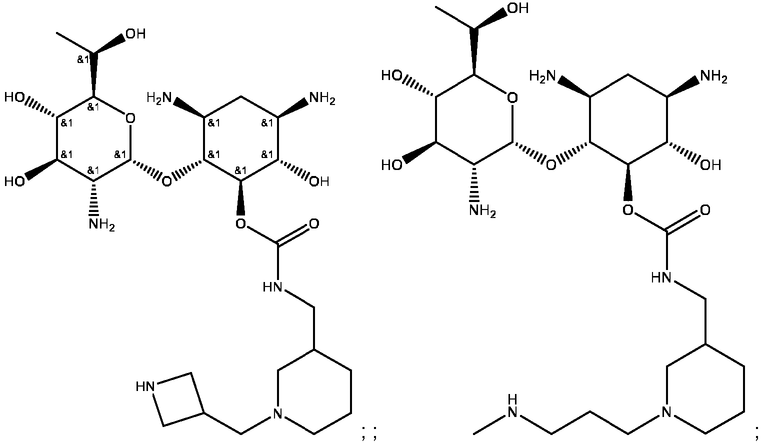 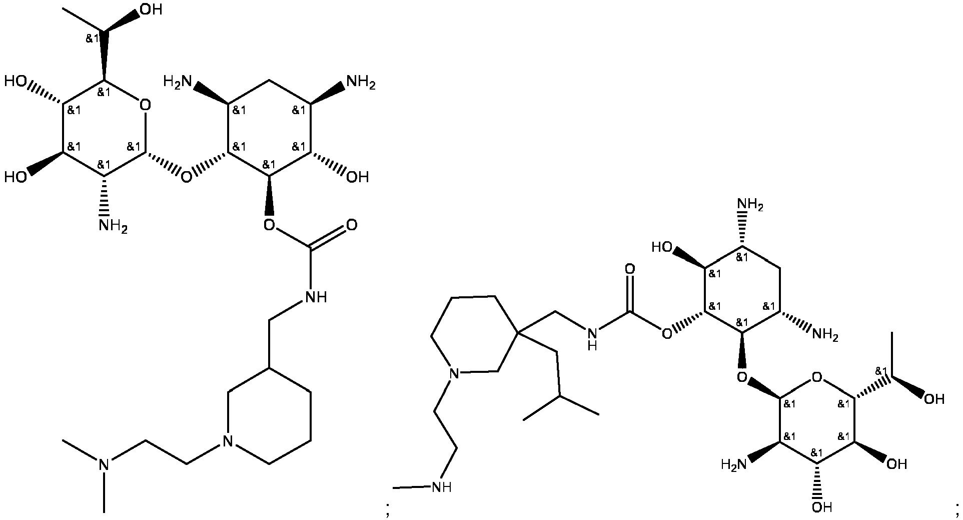 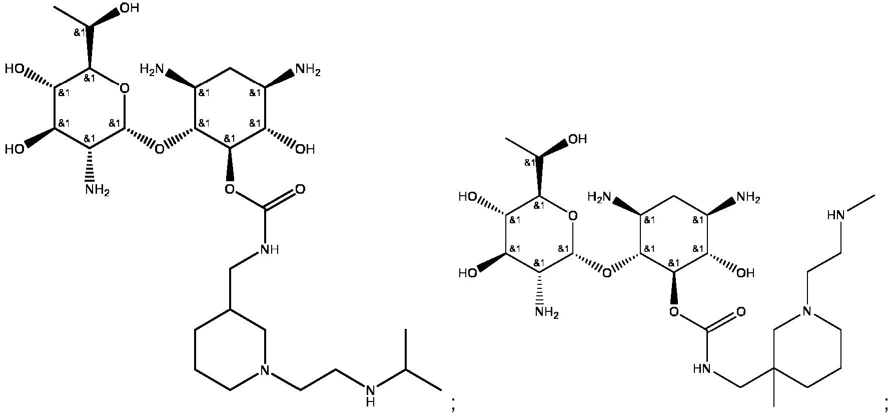 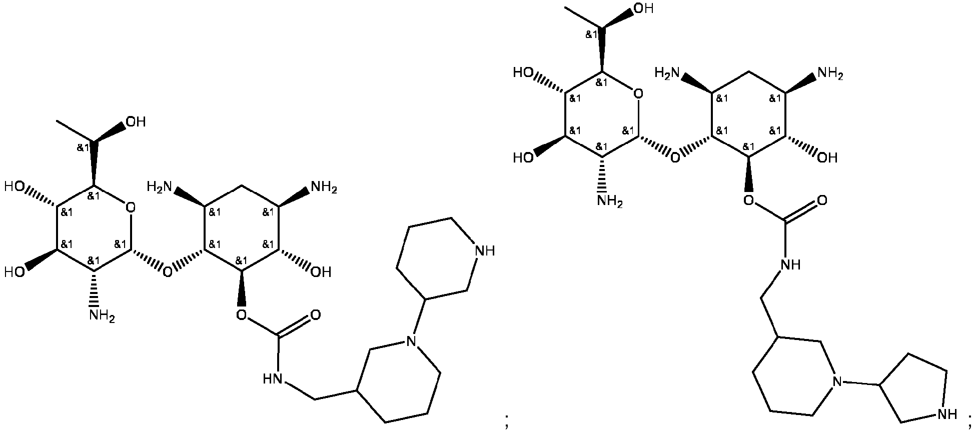 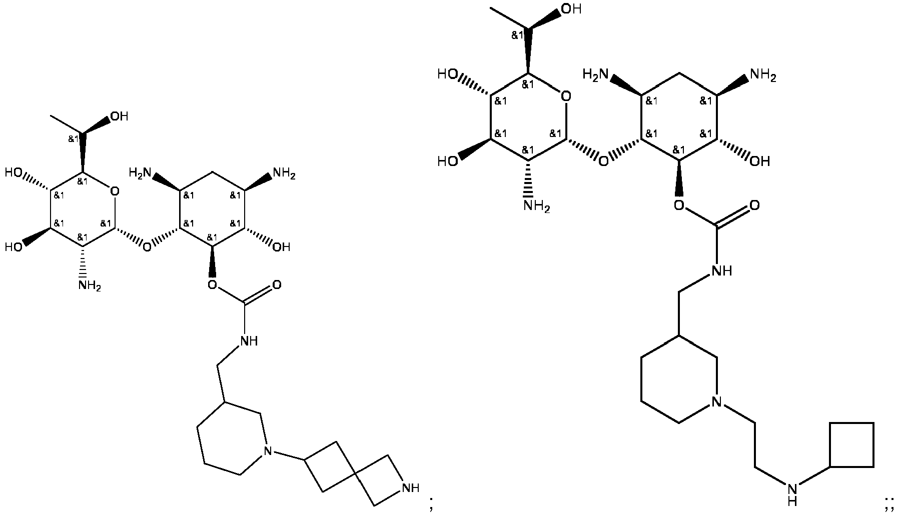 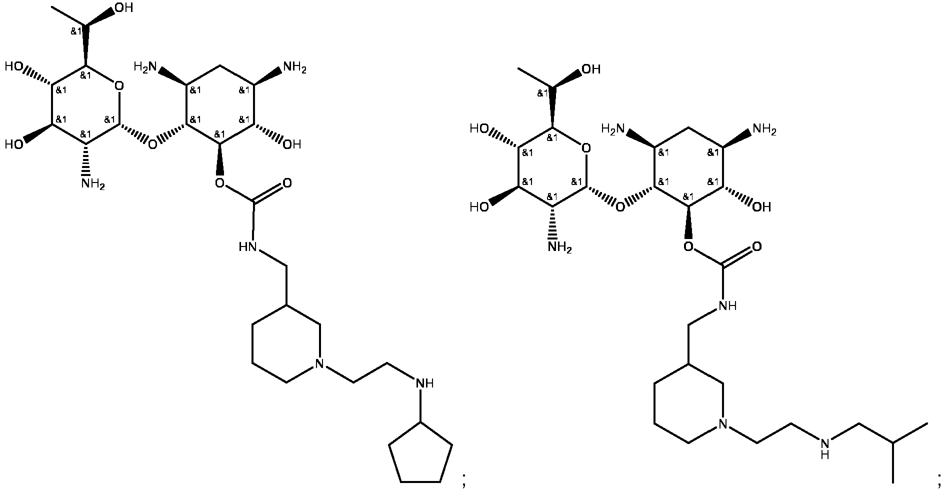 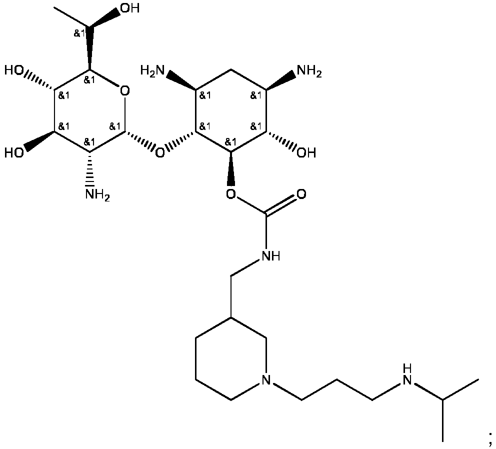 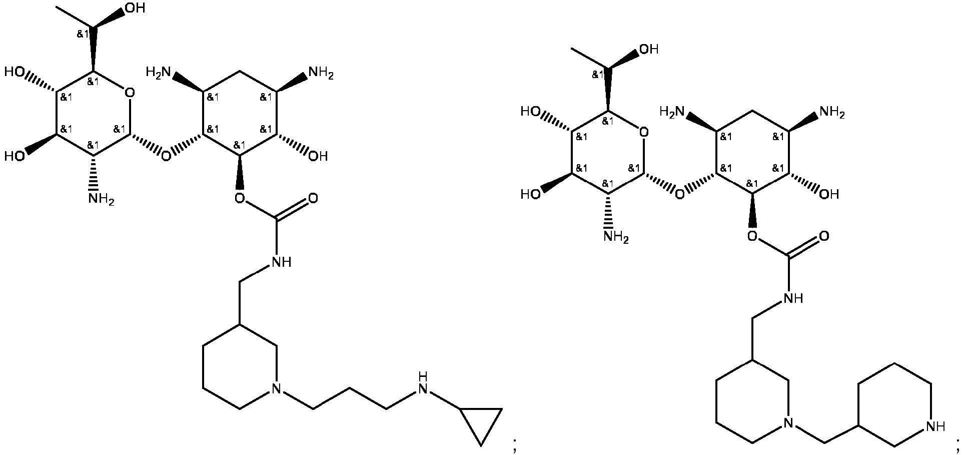 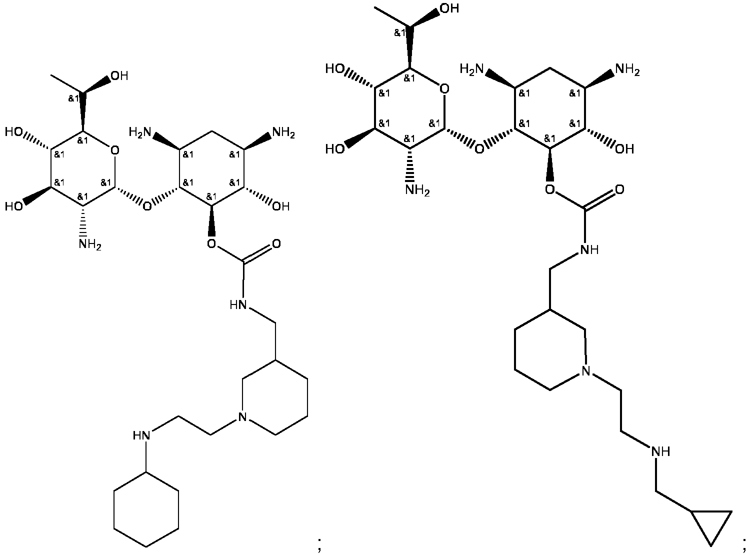 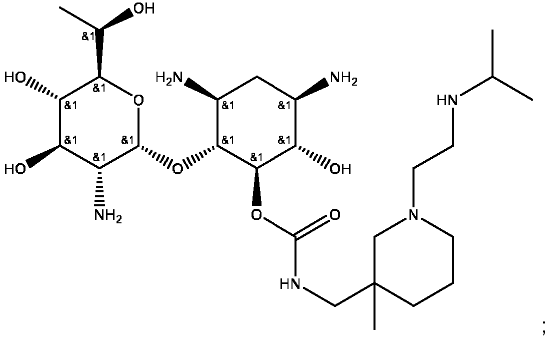 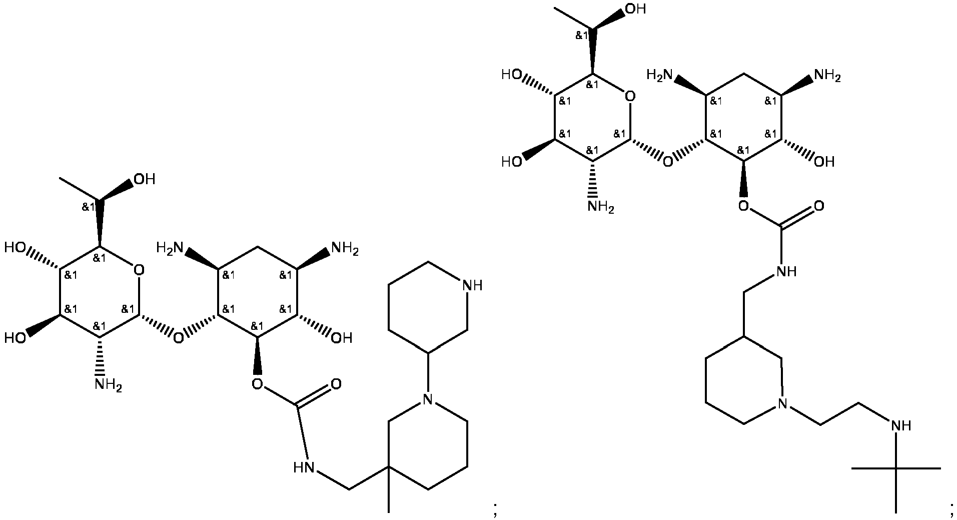 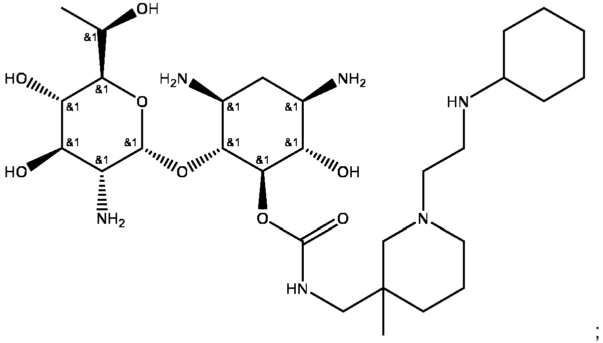 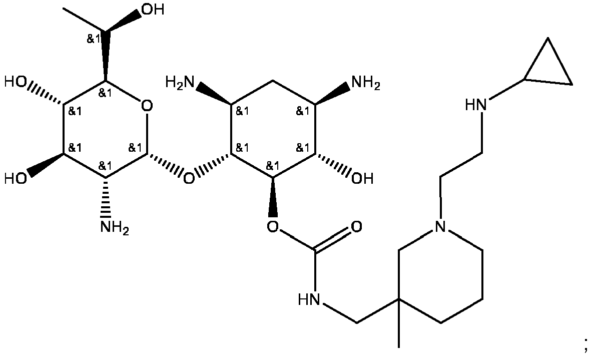 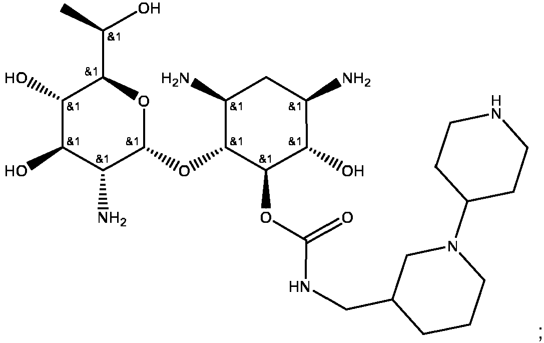 and 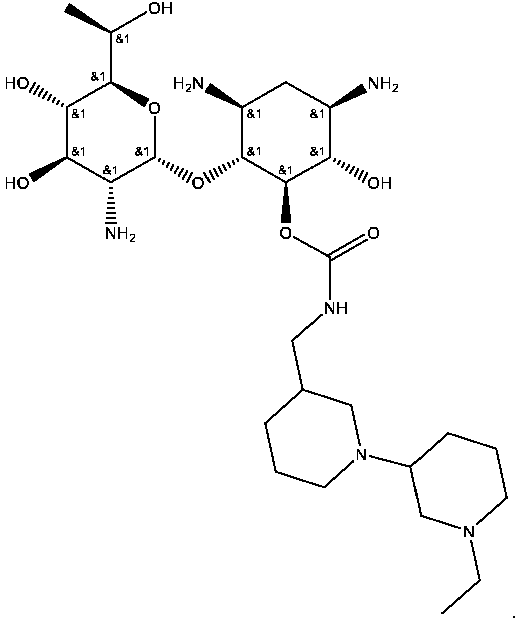 According to an embodiment, the compound is selected from 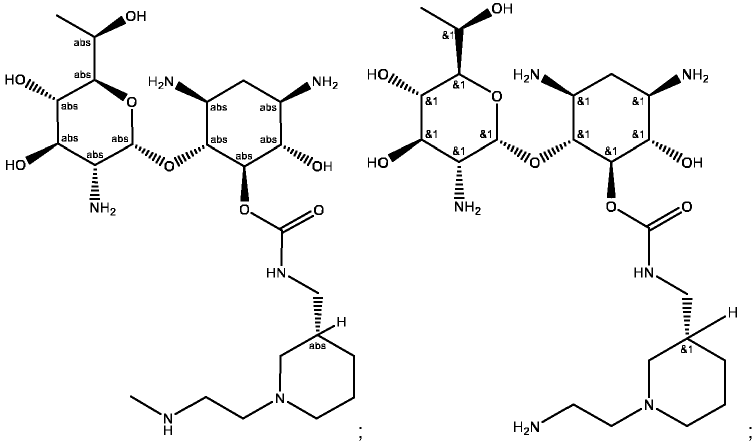 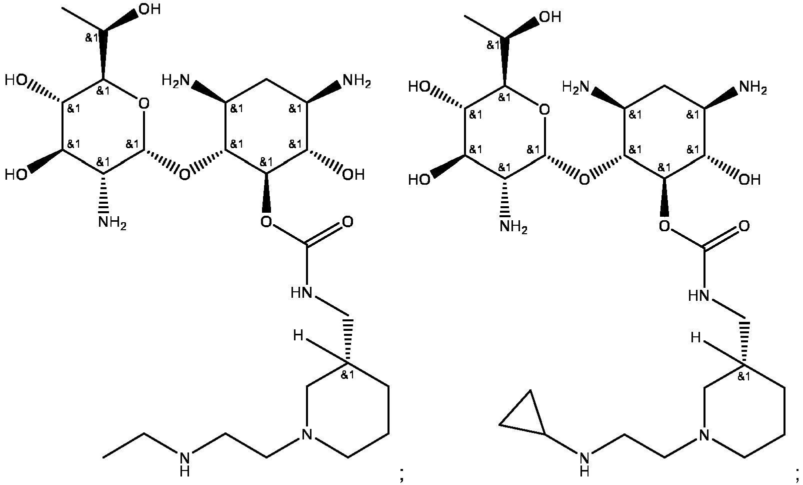 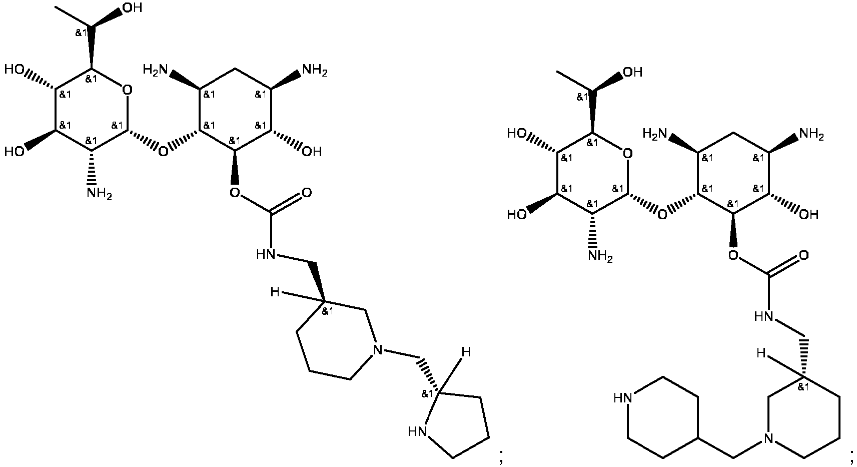 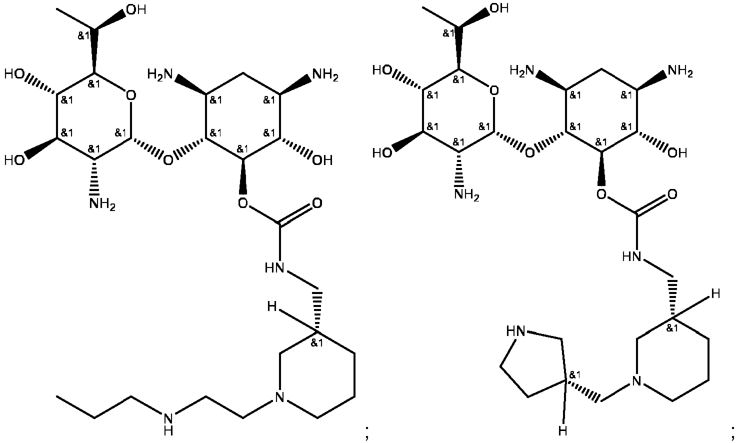 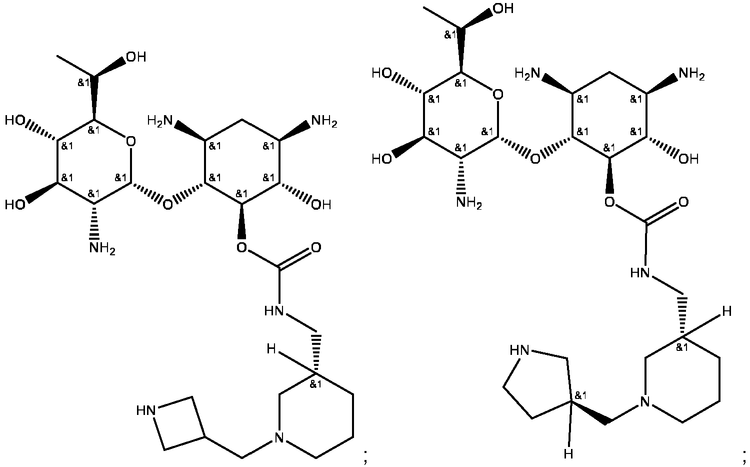 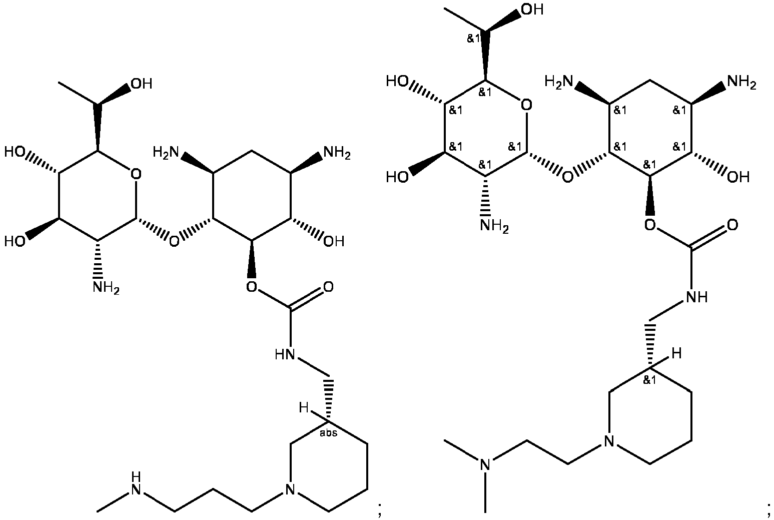 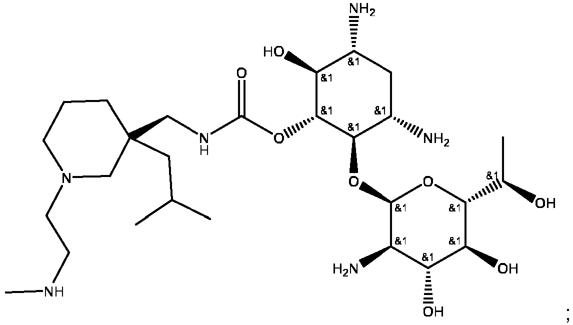 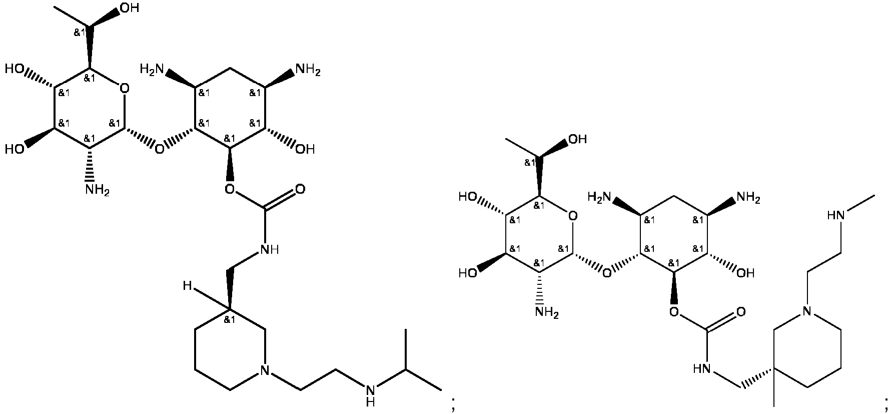 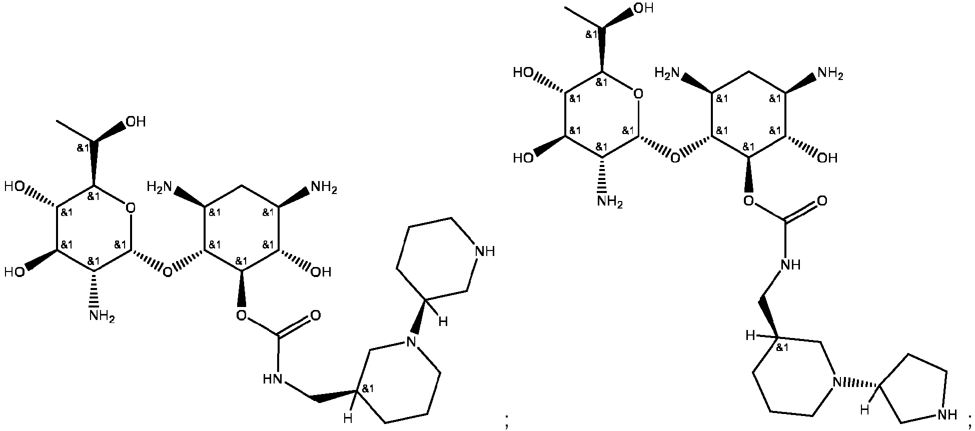 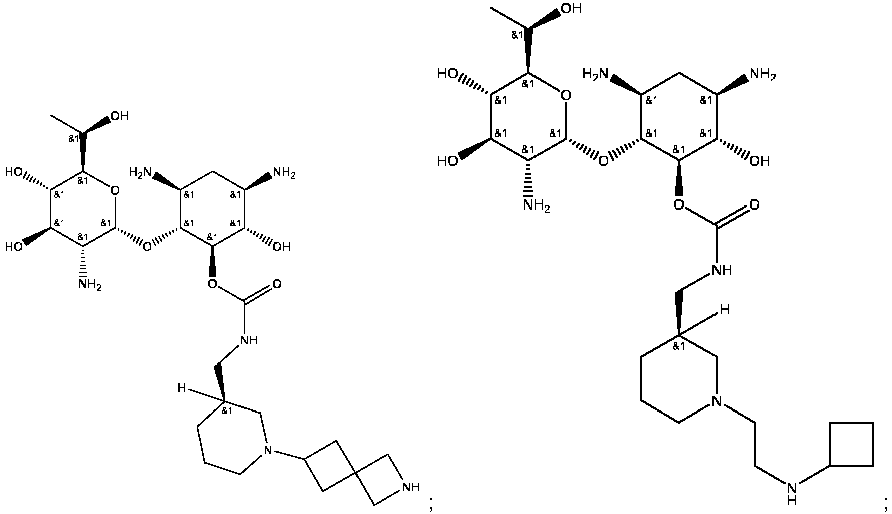 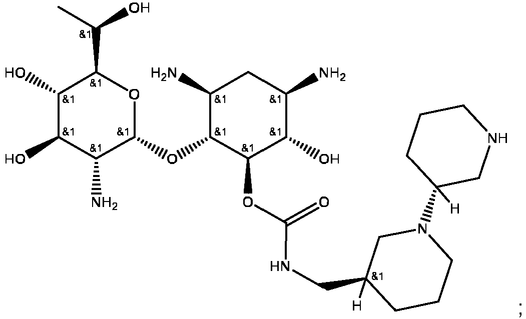 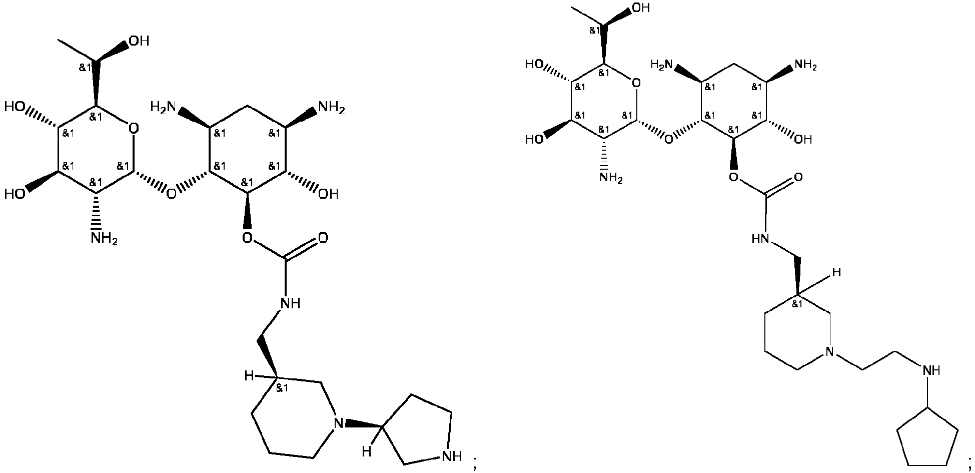 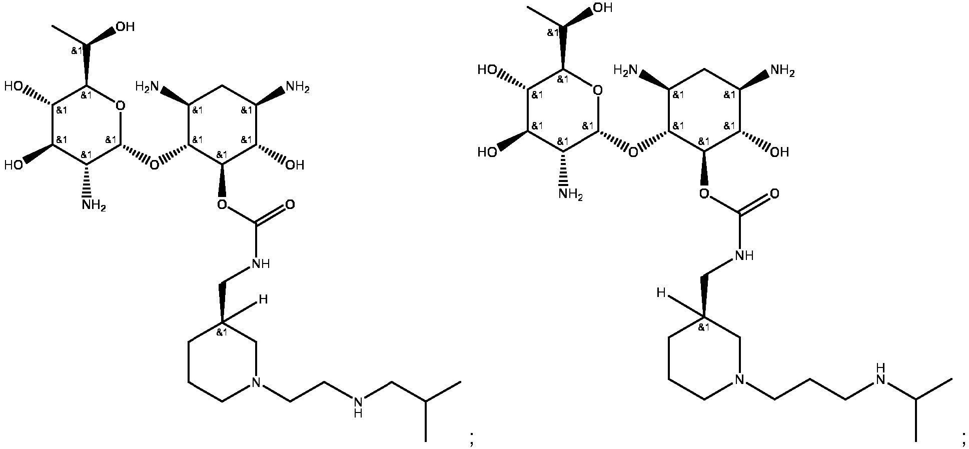 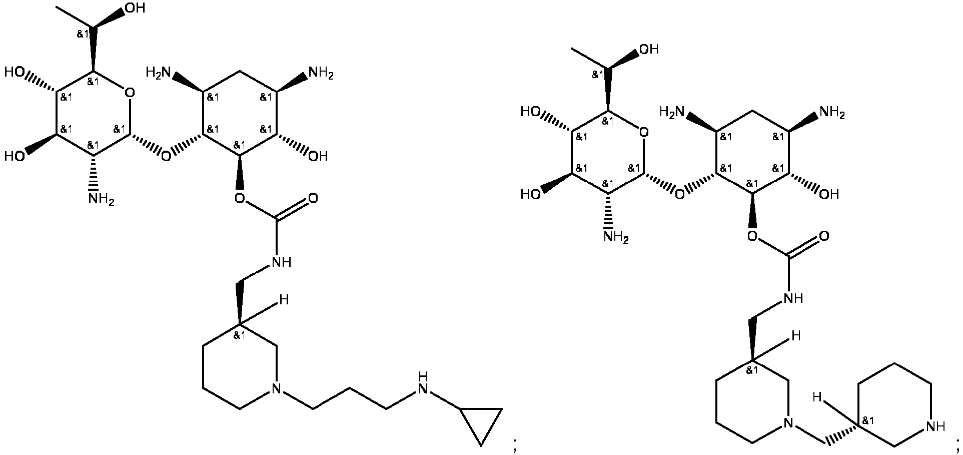 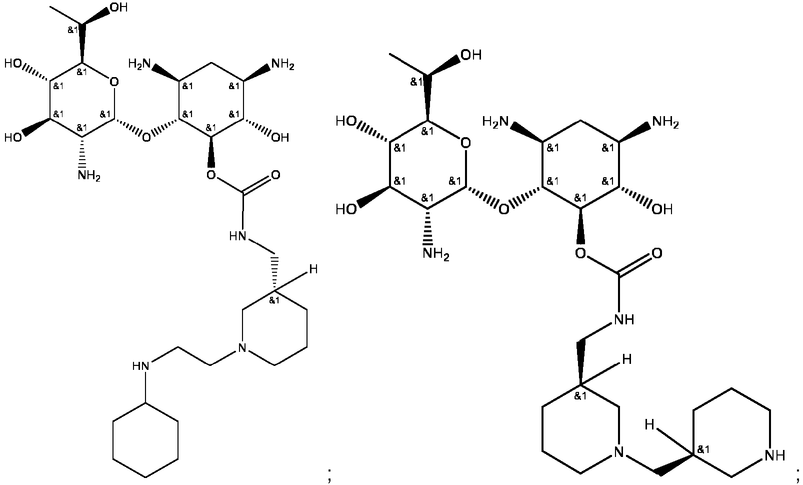 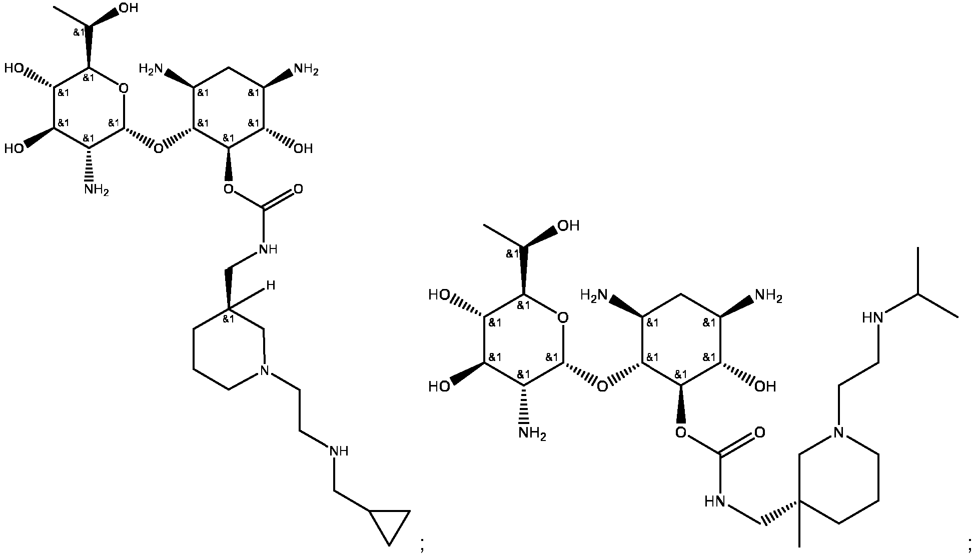 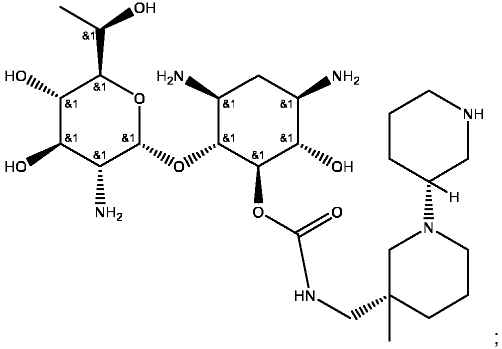 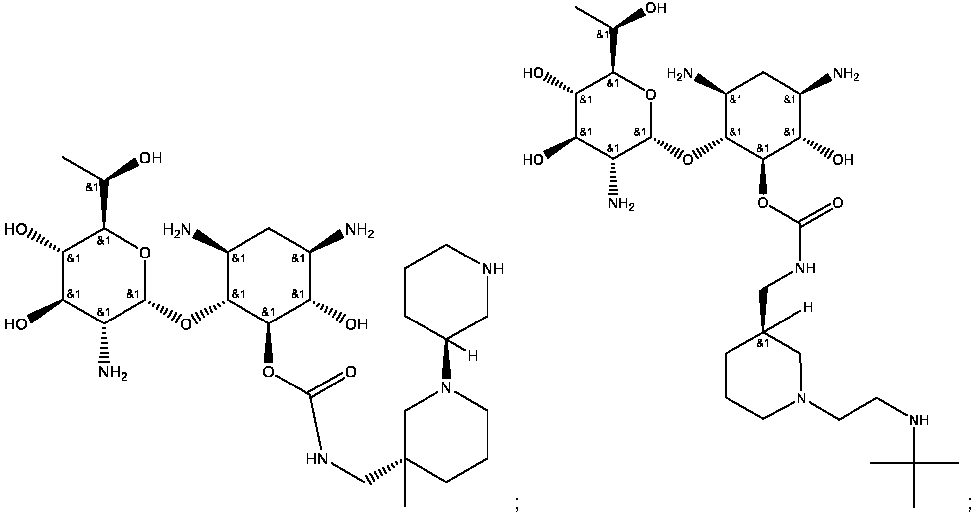 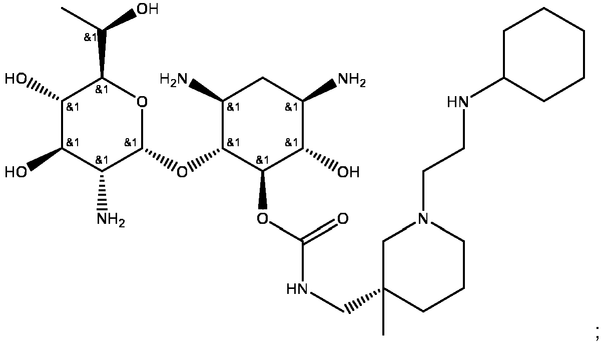 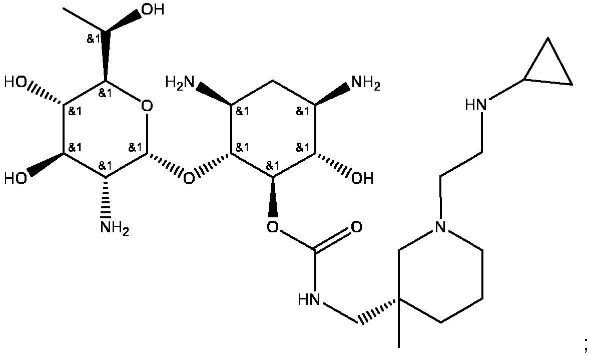 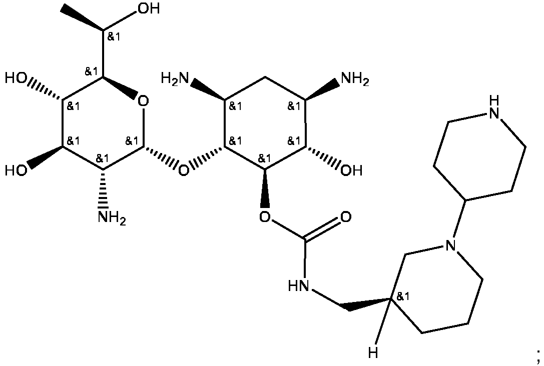 and

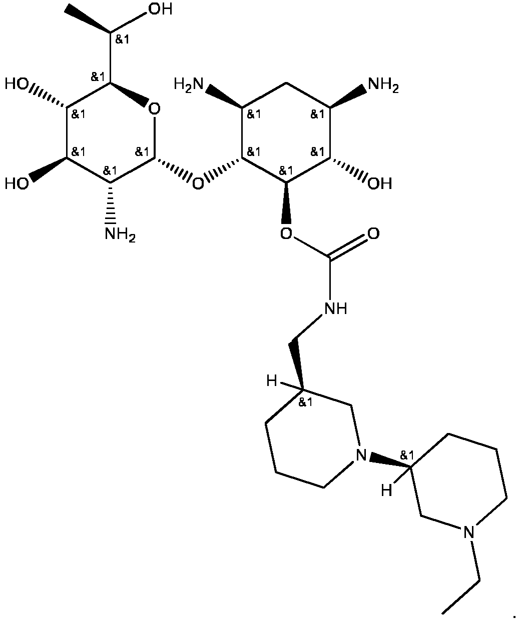

According to an embodiment, the compound is

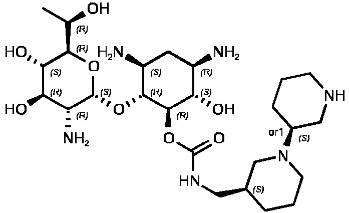

According to an embodiment, the present disclosure relates to a compound of the disclosure having a low toxicity.

According to an embodiment, the present disclosure relates to a compound of the disclosure that is active in readthrough of premature stop codons.

### Pharmaceutical composition

The disclosure also relates to a pharmaceutical composition comprising a compound of the present disclosure and a pharmaceutically acceptable carrier.

In another aspect, the present disclosure provides a composition, e.g. a pharmaceutical composition, containing one or a combination of compounds disclosed herein, (for example, compounds of formula (I)), formulated together with a pharmaceutically acceptable carrier.

As used herein, "pharmaceutically acceptable carrier" includes any and all solvents, dispersion media, coatings, antibacterial and antifungal agents, isotonic and absorption delaying agents, and the like that are physiologically compatible. The pharmaceutical formulation of the disclosure may further comprise one or more pharmaceutically acceptable excipients selected from stabilizers, surfactants, buffering agents, antimicrobial preservatives, protectants, antioxidants, and bulking agents.

As used herein, a "solvent" is any pharmaceutically acceptable (i.e., safe and non-toxic for administration to a human or another mammal) and useful ingredient for the preparation of a liquid formulation, such as an aqueous formulation. Exemplary solvents include water such as sterile water for injection (WFI) or bacteriostatic water for injection (BWFI), pH buffered solution (e.g., phosphate-buffered saline), sterile saline solution, Ringer's solution or dextrose solution, and combinations thereof. Preferably, the solvent is sterile water for disclosure or bacteriostatic water for injection (BWFI).

As used herein, stabilizers are compounds increasing protein stability, especially against unfolding and aggregation. Preferably, the stabilizer is admitted by the authorities as a suitable additive or excipient in pharmaceutical formulations.

The stabilizer may be a saccharide. A "saccharide" herein comprises the general composition (CH₂O)ₙ and derivatives thereof, including monosaccharides, disaccharides, trisaccharides, polysaccharides, sugar alcohols, reducing sugars, nonreducing sugars, etc. Examples of saccharides herein include glucose, sucrose, trehalose, lactose, fructose, maltose, dextran, glycerin, dextran, erythritol, glycerol, arabitol, sylitol, sorbitol, mannitol, mellibiose, melezitose, raffinose, mannotriose, stachyose, maltose, lactulose, maltulose, glucitol, maltitol, lactitol, iso-maltulose, etc.

The concentration of the stabilizer in the pharmaceutical formulation of the disclosure be comprised between 1 and 500 mM.

As used herein, the term 'surfactant' refers to a surface-active agent. Surfactants are generally added to protein formulations to reduce the exposure of hydrophobic regions and thereby reduce protein-protein interactions and interface-induced aggregation, which is also prevented by competition for adsorption sites.

Examples of surfactants herein include polysorbate (for example, polysorbate 20 and, polysorbate 80); poloxamer (e.g. poloxamer 188); Triton; sodium dodecyl sulfate (SDS); sodium laurel sulfate; sodium octyl glycoside; lauryl-, myristyl-, linoleyl-, or stearyl- sulfobetaine; lauryl-, myristyl-, linoleyl- or stearyl-sarcosine; linoleyl-, myristyl-, or cetyl- betaine; lauroamidopropyl-, cocamidopropyl-,linoleamidopropyl-, myristamidopropyl-, palmidopropyl-, or isostearamidopropyl- betaine (e.g. lauroamidopropyl); myristamidopropyl-, palmidopropyl-, or isostearamidopropyl- dimethylamine; sodium methyl cocoyl-, or disodium methyl oleyl-taurate; polyethylglycol, polypropyl glycol, and copolymers of ethylene and propylene glycol (e.g. Pluronics, PF68 etc). Other examples of pharmaceutically acceptable surfactants include polyoxyethylen-sorbitan fatty acid esters (Tween), polyethylene-polypropylene glycols, polyoxyethylene-stearates, polyoxyethylene alkyl ethers, e.g. polyoxyethylene monolauryl ether, alkylphenylpolyoxyethylene ethers (Triton-X), polyoxyethylene-polyoxypropylene copolymer (Poloxamer, Pluronic), and sodium dodecyl sulphate (SDS). Most suitable polyoxyethylenesorbitan-fatty acid esters are polysorbate 20, (sold under the trademark Tween 20^{™}) and polysorbate 80 (sold under the trademark Tween 80^{™}).

Most suitable polyethylene-polypropylene copolymers are those sold under the names Pluronic^{®} F68 or Poloxamer 188^{™}. Most suitable polyoxyethylene alkyl ethers are those sold under the trademark Brij^{™}. Most suitable alkylphenol-polyoxyethylene ethers are sold under the trade name Triton-X.

The concentration of surfactant in the pharmaceutical formulation of the disclosure may be comprised between 0.01 and 0.1 % (w/v).

As used herein, the term "buffering agent" refers to an agent which provides that the solution comprising it resists changes in pH by the action of its acid/base conjugate components. Examples of buffering agents that will control the pH in this range include acetate, succinate, gluconate, histidine, citrate, glycylglycine and other organic acid buffers.

A "preservative" is a compound which can be added to the formulations herein to reduce contamination by and/or action of bacteria, fungi, or another infectious agent. The addition of a preservative may, for example, facilitate the production of a multi-use (multiple-dose) formulation. Examples of potential preservatives include octadecyldimethylbenzylammonium chloride, hexamethonium chloride, benzalkonium chloride (a mixture of alkylbenzyldimemylammonium chlorides in which the alkyl groups are long- chained), and benzethonium chloride. Other types of preservatives include aromatic alcohols such as phenol, butyl and benzyl alcohol, alkyl parabens such as methyl or propyl paraben, catechol, resorcinol, cyclohexanol, 3-pentanol, and w-cresol.

A "protectant", as generally used herein, is a substance which, when combined with a protein, significantly reduces chemical and/or physical instability of the protein upon lyophilization and/or subsequent refrigerated storage. Exemplary protectants include sugars and their corresponding sugar alcohols, such as sucrose, lactose, trehalose, dextran, erythritol, arabitol, xylitol, sorbitol, and mannitol; amino acids, such as arginine or histidine; lyotropic salts, such as magnesium sulfate; polyols, such as propylene glycol, glycerol, poly(ethylene glycol), or poly(propylene glycol); and combinations thereof. Additional exemplary of protectants include gelatin, dextrins, modified starch, and carboxymethyl cellulose.

The protectant may be added to a pre-lyophilized formulation in a "lyoprotecting amount". This means that, following lyophilization of the protein in the presence of lyoprotecting amount of the protectant, the protein essentially retains its physical and chemical stability and integrity.

An "antioxidant", as generally used herein is a pharmaceutically acceptable excipient generally used to limit oxidation reactions and maintain the stability and safety of proteins. Examples of antioxidants are ascorbic acid, sodium metabisulfite, histamine, methionine, ascorbic acid, glutathione, vitamin E, polyethylenimine.

The antioxidant concentration in the pharmaceutical formulation of the disclosure may be comprised between 5 and 25 mM.

A "bulking agent," as generally used herein, is a pharmaceutically acceptable excipient generally used to add mass to a lyophilized mixture and contributes to the physical structure of the lyophilized cake (e.g. facilitates the production of an essentially uniform lyophilized cake which maintains an open pore structure). Exemplary bulking agents include mannitol, glycine, lactose, modified starch, polyethylene glycol), and sorbitol.

The form of the pharmaceutical compositions, the route of administration, the dosage and the regimen naturally depend upon the condition to be treated, the severity of the illness, the age, weight, and sex of the patient.

The pharmaceutical compositions of the disclosure can be formulated for a topical, oral, parenteral, intraperitoneal, intranasal, intravenous, intramuscular, subcutaneous or intraocular administration and the like, preferably intraperitoneal or intravenous.

Preferably, the pharmaceutical compositions contain vehicles, which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The doses used for the administration can be adapted as a function of various parameters, and in particular as a function of the mode of administration used, of the relevant pathology, or alternatively of the desired duration of treatment.

To prepare pharmaceutical compositions, an effective amount of the compound of the disclosure may be dissolved or dispersed in a pharmaceutically acceptable carrier or aqueous medium.

The pharmaceutical forms suitable for injectable use include sterile aqueous solutions or dispersions; formulations including sesame oil, peanut oil or aqueous propylene glycol; and sterile powders or lyophilisates for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

Solutions of the active compounds as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

A compound of the disclosure can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

The preparation of more, or highly concentrated solutions for direct injection is also contemplated, where the use of DMSO as solvent is envisioned to result in extremely rapid penetration, delivering high concentrations of the active agents to a small tumor area.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. The person responsible for administration will, in any event, determine the appropriate dose for the individual subject.

The compound of the disclosure may be formulated within a therapeutic mixture to comprise about 0.0001 to 1.0 milligrams, or about 0.001 to 0.1 milligrams, or about 0.1 to 1.0 or even 1.0 to about 10 milligrams per dose. Multiple doses can also be administered.

The pharmaceutical formulation comprising the compound of the disclosure may be a "Ready-to-Use" injectable formulation or a lyophilized formulation.

In a specific embodiment, the pharmaceutical formulation comprising the compound of the disclosure may be supplied in a pre-filled syringe.

### Method of use

The disclosure also relates to a compound of the disclosure for use as a drug.

Compounds of the present disclosure have therapeutic utilities. For example, these molecules can be administered in a subject, e.g. in vivo, to treat, or prevent a variety of disorders.

It is contemplated herein to use the compound of the present disclosure as a medicament, in particular for use in the treatment of a genetic disorder associated with a premature stop- codon truncation mutation and/or a protein truncation phenotype.

The present disclosure also relates to a method for treating a genetic disorder associated with a premature stop-codon truncation mutation and/or a protein truncation phenotype, the method comprising administering to a subject in need thereof a therapeutically effective amount of a compound as described herein.

The present disclosure also relates to a use of the compound as described herein, in the manufacture of a medicament for treating a genetic disorder associated with a premature stop-codon truncation mutation and/or a protein truncation phenotype.

The genetic disorder can be selected from the group of monogenic diseases including among many others cystic fibrosis (CF), Duchenne muscular dystrophy (DMD), Becker muscular dystrophy (BMD), Congenital muscular dystrophy (CMD), Louis-Bar syndrome / ataxia-telangiectasia (AT), mucopolysaccharidoses such as Hurler syndrome, hemophilia A, hemophilia B, Usher syndrome, Tay-Sachs, Neurofibromatosis type 1 (NF1), Factor VII deficiency, Familial atrial fibrillation, McArdle disease, Nephropathic cystinosis, Polycystic kidney disease, Alport syndrome, Rett syndrome, Spinal muscular atrophy (SMA), various forms of epidermolysis bullosa, Hailey-Hailey disease, Dravet syndrome, X-linked nephrogenic diabetes insipidus (XNDI), X-linked retinitis pigmentosa and cancers caused by a nonsense mutation affecting a tumor suppressor gene such as *TP53, APC, PTEN* or *BRCA2.*

The present disclosure also relates to a method of increasing the expression level of a gene having a premature stop-codon mutation, the method comprising translating the gene into a protein in the presence of a compound as described herein.

The present disclosure also relates to a compound as described herein for use in increasing the expression level of a gene having a premature stop-codon mutation.

The present disclosure also relates to a use of a compound as described herein in the manufacture of a medicament for increasing the expression level of a gene having a premature stop-codon mutation.

According to an embodiment, the premature stop-codon mutation has an RNA code selected from the group consisting of UGA, UAG and UAA.

The present disclosure also relates to a compound as described herein for use in attenuating nonsense mutation mRNA decay (NMD) and/or in treating a disease or disorder in which attenuating NMD is beneficial (e.g., cancer).

According to an embodiment, the provided therapeutic approach is aimed at inducing and/or promoting translational readthrough of the disease causing PTCs, to enable the synthesis and expression of full-length functional proteins.

According to an embodiment, any of the compounds presented herein, are for use in attenuating nonsense-mediated mRNA decay (NMD), and/or are for use in the manufacture of a medicament for attenuating nonsense-mediated mRNA decay (NMD) and/or for treating a disease or disorder associated with dysregulated nonsense-mediated mRNA decay (NMD) and/or a disease or disorder that is treatable by attenuating nonsense-mediated mRNA decay (NMD). In some embodiments, the disease or disorder is a genetic disease or disorder as described herein.

According to an embodiment, any of the compounds presented herein, are for use in treating cancer, as defined herein, or for use in the manufacture of a medicament for treating cancer, as defined herein, and/or for use in a method of treating cancer, as defined herein. According to an embodiment, a compound as described herein is for use in inducing and/or promoting readthrough of a premature stop codon (nonsense) mutation in a tumor suppressing gene. According to some embodiments, a compound as described herein is for use in treating cancer by attenuating NMD.

The phrase "genetic disorder", as used herein, refers to a chronic disorder which is caused by one or more defective genes that are often inherited from the parents, and which can occur unexpectedly when two healthy carriers of a defective recessive gene reproduce, or when the defective gene is dominant. Genetic disorders can occur in different inheritance patterns which include the autosomal dominant pattern wherein only one mutated copy of the gene is needed for an offspring to be affected, and the autosomal recessive pattern wherein two copies of the gene must be mutated for an offspring to be affected.

The phrase "genetic disorder", as used herein, encompasses a genetic disorder, genetic disease, genetic condition or genetic syndrome.

According to some of any of the embodiments of the present invention, the genetic disorder, genetic disease, genetic condition or genetic syndrome, involves a gene having a premature stop-codon mutation, also referred to herein as a truncation mutation and/or a nonsense mutation, which leads to improper translation thereof. The improper translation produces a dysfunctional essential protein or causes a reduction or abolishment of synthesis of an essential protein. In the context of the some embodiments of the present invention, the genetic disorders which are contemplated within the scope of the present embodiments are referred to as genetic disorders associated with a premature stop-codon mutation and/or a protein truncation phenotype.

According to some of any of the embodiments of the present invention, a genetic disorder associated with a premature stop-codon mutation and/or a protein truncation phenotype is treatable by inducing and/or promoting readthrough of the mutation in the complete but otherwise defective transcript (mRNA), or in other words, by inducing and/or promoting suppression of the nonsense mutation (the premature stop-codon mutation and/or the truncation mutation). In the context of embodiments of the present invention, a genetic disorder is one that is treatable by readthrough-inducing and/or promoting compounds.

Methods for identification of a genetic disorder associated with a premature stop-codon mutation and/or a protein truncation phenotype are well known in the art, and include full or partial genome elucidation, genetic biomarker detection, phenotype classification and hereditary information analysis.

Such methods often result in pairs of mutant/wild type (WT) sequences, and these pairs can be used in known methodologies for identifying if the genetic disorder is associated with a premature stop-codon mutation and/or a protein truncation phenotype.

A readthrough-inducing/promoting activity of compounds for treating such genetic disorders can be established by methods well known in the art.

For example, a plasmid comprising two reporter genes interrupted by a sequence of the mutated gene (the genetic disorder-causing gene) is transected into a protein expression platform, either in full cells or in a cell-free systems, and the ratio between the expression level of the two genes in the presence of a tested compound is measured, typically in series of concentrations and duplications, and compared to the gene expression level ratio of the wild-type and/or to the expression level ratio measured in a control sample not containing the tested compound.

It is noted that the experimental model for readthrough activity, namely the nucleotide sequence of gene containing the premature stop-codon mutation, is a byproduct of the process of identifying a genetic disorder as associated with a premature stop-codon mutation and/or a protein truncation phenotype, and further noted that with the great advances in genomic data acquisition, this process is now well within the skills of the artisans of the art, and that once the mechanism of action of a drug candidate is established, as in the case of genetic disorders which have been shown to be associated with a premature stop-codon mutation and/or a protein truncation phenotype, it is well within the skills of the artisans of the art to identify, characterize and assess the efficacy, selectivity and safety of any one of the readthrough-inducing compounds presented herein. It is further well within the skills of the artisans of the art to take the readthrough-inducing compounds presented herein further through the routine processes of drug development.

Methodologies for testing readthrough of a premature stop-codon mutation and/or a truncation mutation, referred to herein as readthrough activity, are known in the art, and several exemplary experimental methods are provided in the Examples section that follows, by which the readthrough-inducing compounds, according to some embodiments of the present invention, can be characterized. It is to be understood that other methods can be used to characterized readthrough-inducing compounds, and such methods are also contemplated within the scope of the present invention. Methods such as provided herein can also be adapted for high throughput screening technology that can assay thousands of compounds in a relatively short period of time.

The skilled person would appreciate that many in vitro methodologies can be used to characterize readthrough-inducing compounds provided herein in terms of safety of use as drugs, and assess the drug candidates in terms of their cytotoxicity versus their efficacy. The skilled artisan would also appreciate that many in vitro methodologies can be used to characterize the readthrough-inducing compounds provided herein for eukaryotic versus prokaryotic selectivity, and such methodologies may also be adapted for high throughput screening technology that can assay thousands of compounds in a relatively short period of time.

Non-limiting examples of genetic disorders, diseases, conditions and syndromes, which are associated with the presence of at least one premature stop-codon or other nonsense mutations include among many others cystic fibrosis (CF), Duchenne muscular dystrophy (DMD), Becker muscular dystrophy (BMD), Congenital muscular dystrophy (CMD), Louis-Bar syndrome / ataxia-telangiectasia (AT), mucopolysaccharidoses such as Hurler syndrome, hemophilia A, hemophilia B, Usher syndrome, Tay-Sachs, Neurofibromatosis type 1 (NF1), Factor VII deficiency, Familial atrial fibrillation, McArdle disease, Nephropathic cystinosis, Polycystic kidney disease, Alport syndrome, Rett syndrome, Spinal muscular atrophy (SMA), various forms of epidermolysis bullosa, Hailey-Hailey disease, Dravet syndrome, X-linked nephrogenic diabetes insipidus (XNDI), X-linked retinitis pigmentosa and cancers caused by a nonsense mutation affecting a tumor suppressor gene such as *TP53, APC, PTEN* or *BRCA2.*

Additional genetic disorders, diseases, conditions and syndromes, which are associated with the presence of at least one premature stop-codon or other nonsense mutations, are listed in "Suppression of nonsense mutations as a therapeutic approach to treat genetic diseases" by Kim M. Keeling, K.M Bedwell, D.M., Wiley Interdisciplinary Reviews: RNA, 2011, 2(6), p. 837-852; "Cancer syndromes and therapy by stop-codon readthrough" by Bordeira-Carrigo, R. et al., Trends in Molecular Medicine, 2012, 18(11), p. 667-678.

The terms "cancer" refers to or describe the physiological condition in mammals that is typically characterized by unregulated cell growth. Examples of cancer include, but are not limited to, carcinoma, lymphoma, blastoma (including medulloblastoma and retinoblastoma), sarcoma (including liposarcoma and synovial cell sarcoma), neuroendocrine tumors (including carcinoid tumors, gastrinoma and islet cell cancer), mesothelioma, schwannoma (including acoustic neuroma), meningioma, adenocarcinoma, melanoma, and leukemia or lymphoid malignancies. More particular examples of such cancers include squamous cell cancer (e.g. epithelial squamous cell cancer), lung cancer including small-cell lung cancer, non- small cell lung cancer, adenocarcinoma of the lung and squamous carcinoma of the lung, cancer of the peritoneum, hepatocellular cancer, gastric or stomach cancer including gastrointestinal cancer, pancreatic cancer, glioblastoma, cervical cancer, ovarian cancer, liver cancer, bladder cancer, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, testicular cancer, esophageal cancer, tumors of the biliary tract, as well as head and neck cancer.

Hence, the disclosure relates to a method of treating cancer, in particular one of the above listed cancers, said method comprising administering a therapeutically efficient amount of a compound of formula (I) as disclosed herein.

The compound for use as disclosed above may be administered as the sole active ingredients or in conjunction with, e.g. as an adjuvant to or in combination to, other drugs e.g. anti-viral, anti-inflammatory agents or cytotoxic, anti-proliferative, chemotherapy or anti-tumor agents, e.g. for the treatment or prevention of diseases mentioned above.

Also within the scope of the present disclosure are kits consisting of the compositions (e.g. comprising a compound of formula (I)) disclosed herein and instructions for use. The kit can further contain at least one additional reagent. Kits typically include a label indicating the intended use of the contents of the kit. The term label includes any writing, or recorded material supplied on or with the kit, or which otherwise accompanies the kit. The kit may further comprise tools for diagnosing whether a patient belongs to a group that will respond to a treatment, as defined above.

### Process of making the compounds of the disclosure

Compounds of the disclosure can be synthesized by any techniques known in the art. The examples of the present disclosure

### Brief description of the drawings

[Fig.1] represents the *in vitro* readthrough activity of the compounds (expressed as pEC₅₀) according to the examples.
[Fig.2] represents the stop codon readthrough assay for 5 premature stop codon found in CFTR. Each value is the median of at least four independent assays. For each stop codon, the left bar represents the reference compound ELX-02, the middle bar represents compound 613, and the right bar represents compound 829.
[Fig.3] represents a ribosome profiling study in HeLa cells treated for 24 h with 50 µm 829 (grey), and DMSO (black) as a negative control. The dashed line indicates the first position of the stop codon.
[Fig.4] represent the pharmacokinetics of compound 829 following a single intravenous (IV) or subcutaneous (SC) administration to CD1 mice.

### Examples

### General Experimental Details

### ¹H NMR analyses

¹H NMR, ¹H{¹⁹F} NMR were recorded at 400 MHz on a Bruker Ultrashield (400) spectrometer. ¹⁹F NMR were recorded at 376 MHz on a Bruker Ultrashield (400) spectrometer. Chemical shifts were reported in ppm. The multiplicity of a signal is designated by the following abbreviations: br s, broad singlet; s, singlet; d, doublet; t, triplet; q, quartet; m, multiplet or a combination of the above. All observed coupling constants, J, are reported in Hertz (Hz). Exchangeable protons are not always observed.

### Enantiopure compound with unknown chirality

Separation of racemic or diastereomeric mixtures by preparative chiral SFC purification led to enantiopure compounds with unknown chirality which was arbitrarily assigned.

The compounds highlighted with stereochemistry "*R**" corresponds to an enantiopure compound for which the stereochemistry can be "*R*" or "S". Similarly the compounds highlighted with stereochemistry "*S**" corresponds to a pure enantiomeric compound for which the stereochemistry can be "*S*" or "*R*".

For the chiral SFC separation of compounds **171/172, 173/174** and **185/186,** the stereochemistry "*R**" was arbitrarily assigned to the first eluted product by chiral SFC analysis/purification and then the second eluted product was assigned "*S**".

For the chiral SFC separation of compounds **181/182** and **187/188,** the stereochemistry "*S**" was arbitrarily assigned to the first eluted product by chiral SFC analysis/purification and then the second eluted product was assigned "*R**".

### General Procedures

### General Procedure A: Carbamate preparation

To a solution of intermediate **6** (1 eq) in DCM (9 mL/mmol of **6**) were added a solution of the corresponding amine (1.2 to 2 eq) in DCM (5 mL/mmol of amine) and Et₃N (2.5 eq.) at room temperature. The reaction mixture was stirred at 25 °C for 1 h to 22 h. The reaction mixture was diluted with DCM and H₂O. The layers were separated and the aqueous layer was extracted with DCM (x3). The combined organic layers were washed with brine dried over Na₂SO₄, filtered and concentrated under reduced pressure. The crude residue was purified by silica gel flash chromatography (see conditions for each compound) to afford the title compound.

### General Procedure B: O-acetyl deprotection

To a solution of **protected alcohol** (1 eq.) in MeOH (11 mL/mmol of protected alcohol) was added Amberlyst A26(-OH) (300 wt%) at room temperature. The reaction mixture was stirred at 25 °C for 17 h to 96 h. The reaction mixture was filtered, rinsed with MeOH (x3) and the resulting filtrate was concentrated under reduced pressure. The crude residue was purified by preparative HPLC (see conditions for each compound) to afford the title compound.

### General Procedures C: Hydrogenolysis

### General procedure C1: Azido reduction (and N-Cbz deprotection)

To an argon-purged solution of **triazido derivative** in dioxane (9 mL/mmol of triazido derivative) and water (9 mL/mmol of azido derivative) was added 20wt% Pd(OH)₂ (0.25 eq.). The resulting mixture was degassed with Argon (x3) and then with H₂ (x3). The reaction mixture was stirred under atmospheric H₂ pressure at 25 °C for 18 h. The resulting mixture was degassed with Argon, filtered through a pad of Celite, rinsed with MeOH and the filtrate was concentrated under reduced pressure. The residue was dissolved in a minimum of MeOH and loaded onto a SiliaPrep SPE-thiol cartridge (230-400 mesh, 40-63 µm). The cartridge was eluted and rinsed with MeOH (3xCV) and the filtrate was concentrated under reduced pressure to afford the title compound.

### General procedure C2: Azido reduction

To an argon-purged solution of **triazido derivative** in MeOH (20 mL/mmol of triazido derivative) was added 10% Pd/C (0.4 eq.). The resulting mixture was degassed with Argon (x3) and then with H₂ (x3). The reaction mixture was stirred under atmospheric H₂ pressure at 25 °C for 18 h. The resulting mixture was degassed with Argon, filtered through a pad of Celite and rinsed with MeOH. The filtrate was concentrated under reduced pressure and the resulting residue was dissolved in a minimum of MeOH and loaded onto a SiliaPrep SPE-thiol cartridge (230-400 mesh, 40-63 µm). The cartridge was eluted and with MeOH (3xCV) and the filtrate was concentrated under reduced pressure to afford the title compound.

### General procedure C3: Azido reduction / N-Cbz deprotection / HCl salt preparation (12N HCl)

To an argon-purged solution of **triazido derivative** in dioxane (9 mL/mmol of triazido derivative) and water (9 mL/mmol of azido derivative) was added 20wt% Pd(OH)₂ (0.25 eq.). The resulting mixture was degassed with Argon (x3) and then with H₂ (x3). The reaction mixture was stirred under atmospheric H₂ pressure at 25 °C for 18 h. The resulting mixture was degassed with Argon, filtered through a pad of Celite, rinsed with MeOH and the filtrate was concentrated under reduced pressure. The residue was dissolved in a minimum of MeOH and loaded onto a SiliaPrep SPE-thiol cartridge (230-400 mesh, 40-63 µm). The cartridge was rinsed with MeOH (3xCV) and the filtrate was concentrated under reduced pressure to afford **amine**

### freebase derivative.

To a solution of **amine freebase derivative** (1 eq.) in MeOH (7 mL/mmol of amine) and H₂O (0.3 mL/mmol of amine) was added a 12N HCl aqueous solution (18 eq.) at 25 °C. The reaction mixture was stirred at 35 °C for 2 h and then concentrated under reduced pressure.

The resulting solid was dissolved in a minimum of MeOH and precipitated with Et₂O (6V). The mixture was centrifugated (4000 rpm, 5 min) and the supernatant was removed. This procedure was repeated two times and the resulting solid was vacuum-dried at 40 °C for 18 h to afford the title compound.

### General procedure C4: Azido reduction / N-Cbz deprotection / HCl salt preparation (1.25N HCl)

To an argon-purged solution of **triazido derivative** in dioxane (9 mL/mmol of triazido derivative) and water (9 mL/mmol of azido derivative) was added 20wt% Pd(OH)₂ (0.25 eq.). The resulting mixture was degassed with Argon (x3) and then with H₂ (x3). The reaction mixture was stirred under atmospheric H₂ pressure at 25 °C for 18 h. The resulting mixture was degassed with Argon, filtered through a pad of Celite, rinsed with MeOH and the filtrate was concentrated under reduced pressure. The residue was dissolved in a minimum of MeOH and loaded onto a SiliaPrep SPE-thiol cartridge (230-400 mesh, 40-63 µm). The cartridge was rinsed with MeOH (3x10 mL) and the filtrate was concentrated under reduced pressure to afford **amine freebase derivative.**

**Amine freebase derivative** (1 eq.) in a 1.25N HCl solution in MeOH (10 eq.) was stirred at 25 °C for 0.5 h. The reaction mixture was concentrated under reduced pressure. The resulting solid was dissolved in a minimum of MeOH and precipitated with Et₂O (6V). The resulting mixture was centrifugated (4000 rpm, 5 min) and the supernatant was removed. This procedure was repeated two times and the resulting solid was vacuum-dried at 40 °C for 2 h to afford the title compound.

### General procedure C5: N-Cbz deprotection

To an argon-purged solution of **N-Cbz protected amine derivative** in MeOH (20 mL/mmol of triazido derivative) was added 10% Pd/C (0.1 eq.). The resulting mixture was degassed with Argon (x3) and then with H₂ (x3). The reaction mixture was stirred under atmospheric H₂ pressure at 25 °C for 18 h. The resulting mixture was degassed with Argon, filtered through a pad of Celite, and rinsed with MeOH. The filtrate was concentrated under reduced pressure to afford the title compound.

### General Procedures D: N-Boc deprotection

### General procedure D1:

***N*-Boc protected amine derivative** (1 eq.) in a 1.25N HCl solution in MeOH (10 eq.) was stirred at 25 °C for 2 h. The reaction mixture was concentrated under reduced pressure at 25 °C. The residue was dissolved in a 1.25N HCl solution in MeOH (10 eq.) and stirred at 25 °C for 2 h. The reaction mixture was concentrated under reduced pressure.

The resulting solid was dissolved in a minimum of MeOH and precipitated with Et₂O (6V). The resulting mixture was centrifugated (4000 rpm, 5 min) and the supernatant was removed. This procedure was repeated two times and the resulting solid was vacuum-dried at 40 °C for 2 h to afford the title compound.

### General procedure D2:

**N-Boc protected amine derivative** (1 eq.) in a 1.25N HCl solution in MeOH (10 eq.) was stirred at 40 °C for 2 h to 18 h. The reaction mixture was concentrated under reduced pressure then co-evaporated with MeOH (x2) to afford the title compound.

### General Procedures E: Reductive amination

### General procedure E1:

To a solution of **amine derivative** (1 eq.) in DCM (8 mL/mmol of amine) was added Et₃N (2.5 eq.) at room temperature. The reaction mixture was stirred for 15 min and a solution of **aldehyde** (1.2-2 eq.) in DCM (2 mL/mmol of aldehyde) was added at room temperature. The reaction mixture was stirred for 15 min and NaBH(OAc)₃ (3 eq.) was added at room temperature. The reaction mixture was stirred at 25 °C for 18 h. The resulting mixture was diluted with DCM and washed with a saturated NaHCO₃ aqueous solution. The aqueous layer was extracted with DCM and the combined organic layers were washed with brine dried over Na₂SO₄, filtered and concentrated under reduced pressure. The crude residue was purified by silica gel flash chromatography or/and by preparative HPLC (see conditions for each compound) to afford the title compound.

### General procedure E2:

To a solution of **amine derivative** (1 eq.) and **aldehyde** (1-1.2 eq.) in MeOH (8 mL/mmol of amine) was added 2-picoline borane complex (1 eq.) at room temperature. The reaction mixture was stirred at 25 °C for 18 h and then concentrated under reduced pressure. The crude residue was purified by silica gel flash chromatography or by preparative HPLC (see conditions for each compound) to afford the title compound.

### General procedure E3:

To a solution of **amine** (1 eq.) in MeOH (6 mL/mmol of amine) was added **aldehyde** (6-10 eq.) at room temperature. The reaction mixture was stirred for 1 h and NaBH₃CN (5 eq.) was added at room temperature. The reaction mixture was stirred at 25 °C for 18 h and then concentrated under reduced pressure. The crude residue was purified by silica gel flash chromatography or preparative HPLC (see conditions for each compound) to afford the title compound.

### General Procedure F: Oxidation

To a solution of **alcohol** (1 eq.) in DCM (2 mL/mmol of alcohol) were added DMSO (10 eq.) and DIPEA (5 eq.) at -10 °C. The reaction mixture was stirred at -10 °C for 15 min before the addition of sulfur trioxide pyridine complex (5 eq.). The resulting mixture was allowed to warm to room temperature and stirred for 0.5 h to 2 h. The reaction mixture was quenched with a 1N HCl aqueous solution (5 eq.), stirred for 15 min and diluted with DCM. The layers were separated and the aqueous layer was extracted with DCM. The combined organic layers were washed successively with a saturated NaHCO₃ aqueous solution, brine, dried over Na₂SO₄, filtered and concentrated under reduced pressure to afford the title compound. The crude residue was used as such in the next step without further purification.

### Experimental Procedures

### Representative examples for the preparation of amine and aldehyde derivatives

### Preparation of some intermediates

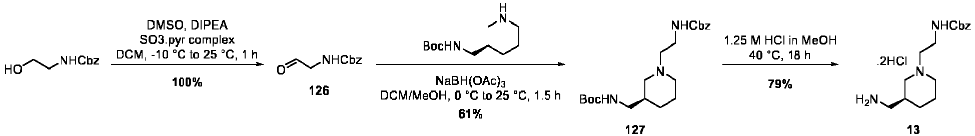

### Intermediate 13: benzyl (S)-(2-(3-(aminomethyl)piperidin-1-yl)ethyl)carbamate dihydrochloride

LCMS (ESI⁺): RT= 2.15 min, [M-2HCl+H]⁺=292.4 (Method 1). ¹H NMR (400 MHz, DMSO-*d₆*): *δ* 10.68 (br s, 1H), 8.14 (br s, 3H), 7.62 (t, *J* = 5.6 Hz, 1H), 7.40-7.30 (m, 5H), 5.04 (s, 2H), 3.64 (d, *J* = 11.7 Hz, 1H), 3.53-3.45 (m, 3H), 3.18-3.00 (m, 2H), 2.87-2.71 (m, 2H), 2.71-2.60 (m, 2H), 2.32-2.20 (m, 1H), 1.91-1.76 (m, 3H), 1.18-1.05 (m, 1H).

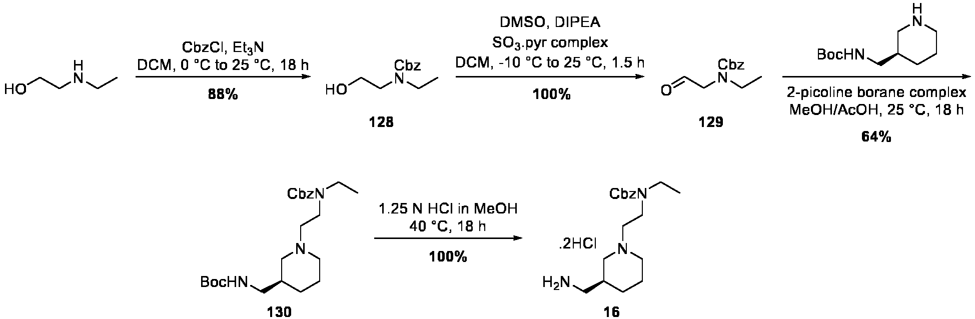

### Intermediate 16: benzyl (S)-(2-(3-(aminomethyl)piperidin-1-yl)ethyl)(ethyl)carbamate dihydrochloride

LCMS (ESI⁺): RT= 2.38 min, [M-2HCl+H]⁺=320.5 (Method 1). ¹H NMR (400 MHz, DMSO-*d₆*): *δ* 10.98-10.66 (m, 1H), 8.16 (br s, 3H), 7.44-7.30 (m, 5H), 5.10 (s, 2H), 3.76-3.58 (m, 3H), 3.33-3.27 (m, 2H), 3.21-3.07 (m, 2H), 3.05-2.93 (m, 1H), 2.90-2.61 (m, 4H), 2.29-2.19 (m, 1H), 1.92-1.75 (m, 3H), 1.18-1.10 (m, 1H), 1.08 (t, *J* = 7.0 Hz, 3H).

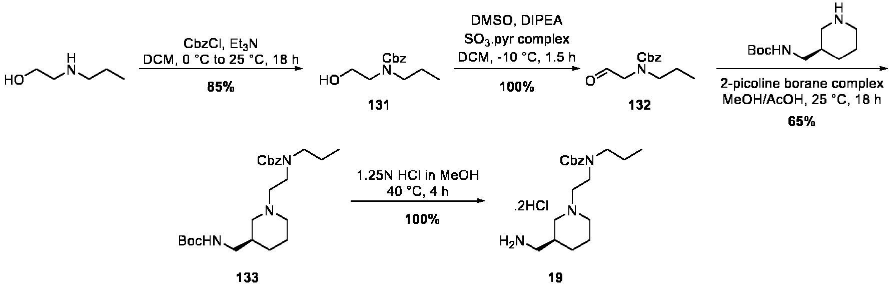

### Intermediate 19: benzyl (S)-(2-(3-(aminomethyl)piperidin-1-yl)ethyl)(propyl)carbamate dihydrochloride

LCMS (ESI⁺): RT= 2.30 min, [M-2HCl+H]⁺=334.4 (Method 1). ¹H NMR (400 MHz, DMSO-*d*₆): *δ* 11.07-10.84 (m, 1H), 8.19 (s, 3H), 7.43-7.30 (m, 5H), 5.09 (s, 2H), 3.78-3.53 (m, 3H), 3.42-3.34 (m, 1H), 3.26-3.07 (m, 4H), 2.87-2.57 (m, 4H), 2.29-2.22 (m, 1H), 1.92-1.73 (m, 3H), 1.52 (h, *J* = 7.4 Hz, 2H), 1.18-1.04 (m, 1H), 0.82 (t, *J =* 7.3 Hz, 3H).

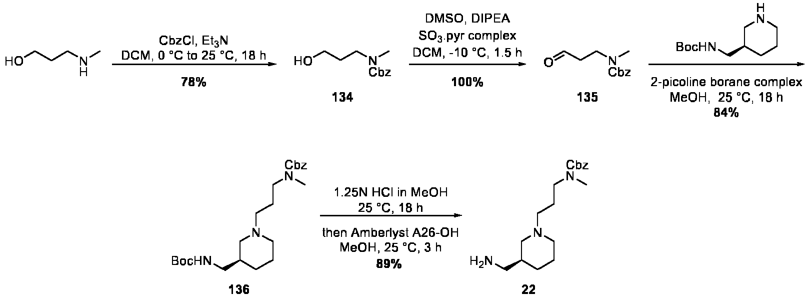

### Intermediate 22: benzyl (S)-(3-(3-(aminomethyl)piperidin-1-yl)propyl)(methyl)carbamate

LCMS (ESI⁺): RT= 1.54 min, [M+H]⁺=320.4 (Method 1). ¹H NMR (400 MHz, CDCl₃): *δ* 7.36-7.28 (m, 5H), 5.12 (s, 2H), 3.36-3.26 (m, 2H), 2.92 (s, 3H), 2.88-2.74 (m, 2H), 2.57-2.51 (m, 2H), 2.34-2.22 (m, 2H), 1.91-1.82 (m, 1H), 1.79-1.62 (m, 6H), 1.61-1.51 (m, 3H), 0.92-0.84 (m, 1H).

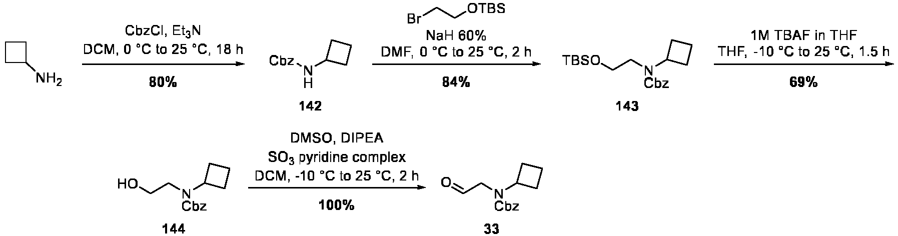

### Intermediate 33: benzyl cyclobutyl(2-oxoethyl)carbamate

¹H NMR (400 MHz, CDCl₃): *δ* 9.62-9.52 (m, 1H), 7.44-7.28 (m, 5H), 5.20-5.05 (m, 2H), 4.14-3.96 (m, 2H), 3.69-3.54 (m, 1H), 1.99-1.87 (m, 2H), 1.72-1.62 (m, 2H), 1.56-1.49 (m, 2H), presence of rotamers.

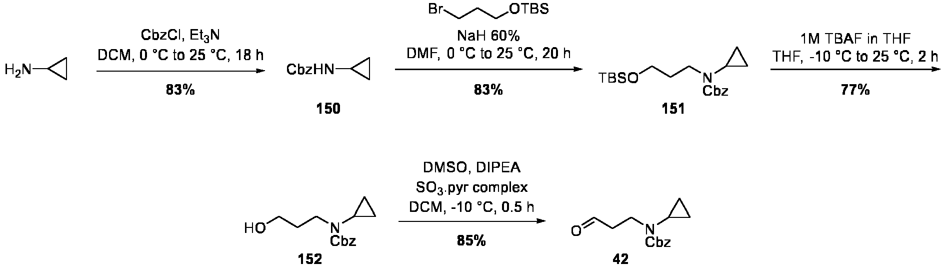

### Intermediate 42: benzyl cyclopropyl(3-oxopropyl)carbamate

LCMS (ESI⁺): RT= 2.19 min, [M+H]⁺=248.2 (Method 1). ¹H NMR (400 MHz, DMSO-*d₆*): *δ* 9.67 (t, *J* = 1.8 Hz, 1H), 7.40-7.28 (m, 5H), 5.07 (s, 2H), 3.52 (d, *J* = 13.7 Hz, 2H), 2.68 (td, *J* = 6.8, 1.8 Hz, 2H), 2.59-2.52 (m, 1H), 0.74-0.68 (m, 2H), 0.64-0.57 (m, 2H).

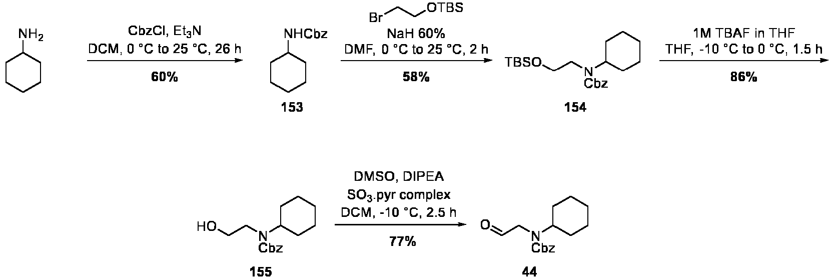

### Intermediate 44: benzyl cyclohexyl(2-oxoethyl)carbamate

LCMS (ESI⁺): RT= 2.50 min, [M+H]⁺=276.4 (Method 1). ¹H NMR (400 MHz, DMSO-*d₆*): *δ* 9.50-9.43 (m, 1H), 7.42-7.22 (m, 5H), 5.16-4.99 (m, 2H), 4.08-3.96 (m, 2H), 3.95-3.78 (m, 1H), 1.77-1.67 (m, 2H), 1.67-1.51 (m, 3H), 1.40-1.16 (m, 4H), 1.10-0.96 (m, 1H), presence of rotamers.

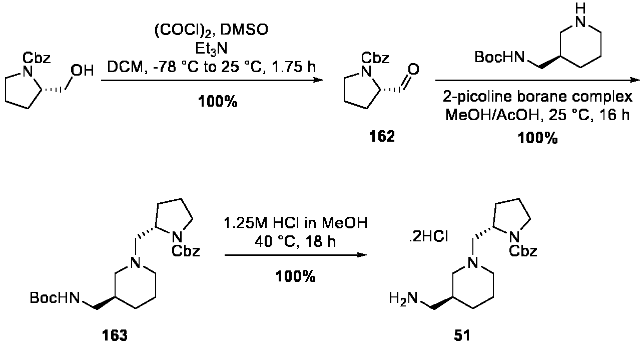

### Intermediate 51: benzyl (S)-2-(((S)-3-(aminomethyl)piperidin-1-yl)methyl)pyrrolidine-1-carboxylate dihydrochloride

LCMS (ESI⁺): RT= 2.13 min, [M-2HCl+H]⁺=332.5 (Method 1). ¹H NMR (400 MHz, CDCl₃): *δ* 10.44 (br s, 1H), 8.73 (br s, 3H), 7.47-7.28 (m, 5H), 5.13 (s, 2H), 4.50-4.35 (m, 1H), 4.35-4.18 (m, 1H), 3.48-3.30 (m, 3H), 3.29-3.13 (m, 2H), 3.13-3.04 (m, 1H), 3.03-2.91 (m, 1H), 2.78-2.63 (m, 1H), 2.27-2.13 (m, 2H), 2.11-1.98 (m, 2H), 1.96-1.88 (m, 2H), 1.70-1.52 (m, 1H), 1.47-1.27 (m, 2H), 0.87-0.76 (m, 1H).

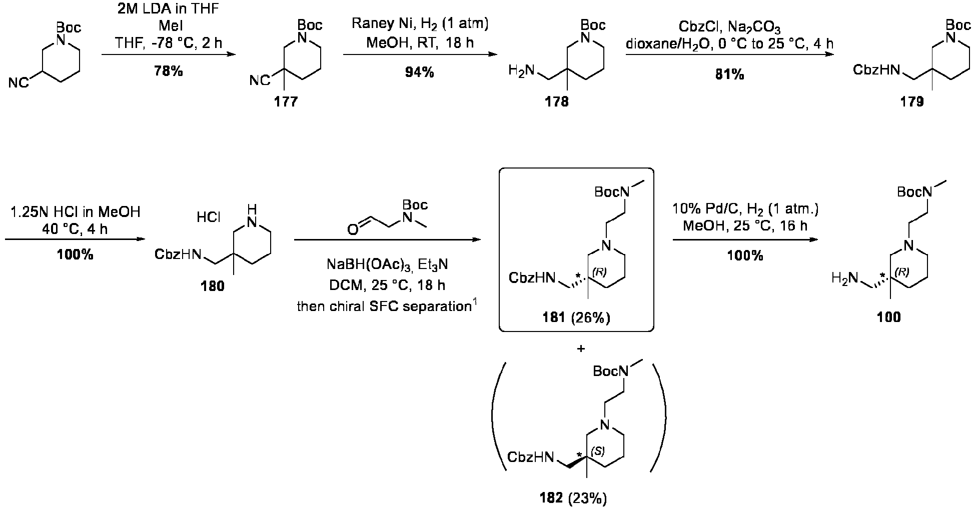

### Intermediate 100: tert-butyl (R*)-(2-(3-(aminomethyl)-3-methylpiperidin-1-yl)ethyl)(methyl)carbamate

LCMS (ESI⁺): RT= 5.43 min, [M+H]⁺=286.3 (Method 4). ¹H NMR (400 MHz, DMSO-*d₆*): *δ* 3.26-3.15 (m, 2H), 2.77 (s, 3H), 2.46-2.36 (m, 2H), 2.35-2.19 (m, 4H), 2.15-2.06 (m, 1H), 2.06-1.97 (m, 1H), 1.64-1.41 (m, 4H), 1.39 (s, 9H), 1.31-1.21 (m, 1H), 1.13-1.03 (m, 1H), 0.81 (s, 3H).

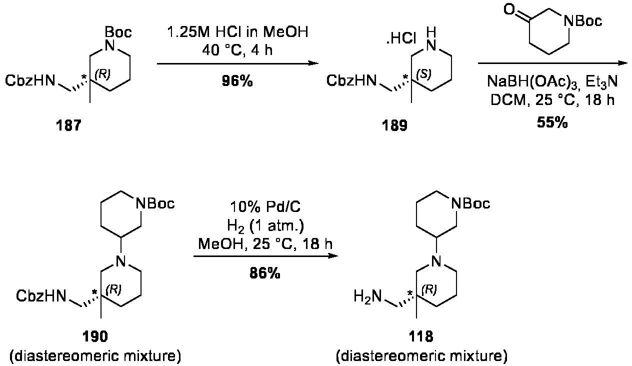

### Intermediate 118: tert-butyl (3R*)-3-(aminomethyl)-3-methyl-[1,3'-bipiperidine]-1'-carboxylate

LCMS (ESI⁺): RT= 5.53 min, [M+H]⁺=312.4 (Method 4). ¹H NMR (400 MHz, DMSO-*d₆*): *δ* 3.96-3.66 (m, 2H), 2.79-2.59 (m, 2H), 2.48-2.35 (m, 3H), 2.27-2.03 (m, 3H), 1.81-1.70 (m, 1H), 1.68-1.59 (m, 1H), 1.57-1.41 (m, 3H), 1.42-1.35 (m, 10H), 1.34-1.16 (m, 3H), 1.16-0.98 (m, 2H), 0.89-0.74 (m, 3H).

### Preparation of intermediate 10

### Synthetic scheme for the preparation of intermediate 10

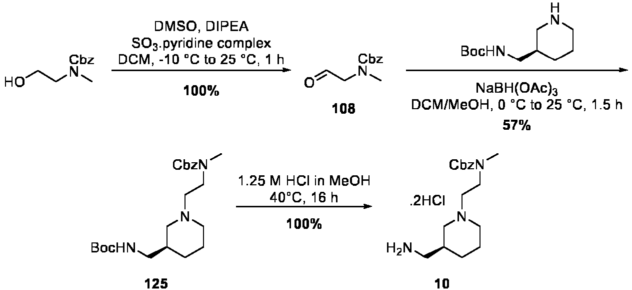

### Compound 108

### benzyl methyl(2-oxoethyl)carbamate

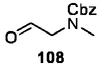

Starting from benzyl (2-hydroxyethyl)(methyl)carbamate (2.50 g, 12.0 mmol, 1 eq.), using general procedure **F,** compound **108** was obtained as an orange oil (2.47 g, 100%). ¹H NMR (400 MHz, CDCl₃): *δ* 9.59-9.50 (m, 1H), 7.34-7.20 (m, 5H), 5.11-5.01 (m, 2H), 4.07-3.95 (m, 2H), 2.98-2.88 (m, 3H), presence of rotamers.

### Compound 125

### benzyl (S)-(2-(3-(((tert-butoxycarbonyl)amino)methyl)piperidin-1-yl)ethyl)(methyl)carbamate

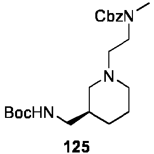

A mixture of tert-butyl (R)-(piperidin-3-ylmethyl)carbamate (2.13 g, 9.93 mmol, 1 eq.) and intermediate **108** (2.47 g, 11.9 mmol, 1.2 eq.) in DCM (97 mL) and MeOH (39 mL) was stirred at 25 °C for 30 min. NaBH(OAc)₃ (6.32 g, 29.8 mmol, 3 eq.) was then added at 0 °C and the reaction mixture was allowed to warm to room temperature and stirred for 1 h. The resulting mixture was diluted with DCM (100 mL) and washed with a saturated NaHCO₃ aqueous solution (100 mL). The aqueous layer was extracted with DCM (2x100 mL) and the combined organic layers were washed with brine (150 mL) dried over Na₂SO₄, filtered and concentrated under reduced pressure. The crude residue was purified by silica gel flash chromatography (DCM/MeOH= from 100:0 to 90:10) to afford intermediate **125** as a yellow oil (2.30 g, 57%). LCMS (ESI⁺): RT= 2.92 min, [M+H]⁺=406.5 (Method 1).

### Compound 10

### benzyl (S)-(2-(3-(aminomethyl)piperidin-1-yl)ethyl)(methyl)carbamate dihydrochloride

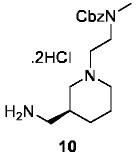

Starting from intermediate **125** (1.80 g, 4.44 mmol, 1 eq.), using general procedure **D2,** compound **10** was obtained as a yellow gum (1.67 g, 100%). LCMS (ESI⁺): RT= 2.60 min, [M-2HCl+H]⁺=306.4 (Method 1). ¹H NMR (400 MHz, DMSO-*d₆*): *δ* 10.78 (br s, 1H), 8.19 (br s, 3H), 7.39-7.27 (m, 5H), 5.09 (s, 2H), 3.81-3.53 (m, 3H), 3.26-3.06 (m, 3H), 2.95-2.87 (m, 3H), 2.84-2.61 (m, 4H), 2.32-2.19 (m, 1H), 1.92-1.73 (m, 3H), 1.18-1.02 (m, 1H).

### Preparation of intermediate 33

### Synthetic scheme for the preparation of intermediate 33

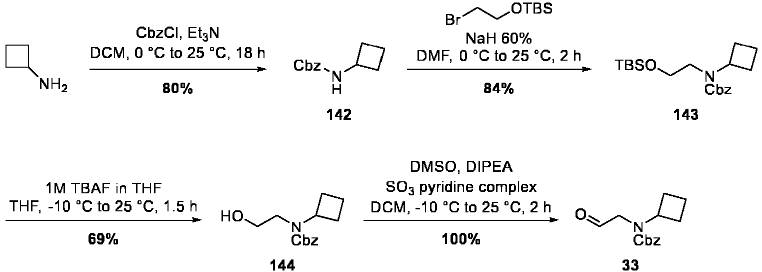

### Compound 142

### benzyl cyclobutylcarbamate

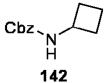

To a solution of cyclobutanamine (1.00 g, 14.1 mmol, 1 eq.) and Et₃N (2.15 mL, 15.5 mmol, 1.1 eq.) in DCM (30 mL) were added benzylchloroformate (2.00 mL, 14.1 mmol, 1 eq.) at 0 °C. The reaction mixture was allowed to warm to room temperature and stirred for 18 h. The resulting mixture was quenched with a 1N HCl aqueous solution (30 mL) and the layers were separated. The aqueous layer was extracted with DCM (2x30 mL) and the combined organic layers were washed with brine (30 mL), dried over Na₂SO₄, filtered and concentrated under reduced pressure. The crude residue was purified by silica gel flash chromatography (Cyclohexane/EtOAc= from 100:0 to 80:20) to afford intermediate **142** as a white solid (2.32 g, 80%). LCMS (ESI⁺): RT= 2.28 min, [M+H]⁺=206.3 (Method 1).

### Compound 143

### benzyl (2-((tert-butyldimethylsilyl)oxy)ethyl)(cyclobutyl)carbamate

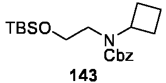

To a solution of intermediate **142** (2.09 g, 10.2 mmol, 1 eq.) in anhydrous DMF (30 mL) was added NaH (60% in mineral oil, 815 mg, 20.4 mmol, 2 eq.) at 0 °C under argon atmosphere. The resulting mixture was stirred for 30 min at 0 °C before the addition of (2-bromoethoxy)(tert-butyl)dimethylsilane (4.37 mL, 20.4 mmol, 2 eq.). The reaction mixture was allowed to warm to room temperature and stirred for 1.5 h. The resulting mixture was cooled to 0 °C, quenched with a saturated NH₄Cl aqueous solution (40 mL) and extracted with EtOAc (3x50 mL). The combined organics layers were washed with brine (3x40 mL), dried over Na₂SO₄, filtered and concentrated under reduced pressure. The crude residue was purified by silica gel flash chromatography (Cyclohexane/EtOAc= from 100:0 to 90:10) to afford intermediate **143** as a yellowish oil (3.10 g, 84%). LCMS (ESI⁺): RT= 3.20 min, [M+H]⁺=364.7 (Method 1).

### Compound 144

### benzyl cyclobutyl(2-hydroxyethyl)carbamate

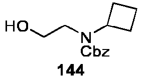

To a solution of intermediate **143** (123 mg, 0.338 mmol, 1 eq.) in anhydrous THF (4 mL) was added a 1M solution of TBAF in THF (0.677 mL, 0.677 mmol, 2 eq.) at -10 °C under argon atmosphere. The reaction mixture was allowed to warm to room temperature and stirred for 1.5 h. The resulting mixture was diluted with H₂O (5 mL) and EtOAc (10 mL). The layers were separated and the aqueous layer was extracted with EtOAc (2x10 mL). The combined organic layers were washed with brine (20 mL), dried over Na₂SO₄, filtered and concentrated under reduced pressure. The crude residue was purified by silica gel flash chromatography (Cyclohexane/EtOAc= from 100:0 to 70:30) to afford intermediate **144** as a colorless oil (58.0 mg, 69%). LCMS (ESI⁺): RT= 2.17 min, [M+Na]⁺=272.3 (Method 1).

### Compound 33

### benzyl cyclobutyl(2-oxoethyl)carbamate

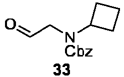

Starting from intermediate **144** (58.0 mg, 0.233 mmol, 1 eq.), using general procedure **F,** compound **33** was obtained as a yellow oil (57 mg, 100%). ¹H NMR (400 MHz, CDCl₃): *δ* 9.62-9.52 (m, 1H), 7.44-7.28 (m, 5H), 5.20-5.05 (m, 2H), 4.14-3.96 (m, 2H), 3.69-3.54 (m, 1H), 1.99-1.87 (m, 2H), 1.72-1.62 (m, 2H), 1.56-1.49 (m, 2H), presence of rotamers.

### Preparation of intermediates 74 and 78

### Synthetic scheme for the preparation of intermediates 74 and 78

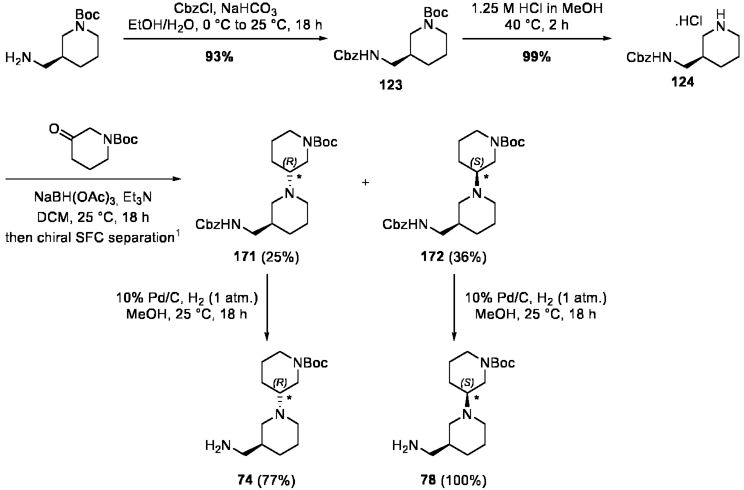

### Compound 123

### tert-butyl (S)-3-((((benzyloxy)carbonyl)amino)methyl)piperidine-1-carboxylate

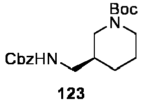

To a solution of *tert-*butyl (*S*)-3-(aminomethyl)piperidine-1-carboxylate (14.0 g, 65.3 mmol, 1 eq.) in a mixture of EtOH (435 mL) and H₂O (218 mL) were added sodium hydrogencarbonate (8.23 g, 98.0 mmol, 1.5 eq.) and benzyl chloroformate (10.2 mL, 71.9 mmol 1.1 eq.) at 0 °C. The reaction mixture was allowed to warm to room temperature and stirred for 18 h. The resulting mixture was diluted with H₂O (600 mL) and extracted with Et₂O (3x300 mL). The combined organic layers were washed with brine (400 mL), dried over Na₂SO₄, filtered and concentrated under reduced pressure. The crude residue was purified by silica gel flash chromatography (Cyclohexane/EtOAc= from 100:0 to 70:30) to afford intermediate **123** as a colorless oil (21.2 g, 93%). LCMS (ESI⁺): RT= 2.53 min, [M+Na]⁺=371.4 (Method 1).

### Compound 124

### benzyl (R)-(piperidin-3-ylmethyl)carbamate hydrochloride

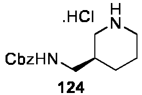

Starting from intermediate **123** (9.25 g, 26.6 mmol, 1 eq.), using general procedure **D2,** compound **124** was obtained as a colorless oil (7.46 g, 99%). LCMS (ESI⁺): RT= 1.63 min, [M-HCl+H]⁺=249.7 (Method 1).¹H NMR (400 MHz, DMSO-*d₆*): *δ* 9.11 (br s, 1H), 8.84 (br s, 1H), 7.44 (t, *J* = 5.8 Hz, 1H), 7.40-7.27 (m, 5H), 5.02 (s, 2H), 3.19-3.11 (m, 2H), 3.03-2.85 (m, 2H), 2.75-2.64 (m, 1H), 2.58-2.49 (m, 1H), 1.92-1.81 (m, 1H), 1.79-1.54 (m, 3H), 1.20-1.07 (m, 1H).

### Compound 171 tert-butyl (3S,3'R*)-3-((((benzyloxy)carbonyl)amino)methyl)-[1,3'-bipiperidine]-1'-carboxylate and Compound 172 tert-butyl (3S,3'S*)-3-((((benzyloxy)carbonyl)amino)methyl)-[1,3'-bipiperidine]-1'-carboxylate

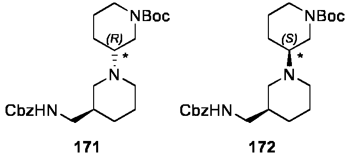

Intermediates **171** and **172** were prepared according to general procedure **E1,** starting from intermediate **124** (14.8 g, 52.0 mmol, 1 eq.) and *tert*-butyl 3-oxopiperidine-1-carboxylate (20.7 g, 10.4 mmol, 2 eq.)(performed in 3 batches). The crude residue was purified by silica gel flash chromatography (EtOAc= 100%) to afford intermediates **171** and **172** as a mixture of diastereomers as a yellow gum (15.6 g, 70%). The mixture of diastereomers was then separated by preparative chiral SFC (column: Chiralpak IG (30x250 mm), Mobile Phase (Isocratic Conditions): CO₂/*i*-PrOH (+0.3% v/v i-PrNH₂), 70:30) to afford intermediates **171** (5.60 g, 25%) and **172** (7.98 g, 36%) as orange oils.

Remark: The stereochemistry "*R**" was arbitrarily assigned to the first eluted product by chiral SFC analysis/purification and then the second eluted product was assigned "*S**".

### Analytical data of intermediate 171:

LCMS (ESI⁺): RT= 2.02 min, [M+H]⁺=432.3 (Method 1).

Chiral SFC analysis (column: Chiralpak IC-3 (4.6x100 mm), Mobile Phase (Isocratic Conditions): CO₂/*i*-PrOH (+0.3% v/v i-PrNH₂), 70:30): RT=1.39 min, 100%.

### Analytical data of intermediate 172:

LCMS (ESI⁺): RT= 1.98 min, [M+H]⁺=432.3 (Method 1).

Chiral SFC analysis (column: Chiralpak IC-3 (4.6x100 mm), Mobile Phase (Isocratic Conditions): CO₂/*i*-PrOH (+0.3% v/v i-PrNH₂), 70:30): RT=1.85 min, 100%.

### Compound 74

### tert-butyl (3S,3'R*)-3-(aminomethyl)-[1,3'-bipiperidine]-1'-carboxylate

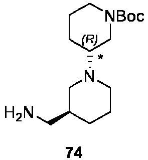

Starting from intermediate **171** (324 mg, 0.751 mmol, 1 eq.), using general procedure **C5,** compound **74** was obtained as a yellow oil (173 mg, 77%). LCMS (ESI⁺): RT= 1.04 min, [M+H]⁺=298.4 (Method 1). ¹H NMR (400 MHz, DMSO-*d₆*): *δ* 4.04-3.80 (m, 1H), 3.76 (d, *J* = 13.2 Hz, 1H), 2.92-2.86 (m, 1H), 2.82-2.75 (m, 1H), 2.72-2.58 (m, 2H), 2.46-2.35 (m, 2H), 2.22-2.05 (m, 2H), 1.88-1.74 (m, 2H), 1.68-1.54 (m, 3H), 1.42-1.34 (m, 13H), 1.33-1.19 (m, 2H), 0.89-0.77 (m, 1H).

### Compound 78

### tert-butyl (3S,3'S*)-3-(aminomethyl)-[1,3'-bipiperidine]-1'-carboxylate

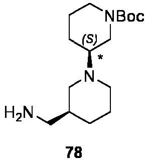

Starting from intermediate **172** (3.73 g, 8.65 mmol, 1 eq.), using general procedure C5, compound **78** was obtained as a yellow oil (2.57 g, 100%). LCMS (ESI⁺): RT= 0.86 min, [M+H]⁺=298.4 (Method 1). ¹H NMR (400 MHz, DMSO-*d₆*): *δ* 4.07-3.81 (m, 1H), 3.81-3.74 (m, 1H), 2.94-2.88 (m, 1H), 2.77-2.69 (m, 1H), 2.69-2.58 (m, 2H), 2.46-2.36 (m, 2H), 2.22-2.09 (m, 2H), 1.88-1.74 (m, 2H), 1.69-1.54 (m, 3H), 1.47-1.40 (m, 2H), 1.39-1.34 (m, 11H), 1.34-1.20 (m, 2H), 0.84 (qd, *J* = 12.0, 3.9 Hz, 1H).

### Preparation of intermediate 104

### Synthetic scheme for the preparation of intermediate 104

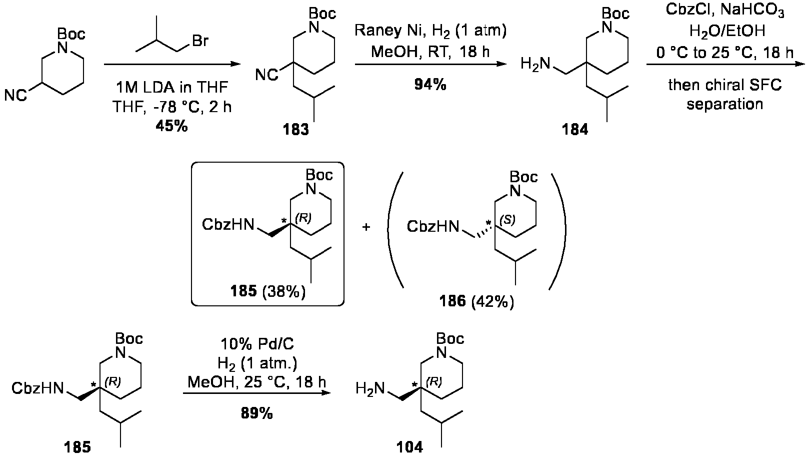

### Compound 183

### tert-butyl 3-cyano-3-isobutylpiperidine-1-carboxylate

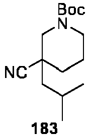

To a solution of *tert-*butyl 3-cyanopiperidine-1-carboxylate (5.00 g, 23.8 mmol, 1 eq.) in anhydrous THF (50 mL) was added a 1M LDA solution in THF/hexanes (47.6 mL, 47.6 mmol, 2 eq.) at -78 °C under argon atmosphere. The reaction mixture was stirred at -78 °C for 30 min before the addition of 1-bromo-2-methylpropane (5.17 mL, 47.6 mmol, 2 eq.). The resulting mixture was stirred at -78 °C for 1.5 h then allowed to warm to room temperature and quenched with a saturated NH₄Cl aqueous solution (150 mL). The mixture was extracted with EtOAc (3x150 mL) and the combined organic layers were dried over Na₂SO₄, filtered and concentrated under reduced pressure. The crude residue was purified by silica gel flash chromatography (Cyclohexane/EtOAc= from 100:0 to 75:25) to afford intermediate **183** as a white solid (2.88 g, 45%). ¹H NMR (400 MHz, DMSO-*d₆*): *δ* 4.19-4.04 (m, 1H), 3.97-3.83 (m, 1H), 2.87-2.61 (m, 2H), 2.03-1.96 (m, 1H), 1.88-1.77 (m, 1H), 1.65-1.45 (m, 3H), 1.44-1.41 (m, 2H), 1.40 (s, 9H), 1.01-0.95 (m, 6H), presence of rotamers.

### Compound 184

### tert-butyl 3-(aminomethyl)-3-isobutylpiperidine-1-carboxylate

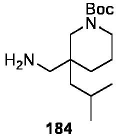

To an argon-purged solution of intermediate **183** (2.88 g, 10.8 mmol, 1 eq.) in MeOH (100 mL) was added Raney-Nickel (50% slurry in H₂O, ~3.81 g, 32.4 mmol, 3 eq.). The resulting mixture was degassed with Argon (x3) and then with H₂ (x3). The reaction mixture was stirred under atmospheric H₂ pressure at 25 °C for 18 h. The resulting mixture was degassed with Argon, filtered through a pad of Celite, and rinsed with MeOH (2x50 mL). The filtrate was concentrated under reduced pressure to afford intermediate **184** as a yellowish oil (2.74 g, 94%). LCMS (ESI⁺): RT= 2.53 min, [M+H]⁺=271.4 (Method 1).

### Compound 185 tert-butyl (R*)-3-((((benzyloxy)carbonyl)amino)methyl)-3-isobutylpiperidine-1-carboxylate and compound 186 tert-butyl (S*)-3-((((benzyloxy)carbonyl)amino)methyl)-3-isobutylpiperidine-1-carboxylate

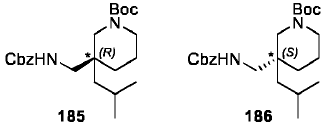

To a solution of intermediate **184** (3.45 g, 12.8 mmol, 1 eq.) in a mixture of EtOH (85 mL) and H₂O (42 mL) were added sodium hydrogencarbonate (1.61 g, 19.1 mmol, 1.5 eq.) and benzyl chloroformate (2.00 mL, 14.0 mmol, 1.1 eq.) at 0 °C. The reaction mixture was allowed to warm to room temperature and stirred for 18 h. The resulting mixture was diluted with H₂O (150 mL) and extracted with Et₂O (3x150 mL). The combined organic layers were washed with brine (150 mL), dried over Na₂SO₄, filtered and concentrated under reduced pressure. The crude residue was purified by silica gel flash chromatography (Cyclohexane/EtOAc= from 100:0 to 80:20) to afford a racemic mixture of intermediates **185** and **186** as a pale yellow oil (5.05 g, 98%).

The racemic mixture was then separated by preparative chiral SFC (column: Chiralpak IG-H (30x250 mm), Mobile Phase (Isocratic Conditions): CO₂/EtOH (+0.3% v/v *i*-PrNH₂), 85:15) to afford intermediates **185** (1.95 g, 38%) and **186** (2.15 g, 42%) as white solids.

Remark: The stereochemistry "*R**" was arbitrarily assigned to the first eluted product by chiral SFC analysis/purification and then the second eluted product was assigned "*S**".

### Analytical data of intermediate 185:

LCMS (ESI⁺): RT= 3.68 min, [M+H]⁺=405.5 (Method 1).

Chiral SFC analysis (column: Chiralpak IG-3 (4.6x100 mm), Mobile Phase (Isocratic Conditions): CO₂/EtOH (+0.3% v/v *i*-PrNH₂), 85:15): RT=1.29 min, 100%.

### Analytical data of intermediate 186:

LCMS (ESI⁺): RT= 3.68 min, [M+H]⁺=405.5 (Method 1).

Chiral SFC analysis (column: Chiralpak IG-3 (4.6x100 mm), Mobile Phase (Isocratic Conditions): CO₂/EtOH (+0.3% v/v *i*-PrNH₂), 85:15): RT=1.54 min, 100%.

### Compound 104

### tert-butyl (R*)-3-(aminomethyl)-3-isobutylpiperidine-1-carboxylate

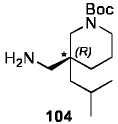

Starting from intermediate **185** (809 mg, 2.00 mmol, 1 eq.), using general procedure **C5,** compound **104** was obtained as a colorless oil (481 mg, 89%). LCMS (ESI⁺): RT= 2.46 min, [M+H]⁺=271.5 (Method 1). ¹H NMR (400 MHz, DMSO-*d₆*): *δ* 3.44-3.33 (m, 1H), 3.23 (d, *J* = 13.1 Hz, 1H), 3.17-3.08 (m, 1H), 3.01-2.82 (m, 1H), 2.47-2.36 (m, 2H), 1.65 (hept, *J =* 6.3 Hz, 1H), 1.48-1.40 (m, 3H), 1.40-1.35 (m, 10H), 1.35-1.22 (m, 2H), 1.21-1.15 (m, 1H), 1.10-1.03 (m, 1H), 0.93-0.83 (m, 6H), presence of rotamers.

### Preparation of key intermediates

### Preparation of intermediate 6

### Synthetic scheme for the preparation of intermediate 6

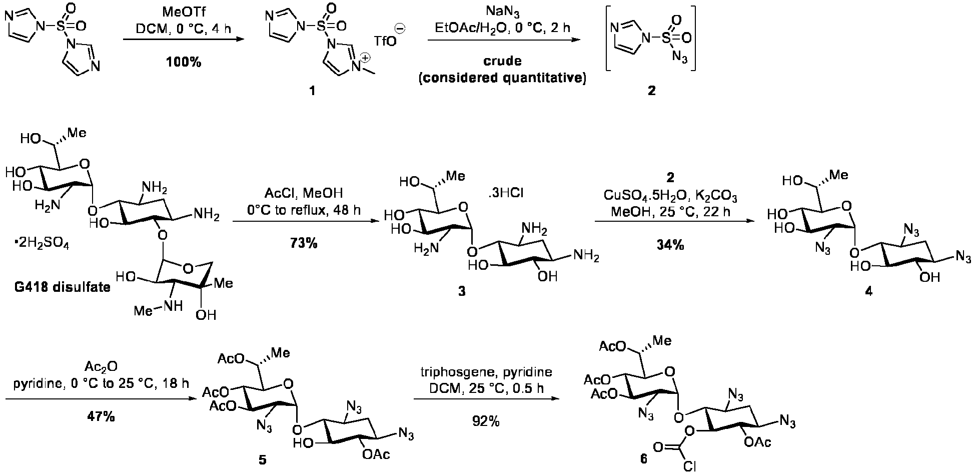

### Compound 1

### 1-(1H-Imidazol-1-ylsulfonyl)-3-methyl-1H-imidazol-3-ium trifluoromethanesulfonate

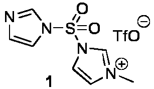

To a suspension of 1,1'-sulfonyldiimidazole (500 g, 2.52 mol, 1.1 eq.) in anhydrous DCM (4.7 L), previously cooled to 0 °C, was added methyl triflate (0.260 L, 2.30 mol, 1 eq.) over 120 min (1.25 mL/min) under Argon atmosphere. The reaction mixture was then stirred at 0 °C for 2 h. The resulting precipitate was filtered, washed with DCM (1 L) and dried under reduced pressure for 1 h to afford intermediate **1** as a white solid (832 g, 100%). ¹H NMR (400 MHz, D₂O): *δ* 9.94 (br s, 1H), 8.52-8.51 (m, 1H), 8.18 (d, *J =* 2.3 Hz, 1H), 7.82-7.79 (m, 1H), 7.73 (d, *J* = 2.3 Hz, 1H), 7.30-7.26 (m, 1H), 4.02 (s, 3H).

### Compound 2

### 1H-Imidazole-1-sulfonyl Azide

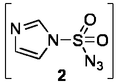

A suspension of intermediate **1** (127.9 g, 353 mmol, 1 eq.) in H₂O (422 mL) was cooled to 0 °C then EtOAc (422 mL) was added. The reaction mixture was stirred at 0 °C for 30 min and sodium azide (27.5 g, 423 mmol, 1.2 eq.) was added slowly by portions. The reaction mixture was stirred at 0 °C for 1.5 h. The two layers were separated and the aqueous layer was extracted with EtOAc (420 mL). The combined organic layers were dried over Na₂SO₄ and filtered to afford a solution of intermediate **2** (considered quantitative) which was directly used in the diazo transfer reaction without further purification (no analysis performed).

### Compound 3

### (2R,3S,4R,5R,6S)-5-amino-6-(((1R,2R,3S,4R,6S)-4,6-diamino-2,3-dihydroxycyclohexyl)oxy)-2-((R)-1-hydroxyethyl)tetrahydro-2H-pyran-3,4-diol trihydrochloride

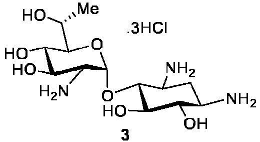

To a solution of anhydrous MeOH (240 mL) was added acetyl chloride (120 mL, 1.69 mol, 39.0 eq.) at 0 °C over 15 min. The reaction mixture was stirred at 0 °C for 15 min and **G418 disulfate** (30.0 g, 43.3 mmol, 1 eq.) was added. The reaction mixture was stirred at reflux for 3 days, then cooled to 0 °C and stored at -20 °C for 18 h. The resulting precipitate was filtered, washed successively with EtOH (500 mL), Et₂O (500 mL) and dried under reduced pressure to afford intermediate **3** as a white solid (14.1 g, 73%). ¹H NMR (400 MHz, D₂O): *δ* 5.59 (d, *J* = 4.1 Hz, 1H), 4.31-4.21 (m, 1H), 3.94-3.83 (m, 3H), 3.69 (t, *J* = 9.2 Hz, 1H), 3.66-3.58 (m, 2H), 3.52-3.43 (m, 2H), 3.40-3.32 (m, 1H), 2.52 (dt, *J =* 12.6, 4.3 Hz, 1H), 1.88 (q, *J* = 12.6 Hz, 1H), 1.24 (d, *J* = 6.5 Hz, 3H). All exchangeable protons are missing.

### Compound 4

### (2R,3S,4R,5R,6S)-5-azido-6-(((1R,2R,3S,4R,6S)-4,6-diazido-2,3-dihydroxycyclohexyl)oxy)-2-((R)-1-hydroxyethyl)tetrahydro-2H-pyran-3,4-diol

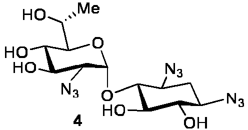

To a solution of intermediate **2** (61.1 g, 353 mmol, 3.6 eq.) in EtOAc (840 mL, from previous step) were added successively a solution of intermediate **3** (43.7 g, 97.9 mmol, 1 eq.) in MeOH (840 mL), K₂CO₃ (108 g, 784 mmol, 8 eq.) and CuSO₄·5H₂O (7.31 g, 29.3 mmol, 0.3 eq.) at room temperature. The resulting suspension was stirred at 25 °C for 20 h. The reaction mixture was diluted with brine (2.5 L), H₂O (1 L) and EtOAc (1.5 L) and filtered. The obtained two layers were separated and the aqueous layer was extracted with EtOAc (3x1.5 L). The combined organic layers were washed with brine (1 L), dried over Na₂SO₄, filtered and concentrated under reduced pressure. The crude residue was purified by silica gel column chromatography (cyclohexane/EtOAc= 80:20 to 0:100) to afford intermediate **4** as a white solid (13.8 g, 34%). ¹H NMR (400 MHz, DMSO-*d₆*): *δ* 5.65 (d, *J* = 3.8 Hz, 1H), 5.54 (d, *J* = 5.8 Hz, 1H), 5.44 (d, *J* = 5.8 Hz, 1H), 5.28 (d, *J =* 5.1 Hz, 1H), 5.22 (d, *J* = 4.6 Hz, 1H), 4.67 (d, *J* = 5.2 Hz, 1H), 3.90-3.82 (m, 1H), 3.73-3.66 (m, 2H), 3.57-3.50 (m, 1H), 3.48-3.34 (m, 3H), 3.23-3.12 (m, 2H), 3.01 (dd, *J =* 10.5, 3.8 Hz, 1H), 2.07 (dt, *J =* 12.4, 4.3 Hz, 1H), 1.28 (q, *J =* 12.3 Hz, 1H), 1.09 (d, *J =* 6.5 Hz, 3H).

### Compound 5

### (2R,3S,4R,5R,6S)-6-(((1R,2S,3S,4R,6S)-3-acetoxy-4,6-diazido-2-hydroxycyclohexyl)oxy)-2-((R)-1-acetoxyethyl)-5-azidotetrahydro-2H-pyran-3,4-diyl diacetate

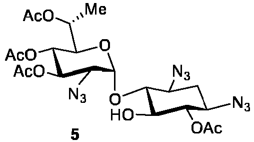

To a solution of intermediate **4** (43.5 g, 105 mmol, 1 eq.) in pyridine (705 mL) was added acetic anhydride (78.6 mL, 837 mmol, 8 eq.) dropwise at 0 °C. The reaction mixture was allowed to warm to room temperature and stirred for 18 h. MeOH (40 mL) was added and the reaction mixture was concentrated under reduced pressure. The resulting residue was diluted with EtOAc (800 mL) and washed successively with a 1M HCl aqueous solution (2x400 mL) and brine (400 mL). The organic layer was dried over Na₂SO₄, filtered and concentrated under reduced pressure. The crude residue was purified by silica gel column chromatography (Pentane/EtOAc= 85:15 to 60:40) to afford intermediate **5** as a white foam (28.5 g, 47%). ¹H NMR (400 MHz, DMSO-*d₆*): *δ* 5.87-5.84 (m, 1H), 5.80 (d, *J* = 3.7 Hz, 1H), 5.30 (dd, J = 10.9, 9.2 Hz, 1H), 4.91 (dd, J = 10.5, 9.2 Hz, 1H), 4.88-4.82 (m, 2H), 4.26 (dd, J = 10.4, 2.2 Hz, 1H), 3.75-3.65 (m, 2H), 3.65-3.58 (m, 3H), 2.22 (dt, *J* = 12.2, 4.3 Hz, 1H), 2.07 (s, 3H), 2.04 (s, 3H), 2.03 (s, 3H), 2.01 (s, 3H), 1.66 (q, *J* = 12.1 Hz, 1H), 1.18 (d, *J =* 6.8 Hz, 3H).

### Compound 6

### (2R,3S,4R,5R,6S)-6-(((1R,2S,3S,4R,6S)-3-acetoxy-4,6-diazido-2-((chlorocarbonyl)oxy)cyclohexyl)oxy)-2-((R)-1-acetoxyethyl)-5-azidotetrahydro-2H-pyran-3,4-diyl diacetate

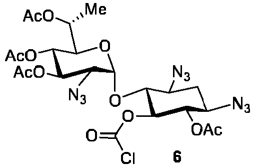

To a solution of intermediate **5** (5.00 g, 8.57 mmol, 1 eq.) in anhydrous DCM (110 mL) was added anhydrous pyridine (6.24 mL, 77.1 mmol, 9 eq.) at 25 °C. The reaction mixture was cooled to 0 °C and triphosgene (3 eq., 7.63 g, 25.706 mmol) was added portionwise (exothermic reaction). The reaction mixture was allowed to warm to 25 °C and stirred for 0.5 h. The resulting mixture was diluted with DCM (50 mL) and washed successively with a saturated NH₄Cl aqueous solution (100 mL), a 1N HCl aqueous solution (100 mL) and a saturated NaHCO₃ aqueous solution (100 mL). The organic layer was dried over Na₂SO₄, filtered and concentrated under pressure to afford intermediate **6** as a pale yellow solid (5.11 g, 92%). ¹H NMR (CDCl₃): *δ* 5.43 (dd, *J* = 10.7, 9.1 Hz, 1H), 5.21 (d, *J* = 3.8 Hz, 1H), 5.07-4.93 (m, 4H), 4.36 (dd, *J* = 10.4, 2.0 Hz, 1H), 3.72-3.58 (m, 2H), 3.57-3.46 (m, 2H), 2.45 (dt, *J =* 13.5, 4.6 Hz, 1H), 2.13 (s, 3H), 2.08 (s, 3H), 2.07 (s, 3H), 2.06 (s, 3H), 1.59 (q, *J =* 12.5 Hz, 1H), 1.25 (d, *J =* 6.7 Hz, 3H).

### Preparation of intermediate 9

### Synthetic scheme for the preparation of intermediate 9

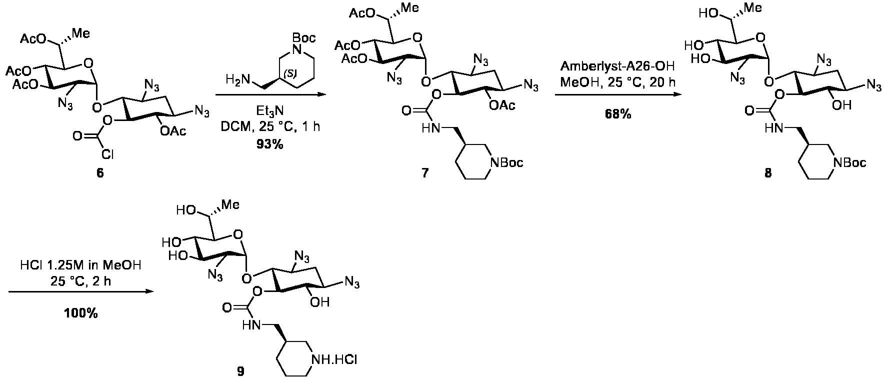

### Compound 7

### (2R,3S,4R,5R,6S)-6-(((1R,2S,3S,4R,6S)-3-acetoxy-4,6-diazido-2-(((((S)-1-(tert-butoxycarbonyl)piperidin-3-yl)methyl)carbamoyl)oxy)cyclohexyl)oxy)-2-((R)-1-acetoxyethyl)-5-azidotetrahydro-2H-pyran-3,4-diyl diacetate

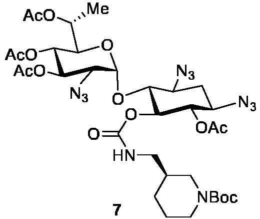

Intermediate **7** was prepared according to general procedure **A,** starting from intermediate **6** (5.31 g, 8.22 mmol, 1 eq.) and *tert-*butyl (3*S*)-3-(aminomethyl)piperidine-1-carboxylate (2.11 g, 9.87 mmol, 1.2 eq.). The crude residue was purified by silica gel flash chromatography (cyclohexane/EtOAc= from 90:10 to 60:40) to afford intermediate **7** as a white solid (6.28 g, 93%). LCMS (ESI⁺): RT= 2.67 min, [M+H]⁺=824.9 (Method 1).

### Compound 8

### tert-butyl (S)-3-((((((1R,2R,3S,5R,6S)-3,5-diazido-2-(((2S,3R,4R,5S,6R)-3-azido-4,5-dihydroxy-6-((R)-1-hydroxyethyl)tetrahydro-2H-pyran-2-yl)oxy)-6-hydroxycyclohexyl)oxy)carbonyl)amino)methyl)piperidine-1-carboxylate

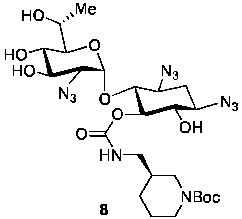

Intermediate 8 was prepared according to general procedure **B,** starting from intermediate 7 (6.28 g, 7.62 mmol, 1 eq.). The crude residue was purified by preparative HPLC (column: XBridge C18 (30x150 mm (5µm)); flow Rate: 43 mL/min; gradient: Water (+0.2% NH₄HCO₃)/Acetonitrile = from 65:35 to 1:1) to afford intermediate **8** as a white solid (3.38 g, 68%). LCMS (ESI⁺): RT= 8.05 min, [M+H]⁺=656.8 (Method 2).

### Compound 9

### (1R,2R,3S,5R,6S)-3,5-diazido-2-(((2S,3R,4R,5S,6R)-3-azido-4,5-dihydroxy-6-((R)-1-hydroxyethyl)tetrahydro-2H-pyran-2-yl)oxy)-6-hydroxycyclohexyl-(((R)-piperidin-3-yl)methyl)-carbamate hydrochloride

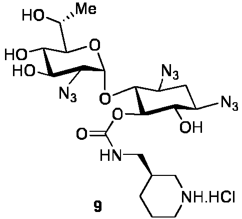

Starting from intermediate **8** (1.70 g, 2.58 mmol, 1 eq.), using general procedure **D1,** intermediate **9** was obtained as hydrochloride salt as a white solid (1.53 g, 100%). LCMS (ESI⁺): RT= 5.35 min, [M-HCl+H]⁺=556.8 (Method 2).

### Synthesis of compounds

### Preparation of 244

### Synthetic scheme

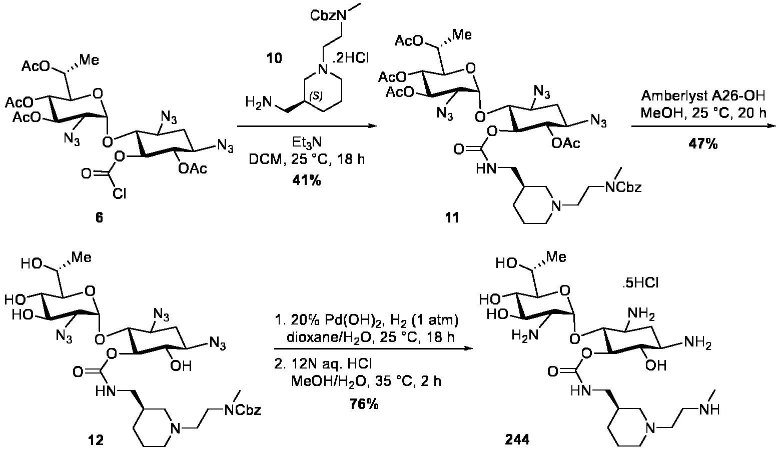

### Compound 11

### (2R,3S,4R,5R,6S)-6-(((1R,2S,3S,4R,6S)-3-acetoxy-4,6-diazido-2-(((((S)-1-(2-(((benzyloxy)carbonyl)(methyl)amino)ethyl)piperidin-3-yl)methyl)carbamoyl)oxy)cyclohexyl)oxy)-2-((R)-1-acetoxyethyl)-5-azidotetrahydro-2H-pyran-3,4-diyl diacetate

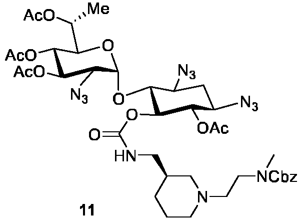

Intermediate 11 was prepared according to general procedure A, starting from intermediate 6 (598 mg, 0.925 mmol, 1 eq.) and intermediate **10** (700 mg, 1.85 mmol, 2 eq.). The crude residue was purified by silica gel flash chromatography (DCM/MeOH= from 100:0 to 90:10) to afford intermediate 11 as a white solid (347 mg, 41%). LCMS (ESI⁺): RT= 2.91 min, [M+H]⁺=915.6 (Method 1).

### Compound 12

### benzyl (2-((S)-3-((((((1R,2R,3S,5R,6S)-3,5-diazido-2-(((2S,3R,4R,5S,6R)-3-azido-4,5-dihydroxy-6-((R)-1-hydroxyethyl)tetrahydro-2H-pyran-2-yl)oxy)-6-hydroxycyclohexyl)oxy)carbonyl)amino)methyl)piperidin-1-yl)ethyl)(methyl)carbamate

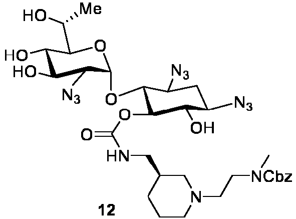

Intermediate **12** was prepared according to general procedure **B,** starting from intermediate **11** (373 mg, 0.408 mmol, 1 eq.). The crude residue was purified by preparative HPLC (column: XBridge C18 (30x150 mm (5µm)); flow Rate: 43 mL/min; gradient: Water (+0.2% NH₄HCO₃)/Acetonitrile = from 75:25 to 55:45) to afford intermediate **12** as a white solid (142 mg, 47%). LCMS (ESI⁺): RT= 7.21 min, [M+H]⁺=747.3 (Method 3).
**244.**

### (1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl (((S)-1-(2-(methylamino)ethyl)piperidin-3-yl)methyl)carbamate pentahydrochloride

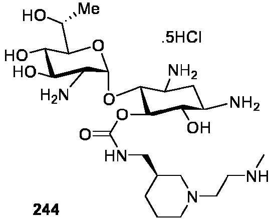

Starting from intermediate **12** (48.0 mg, 0.0643 mmol, 1 eq.), using general procedure **C3,** compound **244** was obtained as a white solid (35.0 mg, 76%). LCMS (ESI⁺): RT= 6.23 min, [M-5HCl+H]⁺=535.5 (Method 5). ¹H NMR (400 MHz, D₂O): *δ* 5.47 (d, *J* = 4.0 Hz, 1H), 4.97 (t, *J* = 9.3 Hz, 1H), 4.34-4.26 (m, 1H), 4.20 (t, *J* = 9.5 Hz, 1H), 3.95 (t, *J =* 9.8 Hz, 1H), 3.85-3.76 (m, 2H), 3.74-3.65 (m, 1H), 3.65-3.41 (m, 9H), 3.31 (dd, *J* = 14.3, 7.0 Hz, 1H), 3.13 (dd, *J =* 14.2, 5.4 Hz, 1H), 3.08-2.96 (m, 1H), 2.93-2.77 (m, 4H), 2.57 (dt, *J =* 13.0, 4.5 Hz, 1H), 2.19-2.03 (m, 2H), 2.00-1.87 (m, 2H), 1.87-1.71 (m, 1H), 1.39-1.30 (m, 1H), 1.27 (d, *J =* 6.6 Hz, 3H). All exchangeable protons are missing.

### Preparation of 488

### Synthetic scheme

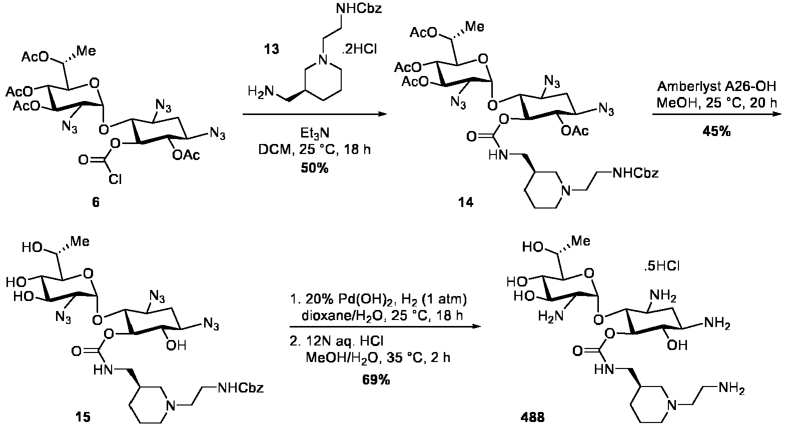

### Compound 14

### (2R,3S,4R,5R,6S)-6-(((1R,2S,3S,4R,6S)-3-acetoxy-4,6-diazido-2-(((((S)-1-(2-(((benzyloxy)carbonyl)amino)ethyl)piperidin-3-yl)methyl)carbamoyl)oxy)cyclohexyl)oxy)-2-((R)-1-acetoxyethyl)-5-azidotetrahydro-2H-pyran-3,4-diyl diacetate

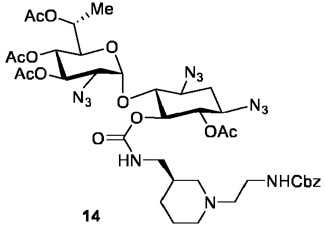

Intermediate **14** was prepared according to general procedure A, starting from intermediate 6 (675 mg, 1.04 mmol, 1 eq.) and intermediate **13** (761 mg, 2.09 mmol, 2 eq.). The crude residue was purified by silica gel flash chromatography (DCM/MeOH= from 100:0 to 90:10) to afford intermediate **14** as a brown solid (473 mg, 50%). LCMS (ESI⁺): RT= 2.93 min, [M+H]⁺=901.5 (Method 1).

### Compound 15

### (1R,2R,3S,5R,6S)-3,5-diazido-2-(((2S,3R,4R,5S,6R)-3-azido-4,5-dihydroxy-6-((R)-1-hydroxyethyl)tetrahydro-2H-pyran-2-yl)oxy)-6-hydroxycyclohexyl (((S)-1-(2-(((benzyloxy)-carbonyl)amino)ethyl)piperidin-3-yl)methyl)carbamate

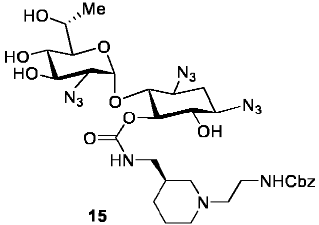

Intermediate **15** was prepared according to general procedure **B,** starting from intermediate **14** (473 mg, 0.525 mmol, 1 eq.). The crude residue was purified by preparative HPLC (column: XBridge C18 (30x150 mm (5µm)); flow Rate: 43 mL/min; gradient: Water (+0.2% NH₄HCO₃)/Acetonitrile = from 70:30 to 55:45) to afford intermediate **15** as a white solid (175 mg, 45%). LCMS (ESI⁺): RT= 7.64 min, [M+H]⁺=733.3 (Method 4).
**488**

### (1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl (((S)-1-(2-aminoethyl)-piperidin-3-yl)methyl)carbamate pentahydrochloride

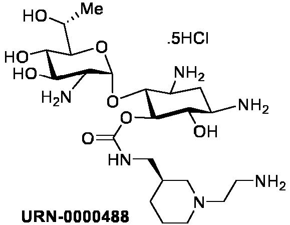

Starting from intermediate **15** (60.0 mg, 0.0819 mmol, 1 eq.), using general procedure C3, compound **488** was obtained as a white solid (40.0 mg, 69%). LCMS (ESI⁺): RT= 6.36 min, [M-5HCl+H]⁺=521.5 (Method 5). ¹H NMR (400 MHz, D₂O): *δ* 5.47 (d, *J* = 3.9 Hz, 1H), 4.96 (t, *J* = 9.4 Hz, 1H), 4.33-4.26 (m, 1H), 4.19 (t, *J* = 9.6 Hz, 1H), 3.95 (t, *J* = 9.7 Hz, 1H), 3.86-3.77 (m, 2H), 3.74-3.56 (m, 3H), 3.56-3.41 (m, 7H), 3.31 (dd, J = 14.3, 6.9 Hz, 1H), 3.14 (dd, *J =* 14.2, 5.3 Hz, 1H), 3.09-2.99 (m, 1H), 2.91-2.81 (m, 1H), 2.62-2.52 (m, 1H), 2.20-2.04 (m, 2H), 1.98-1.89 (m, 2H), 1.89-1.74 (m, 1H), 1.41-1.30 (m, 1H), 1.27 (d, *J* = 6.6 Hz, 3H). All exchangeable protons are missing.

### Preparation of 489

### Synthetic scheme

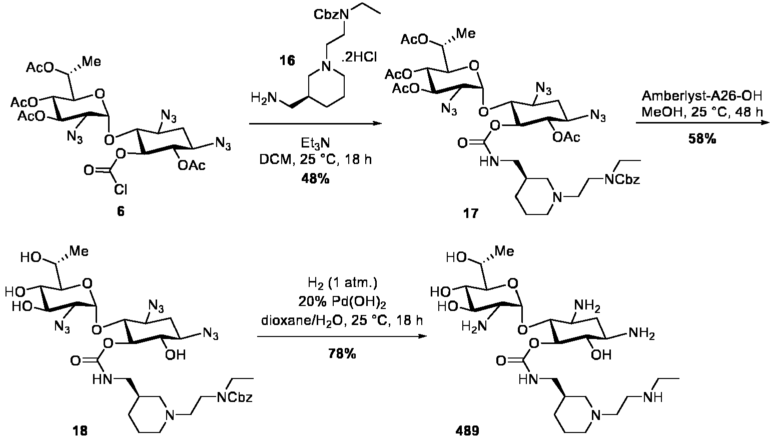

### Compound 17

### (2R,3S,4R,5R,6S)-6-(((1R,2S,3S,4R,6S)-3-acetoxy-4,6-diazido-2-(((((S)-1-(2-(((benzyloxy)carbonyl)(ethyl)amino)ethyl)piperidin-3-yl)methyl)carbamoyl)oxy)cyclohexyl)oxy)-2-((R)-1-acetoxyethyl)-5-azidotetrahydro-2H-pyran-3,4-diyl diacetate

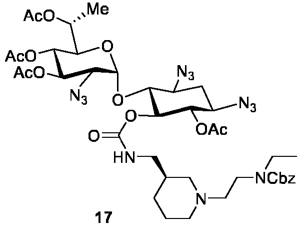

Intermediate **17** was prepared according to general procedure A, starting from intermediate **6** (885 mg, 1.37 mmol, 1 eq.) and intermediate **16** (1.08 g, 2.74 mmol, 2 eq.). The crude residue was purified by silica gel flash chromatography (DCM/EtOAc= from 100:0 to 0:100, then EtOAc/MeOH= from 100:0 to 80:20) to afford intermediate **17** as a white solid (606 mg, 48%). LCMS (ESI⁺): RT= 9.40 min, [M+H]⁺=929.5 (Method 3).

### Compound 18

### benzyl (2-((S)-3-((((((1R,2R,3S,5R,6S)-3,5-diazido-2-(((2S,3R,4R,5S,6R)-3-azido-4,5-dihydroxy-6-((R)-1-hydroxyethyl)tetrahydro-2H-pyran-2-yl)oxy)-6-hydroxycyclohexyl)oxy)carbonyl)amino)methyl)piperidin-1-yl)ethyl)(ethyl)carbamate

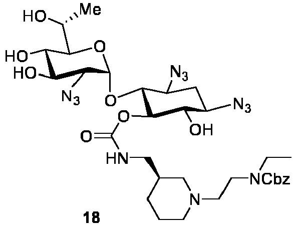

Intermediate **18** was prepared according to general procedure **B,** starting from intermediate **17** (600 mg, 0.646 mmol, 1 eq.). The crude residue was purified by preparative HPLC (column: XBridge C18 (30x150 mm (5µm)); flow Rate: 43 mL/min; gradient: Water (+0.2% NH₄HCO₃)/Acetonitrile = from 70:30 to 55:45) to afford intermediate **18** as a white solid (283 mg, 58%). LCMS (ESI⁺): RT= 9.16 min, [M+H]⁺=761.4 (Method 2).
**489**

### (1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl (((S)-1-(2-(ethylamino)-ethyl)piperidin-3-yl)methyl)carbamate

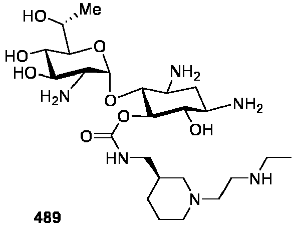

**489** was prepared according to general procedure **C1,** starting from intermediate **18** (50.0 mg, 0.0657 mmol, 1 eq.). The resulting solid was dissolved in a mixture MeOH/H₂O (1:9, 3mL) and freeze-dried for 24 h to afford compound **489** as a white solid (28.0 mg, 78%). LCMS (ESI⁺): RT= 9.01 min, [M+H]⁺=549.2 (Method 5). ¹H NMR (400 MHz, D₂O): *δ* 5.03 (d, *J =* 3.6 Hz, 1H), 4.68 (t, *J =* 9.5 Hz, 1H), 4.16 (qd, *J* = 6.6, 2.6 Hz, 1H), 3.87 (dd, *J =* 10.1, 2.6 Hz, 1H), 3.50-3.43 (m, 2H), 3.32 (t, *J =* 9.6 Hz, 1H), 3.27 (t, *J =* 9.7 Hz, 1H), 3.10-2.97 (m, 2H), 2.96-2.86 (m, 3H), 2.86-2.78 (m, 2H), 2.78-2.65 (m, 4H), 2.58-2.50 (m, 2H), 2.08-1.94 (m, 2H), 1.84-1.68 (m, 4H), 1.58-1.44 (m, 1H), 1.26-1.18 (m, 4H), 1.11 (t, *J* = 7.2 Hz, 3H), 1.05-0.91 (m, 1H). All exchangeable protons are missing.

### Preparation of 526

### Synthetic scheme

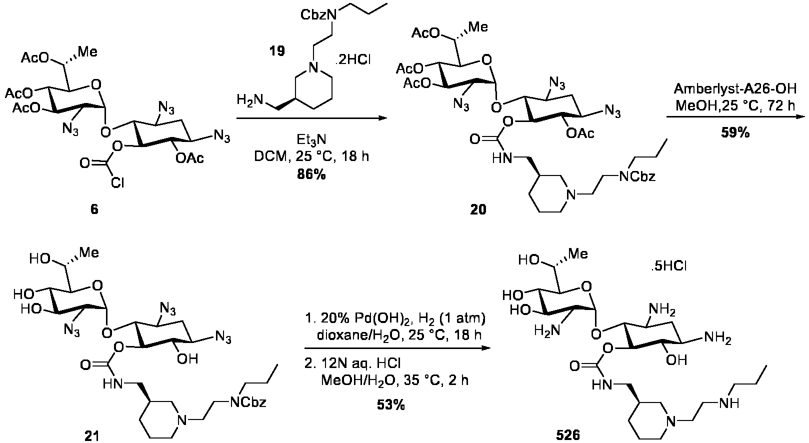

### Compound 20

### (2R,3S,4R,5R,6S)-6-(((1R,2S,3S,4R,6S)-3-acetoxy-4,6-diazido-2-(((((S)-1-(2-(((benzyloxy)carbonyl)(propyl)amino)ethyl)piperidin-3-yl)methyl)carbamoyl)oxy)cyclohexyl)oxy)-2-((R)-1-acetoxyethyl)-5-azidotetrahydro-2H-pyran-3,4-diyl diacetate

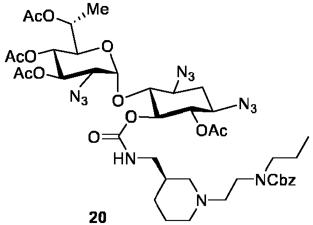

Intermediate **20** was prepared according to general procedure A, starting from intermediate **6** (490 mg, 0.759 mmol, 1 eq.) and intermediate **19** (370 mg, 0.910 mmol, 1.2 eq.). The crude residue was purified by silica gel flash chromatography (DCM/EtOAc= from 1:1 to 0:100, then EtOAc/MeOH= from 100:0 to 90:10) to afford intermediate **20** as a white solid (618 mg, 86%). LCMS (ESI⁺): RT= 3.10 min, [M+H]⁺=943.6 (Method 1).

### Compound 21

### benzyl (2-((S)-3-((((((1R,2R,3S,5R,6S)-3,5-diazido-2-(((2S,3R,4R,5S,6R)-3-azido-4,5-dihydroxy-6-((R)-1-hydroxyethyl)tetrahydro-2H-pyran-2-yl)oxy)-6-hydroxycyclohexyl)oxy)carbonyl)amino)methyl)piperidin-1-yl)ethyl)(propyl)carbamate

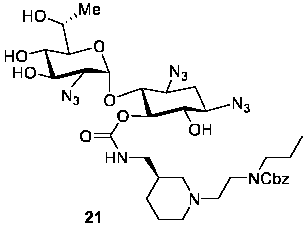

Intermediate **21** was prepared according to general procedure **B,** starting from intermediate **20** (610 mg, 0.647 mmol, 1 eq.). The crude residue was purified by preparative HPLC (column: XBridge C18 (30x150 mm (5µm)); flow Rate: 43 mL/min; gradient: Water (+0.2% NH₄HCO₃)/Acetonitrile = from 60:40 to 45:55) to afford intermediate **21** as a white solid (296 mg, 59%). LCMS (ESI⁺): RT= 9.77 min, [M+H]⁺=775.4 (Method 2).
**526**

### (1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl (((S)-1-(2-(propylamino)-ethyl)piperidin-3-yl)methyl)carbamate pentahydrochloride

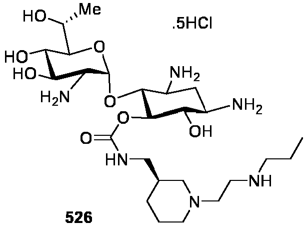

Starting from intermediate **21** (100 mg, 0.129 mmol, 1 eq.), using general procedure **C3,** compound **526** was obtained as a beige solid (50.6 mg, 53%). LCMS (ESI⁺): RT= 8.79 min, [M-5HCl+H]⁺=563.3 (Method 5). ¹H NMR (400 MHz, D₂O): *δ* 5.45 (d, *J* = 4.0 Hz, 1H), 4.95 (t, *J* = 9.3 Hz, 1H), 4.30-4.24 (m, 1H), 4.18 (t, *J* = 9.6 Hz, 1H), 3.94 (t, *J* = 9.3 Hz, 1H), 3.82-3.76 (m, 2H), 3.72-3.65 (m, 1H), 3.61-3.40 (m, 9H), 3.29 (dd, *J* = 14.3, 7.0 Hz, 1H), 3.15-3.06 (m, 3H), 3.06-2.95 (m, 1H), 2.90-2.77 (m, 1H), 2.54 (dt, *J =* 12.7, 4.3 Hz, 1H), 2.16-2.01 (m, 2H), 1.96-1.85 (m, 2H), 1.82-1.67 (m, 3H), 1.36-1.27 (m, 1H), 1.24 (d, *J =* 6.5 Hz, 3H), 0.99 (t, *J* = 7.5 Hz, 3H). All exchangeable protons are missing.

### Preparation of 538

### Synthetic scheme

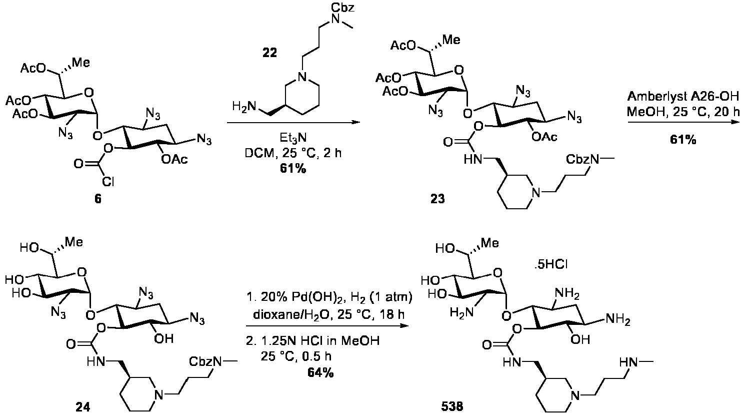

### Compound 23

### (2R,3S,4R,5R,6S)-6-(((1R,2S,3S,4R,6S)-3-acetoxy-4,6-diazido-2-(((((S)-1-(3-(((benzyloxy)carbonyl)(methyl)amino)propyl)piperidin-3-yl)methyl)carbamoyl)oxy)cyclohexyl)oxy)-2-((R)-1-acetoxyethyl)-5-azidotetrahydro-2H-pyran-3,4-diyl diacetate

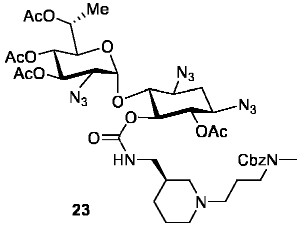

Intermediate **23** was prepared according to general procedure A, starting from intermediate **6** (3.50 g, 5.42 mmol, 1 eq.) and intermediate **22** (2.60 g, 8.13 mmol, 1.5 eq.). The crude residue was purified by silica gel flash chromatography (DCM/EtOAc= from 100:0 to 0:100, then EtOAc/MeOH= from 100:0 to 95:5) to afford intermediate **23** as a white solid (3.06 g, 61%). LCMS (ESI⁺): RT= 2.89 min, [M+H]⁺=929.6 (Method 1).

### Compound 24

### benzyl (3-((S)-3-((((((1R,2R,3S,5R,6S)-3,5-diazido-2-(((2S,3R,4R,5S,6R)-3-azido-4,5-dihydroxy-6-((R)-1-hydroxyethyl)tetrahydro-2H-pyran-2-yl)oxy)-6-hydroxycyclohexyl)oxy)carbonyl)amino)methyl)piperidin-1-yl)propyl)(methyl)carbamate

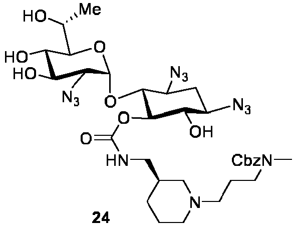

Intermediate **24** was prepared according to general procedure **B,** starting from intermediate **23** (3.06 g, 3.29 mmol, 1 eq.). The crude residue was purified by preparative HPLC (column: XBridge C18 (30x150 mm (5µm)); flow Rate: 43 mL/min; gradient: Water (+0.1% HCO₂H)/Acetonitrile = from 65:35 to 1:1) to afford intermediate **24** as a white solid (1.54 g, 61%). LCMS (ESI⁺): RT= 9.34 min, [M+H]⁺=761.4 (Method 2).
**538**

### (1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl (((S)-1-(3-(methylamino)propyl)piperidin-3-yl)methyl)carbamate pentahydrochloride

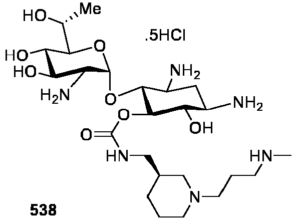

Starting from intermediate **24** (1.75 g, 2.30 mmol, 1 eq.), using general procedure **C4,** compound **538** was obtained as a beige solid (1.08 g, 64%). LCMS (ESI⁺): RT= 5.87 min, [M-5HCl+H]⁺=549.6 (Method 5).¹H NMR (400 MHz, D₂O): *δ* 5.49 (d, *J* = 4.0 Hz, 1H), 4.97 (t, *J* = 9.3 Hz, 1H), 4.30 (qd, *J* = 6.6, 2.5 Hz, 1H), 4.22 (t, *J* = 9.5 Hz, 1H), 3.98 (dd, *J =* 10.6, 8.8 Hz, 1H), 3.87-3.79 (m, 2H), 3.77-3.68 (m, 1H), 3.67-3.56 (m, 2H), 3.55-3.44 (m, 3H), 3.35-3.22 (m, 3H), 3.20-3.09 (m, 3H), 3.00-2.89 (m, 1H), 2.81-2.75 (m, 4H), 2.58 (dt, *J =* 12.8, 4.2 Hz, 1H), 2.28-2.17 (m, 2H), 2.16-2.03 (m, 2H), 2.03-1.88 (m, 2H), 1.87-1.72 (m, 1H), 1.38-1.29 (m, 1H), 1.27 (d, *J* = 6.5 Hz, 3H). All exchangeable protons are missing.

### Preparation of 563

### Synthetic scheme

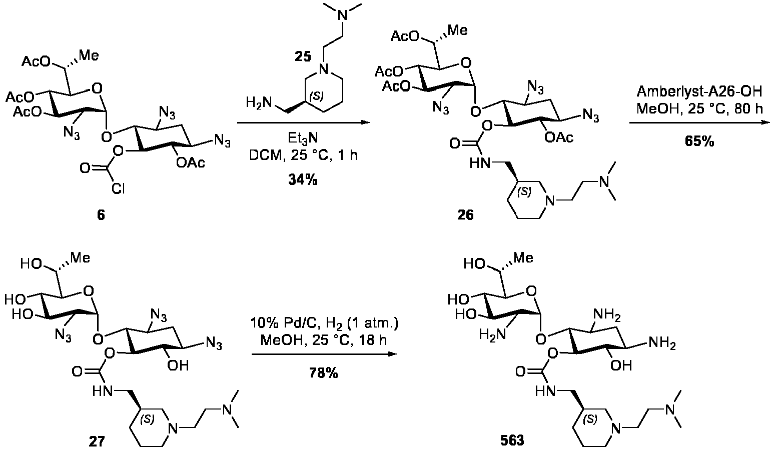

### Compound 26

### (2R,3S,4R,5R,6S)-6-(((1R,2S,3S,4R,6S)-3-acetoxy-4,6-diazido-2-(((((S)-1-(2-(dimethylamino)ethyl)piperidin-3-yl)methyl)carbamoyl)oxy)cyclohexyl)oxy)-2-((R)-1-acetoxyethyl)-5-azidotetrahydro-2H-pyran-3,4-diyl diacetate

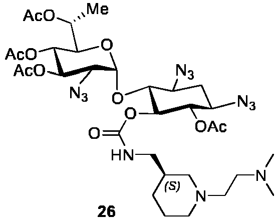

Intermediate **26** was prepared according to general procedure A, starting from intermediate **6** (581 mg, 0.899 mmol, 1 eq.) and intermediate **25** (200 mg, 1.08 mmol, 1.2 eq.). The crude residue was purified by silica gel flash chromatography (DCM/MeOH= from 100:0 to 80:20) to afford intermediate **26** as a colorless gum (244 mg, 34%). LCMS (ESI⁺): RT= 2.55 min, [M+H]⁺=795.5 (Method 1).

### Compound 27

### (1R,2R,3S,5R,6S)-3,5-diazido-2-(((2S,3R,4R,5S,6R)-3-azido-4,5-dihydroxy-6-((R)-1-hydroxyethyl)tetrahydro-2H-pyran-2-yl)oxy)-6-hydroxycyclohexyl (((S)-1-(2-(dimethylamino)ethyl)piperidin-3-yl)methyl)carbamate

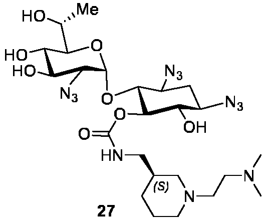

Intermediate **27** was prepared according to general procedure **B,** starting from intermediate **26** (318 mg, 0.400 mmol, 1 eq.). The crude residue was purified by preparative HPLC (column: XBridge C18 (30x150 mm (5µm)); flow Rate: 43 mL/min; gradient: Water (+0.2% NH₄HCO₃)/Acetonitrile = from 91:9 to 75:25) to afford intermediate **27** as a white solid (164 mg, 65%). LCMS (ESI⁺): RT= 8.40 min, [M+H]⁺=627.3 (Method 2).
**563**

### (1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl (((S)-1-(2-(dimethylamino)ethyl)piperidin-3-yl)methyl)carbamate

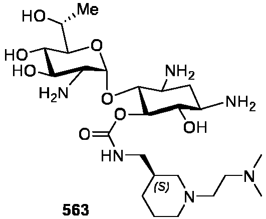

Starting from intermediate **27** (60.0 mg, 0.0957 mmol, 1 eq.), using general procedure **C2**, compound 563 was obtained as a white solid (41 mg, 78%). LCMS (ESI⁺): RT= 6.12 min, [M+H]⁺=549.6 (Method 5).¹H NMR (400 MHz, D₂O): *δ* 5.05 (d, *J =* 3.7 Hz, 1H), 4.69 (t, *J* = 9.4 Hz, 1H), 4.19 (qd, *J* = 6.6, 2.6 Hz, 1H), 3.89 (dd, *J =* 10.1, 2.7 Hz, 1H), 3.54-3.44 (m, 2H), 3.38-3.26 (m, 2H), 3.12-2.98 (m, 2H), 2.98-2.88 (m, 3H), 2.84-2.76 (m, 1H), 2.70 (dd, *J =* 10.2, 3.6 Hz, 1H), 2.63-2.51 (m, 4H), 2.28 (s, 6H), 2.11-1.97 (m, 2H), 1.89-1.69 (m, 4H), 1.59-1.46 (m, 1H), 1.28-1.18 (m, 4H), 1.06-0.94 (m, 1H). All exchangeable protons are missing.

### Preparation of 510

### Synthetic scheme

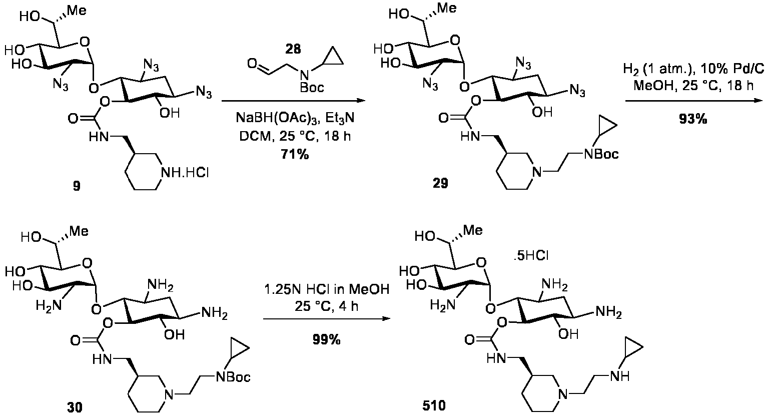

### Compound 29

### tert-butyl cyclopropyl(2-((S)-3-((((((1R,2R,3S,5R,6S)-3,5-diazido-2-(((2S,3R,4R,5S,6R)-3-azido-4,5-dihydroxy-6-((R)-1-hydroxyethyl)tetrahydro-2H-pyran-2-yl)oxy)-6-hydroxycyclohexyl)oxy)carbonyl)amino)methyl)piperidin-1-yl)ethyl)carbamate

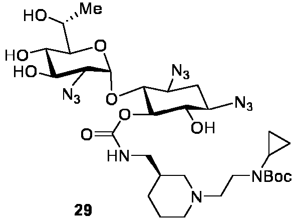

Intermediate **29** was prepared according to general procedure **E1,** starting from intermediate **9** (2.00 g, 3.38 mmol, 1 eq.) and intermediate **28** (808 mg, 4.05 mmol, 1.2 eq.). The crude residue was purified by silica gel flash chromatography (DCM/MeOH= from 100:0 to 80:20) to afford intermediate **29** as a white solid (1.76 g, 71%). LCMS (ESI⁺): RT= 7.21 min, [M+H]⁺=739.9 (Method 4).

### Compound 30

### tert-butyl cyclopropyl(2-((S)-3-((((((1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl)oxy)carbonyl)amino)methyl)piperidin-1-yl)ethyl)carbamate

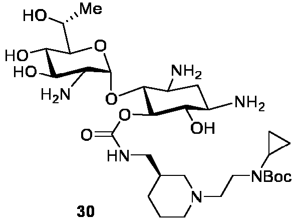

Starting from intermediate **29** (1.71 g, 2.32 mmol, 1 eq.), using general procedure **C2**, compound **30** was obtained as a white solid (1.43 g, 93%). LCMS (ESI⁺): RT= 4.39 min, [M+H]⁺=661.9 (Method 5).
**510**

### (1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl (((S)-1-(2-(cyclopropylamino)ethyl)piperidin-3-yl)methyl)carbamate pentahydrochloride

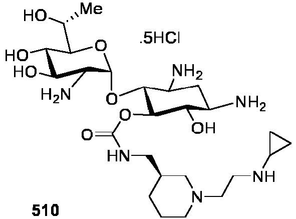

**510** was prepared according to general procedure **D1,** starting from intermediate **30** (1.39 g, 2.10 mmol, 1 eq.). The resulting solid was dissolved in H₂O (10 mL) and freeze-dried for 48 h to afford compound **510** as a white solid (1.55 g, 99%). LCMS (ESI⁺): RT= 5.67 min, [M-5HCl+H]⁺=561.7 (Method 5). ¹H NMR (400 MHz, D₂O): *δ* 5.49 (d, *J* = 4.0 Hz, 1H), 4.97 (t, *J* = 9.2 Hz, 1H), 4.35-4.26 (m, 1H), 4.22 (t, *J* = 9.6 Hz, 1H), 3.97 (dd, *J* = 10.5, 8.8 Hz, 1H), 3.87-3.78 (m, 2H), 3.76-3.56 (m, 7H), 3.55-3.44 (m, 3H), 3.33 (dd, *J* = 14.3, 6.7 Hz, 1H), 3.14 (dd, *J* = 14.3, 5.4 Hz, 1H), 3.11-3.00 (m, 1H), 2.96-2.80 (m, 2H), 2.57 (dt, *J* = 12.4, 4.2 Hz, 1H), 2.22-2.04 (m, 2H), 2.02-1.89 (m, 2H), 1.89-1.75 (m, 1H), 1.41-1.30 (m, 1H), 1.27 (d, *J* = 6.4 Hz, 3H), 1.04-0.92 (m, 4H). All exchangeable protons are missing.

### Preparation of 595

### Synthetic scheme

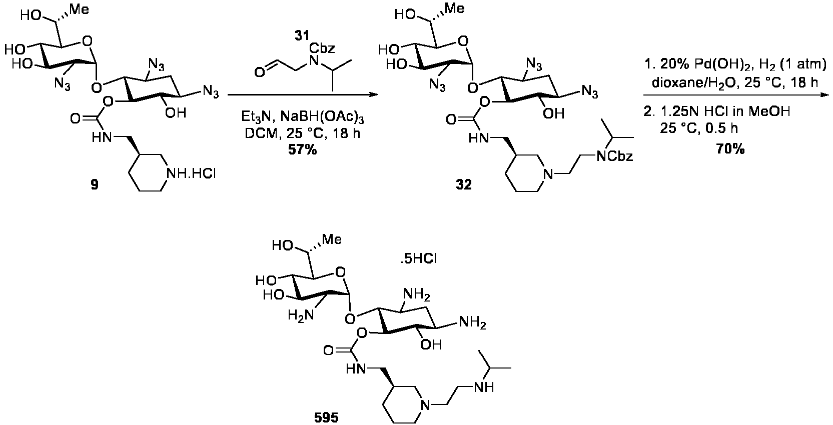

### Compound 32

### benzyl (2-((S)-3-((((((1R,2R,3S,5R,6S)-3,5-diazido-2-(((2S,3R,4R,5S,6R)-3-azido-4,5-dihydroxy-6-((R)-1-hydroxyethyl)tetrahydro-2H-pyran-2-yl)oxy)-6-hydroxycyclohexyl)oxy)carbonyl)amino)methyl)piperidin-1-yl)ethyl)(isopropyl)carbamate

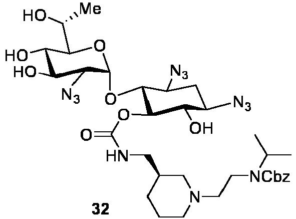

Intermediate **32** was prepared according to general procedure **E1,** starting from intermediate **9** (2.00 g, 3.38 mmol, 1 eq.) and intermediate **31** (954 mg, 4.05 mmol, 1.2 eq.). The crude residue was purified by silica gel flash chromatography (DCM/MeOH= from 100:0 to 80:20) to afford intermediate **32** as a white solid (1.49 g, 57%). LCMS (ESI⁺): RT= 7.55 min, [M+H]⁺=775.9 (Method 4).
**595**

### (1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl (((S)-1-(2-(isopropylamino)ethyl)piperidin-3-yl)methyl)carbamate pentahydrochloride

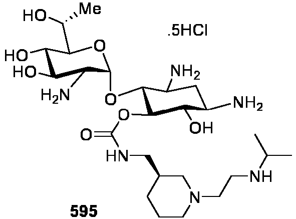

**595** was prepared according to general procedure **C4**, starting from intermediate **32** (2.00 g, 2.58 mmol, 1 eq.). The resulting solid was dissolved in H₂O (15 mL) and freeze-dried for 48 h to afford compound **595** as an off-white solid (1.34 g, 70%). LCMS (ESI⁺): RT= 5.73 min, [M-5HCl+H]⁺=563.6 (Method 5). ¹H NMR (400 MHz, D₂O): *δ* 5.49 (d, *J* = 4.0 Hz, 1H), 4.97 (t, *J* = 9.3 Hz, 1H), 4.30 (qd, *J* = 6.5, 2.5 Hz, 1H), 4.22 (t, *J =* 9.6 Hz, 1H), 3.97 (dd, *J* = 10.6, 8.8 Hz, 1H), 3.86-3.78 (m, 2H), 3.77-3.68 (m, 1H), 3.68-3.59 (m, 2H), 3.59-3.44 (m, 8H), 3.33 (dd, *J* = 14.2, 6.8 Hz, 1H), 3.14 (dd, *J* = 14.2, 5.4 Hz, 1H), 3.10-2.99 (m, 1H), 2.97-2.81 (m, 1H), 2.58 (dt, *J* = 12.7, 4.3 Hz, 1H), 2.21-2.04 (m, 2H), 2.01-1.89 (m, 2H), 1.89-1.75 (m, 1H), 1.39 (d, *J* = 6.6 Hz, 6H), 1.37-1.31 (m, 1H), 1.27 (d, *J* = 6.6 Hz, 3H). All exchangeable protons are missing.

### Preparation of 616

### Synthetic scheme

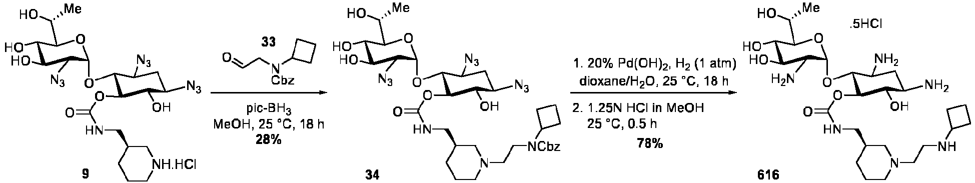

### Compound 34

### benzyl cyclobutyl(2-((S)-3-((((((1R,2R,3S,5R,6S)-3,5-diazido-2-(((2S,3R,4R,5S,6R)-3-azido-4,5-dihydroxy-6-((R)-1-hydroxyethyl)tetrahydro-2H-pyran-2-yl)oxy)-6-hydroxycyclohexyl)oxy)carbonyl)amino)methyl)piperidin-1-yl)ethyl)carbamate

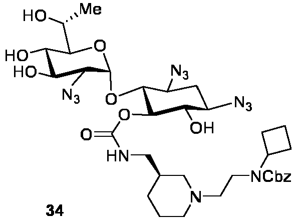

Intermediate **34** was prepared according to general procedure **E2**, starting from intermediate **9** (137 mg, 0.231 mmol, 1 eq.) and intermediate **33** (57.5 mg, 0.231 mmol, 1 eq.). The crude residue was purified by silica gel flash chromatography (DCM/EtOAc= from 100:0 to 0:100, then EtOAc/MeOH= from 100:0 to 80:20) to afford impure compound **34** (95 mg). Impure **34** was then purified by preparative HPLC (column: XBridge C18 (30x150 mm (5µm)); flow Rate: 43 mL/min; gradient: Water (+0.2% NH₄HCO₃)/Acetonitrile = from 60:40 to 45:55) to afford pure intermediate **34** as a white solid (52 mg, 28%). LCMS (ESI⁺): RT= 9.02 min, [M+H]⁺=787.9 (Method 2).
**616**

### (1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl (((S)-1-(2-(cyclobutylamino)ethyl)piperidin-3-yl)methyl)carbamate pentahydrochloride

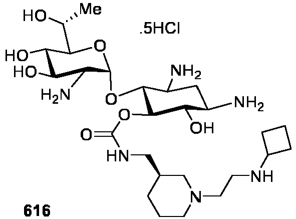

Starting from intermediate **34** (52.0 mg, 0.0661 mmol, 1 eq.), using general procedure **C4**, **616** was obtained as a white solid (39.0 mg, 78%). LCMS (ESI⁺): RT= 5.49 min, [M-5HCl+H]⁺=575.8 (Method 5).¹H NMR (400 MHz, D₂O): *δ* 5.48 (d, *J* = 4.1 Hz, 1H), 4.97 (t, *J* = 9.3 Hz, 1H), 4.35-4.25 (m, 1H), 4.20 (t, *J =* 9.6 Hz, 1H), 3.96 (dd, *J* = 10.6, 8.8 Hz, 1H), 3.92-3.77 (m, 3H), 3.75-3.67 (m, 1H), 3.65-3.39 (m, 9H), 3.31 (dd, *J* = 14.2, 6.8 Hz, 1H), 3.13 (dd, *J* = 14.2, 5.3 Hz, 1H), 3.08-2.97 (m, 1H), 2.92-2.79 (m, 1H), 2.57 (dt, *J* = 12.7, 4.3 Hz, 1H), 2.43-2.33 (m, 2H), 2.28-2.16 (m, 2H), 2.16-2.03 (m, 2H), 2.01-1.86 (m, 4H), 1.86-1.75 (m, 1H), 1.40-1.30 (m, 1H), 1.27 (d, *J* = 6.6 Hz, 3H). All exchangeable protons are missing.

### Preparation of 682

### Synthetic scheme

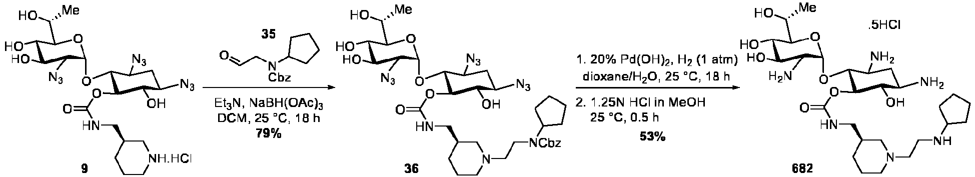

### Compound 36

### benzyl cyclopentyl(2-((S)-3-((((((1R,2R,3S,5R,6S)-3,5-diazido-2-(((2S,3R,4R,5S,6R)-3-azido-4,5-dihydroxy-6-((R)-1-hydroxyethyl)tetrahydro-2H-pyran-2-yl)oxy)-6-hydroxycyclohexyl)oxy)carbonyl)amino)methyl)piperidin-1-yl)ethyl)carbamate

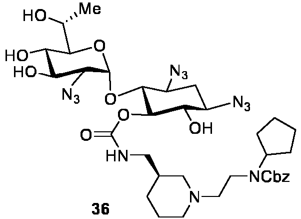

Intermediate **36** was prepared according to general procedure **E1,** starting from intermediate **9** (296 mg, 0.500 mmol, 1 eq.) and intermediate **35** (196 mg, 0.750 mmol, 1.5 eq.). The crude residue was purified by preparative HPLC (column: XBridge C18 (30x150 mm (5µm)); flow Rate: 43 mL/min; gradient: Water (+0.2% NH₄HCO₃)/Acetonitrile = from 60:40 to 45:55) to afford intermediate **36** as a white solid (316 mg, 79%). LCMS (ESI⁺): RT= 9.28 min, [M+H]⁺=801.9 (Method 2).
**682**

### (1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl (((S)-1-(2-(cyclopentylamino)ethyl)piperidin-3-yl)methyl)carbamate pentahydrochloride

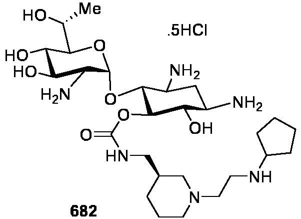

Starting from intermediate **36** (70.0 mg, 0.0875 mmol, 1 eq.), using general procedure **C4**, **682** was obtained as a white solid (36.0 mg, 53%). LCMS (ESI⁺): RT= 5.35 min, [M-5HCl+H]⁺=589.9 (Method 5).¹H NMR (400 MHz, D₂O): *δ* 5.47 (d, *J* = 4.0 Hz, 1H), 4.97 (t, *J* = 9.3 Hz, 1H), 4.34-4.27 (m, 1H), 4.20 (t, *J = 9.6* Hz, 1H), 3.96 (dd, *J* = 10.6, 8.8 Hz, 1H), 3.86-3.78 (m, 2H), 3.75-3.65 (m, 2H), 3.65-3.57 (m, 2H), 3.56-3.50 (m, 5H), 3.50-3.44 (m, 2H), 3.32 (dd, *J* = 14.2, 6.9 Hz, 1H), 3.14 (dd, *J* = 14.2, 5.4 Hz, 1H), 3.09-2.98 (m, 1H), 2.92-2.81 (m, 1H), 2.57 (dt, *J* = 12.6, 4.2 Hz, 1H), 2.22-2.04 (m, 4H), 1.99-1.88 (m, 2H), 1.86-1.75 (m, 3H), 1.75-1.65 (m, 4H), 1.39-1.29 (m, 1H), 1.27 (d, *J* = 6.6 Hz, 3H). All exchangeable protons are missing.

### Preparation of 685

### Synthetic scheme

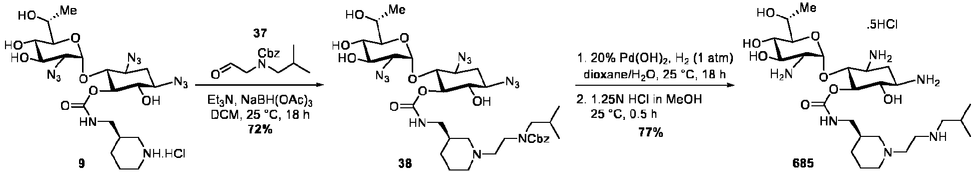

### Compound 38

### benzyl (2-((S)-3-((((((1R,2R,3S,5R,6S)-3,5-diazido-2-(((2S,3R,4R,5S,6R)-3-azido-4,5-dihydroxy-6-((R)-1-hydroxyethyl)tetrahydro-2H-pyran-2-yl)oxy)-6-hydroxycyclohexyl)oxy)carbonyl)amino)methyl)piperidin-1-yl)ethyl)(isobutyl)carbamate

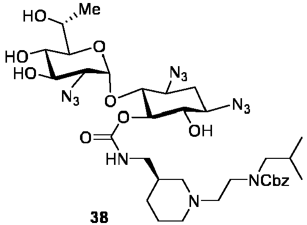

Intermediate **38** was prepared according to general procedure **E1,** starting from intermediate **9** (150 mg, 0.253 mmol, 1 eq.) and intermediate **37** (75.8 mg, 0.304 mmol, 1.2 eq.). The crude residue was purified by silica gel flash chromatography (DCM/MeOH= from 100:0 to 80:20) to afford intermediate **38** as a white solid (143 mg, 72%). LCMS (ESI⁺): RT= 7.94 min, [M+H]⁺=789.9 (Method 4).
**685**

### (1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl (((S)-1-(2-(isobutylamino)ethyl)piperidin-3-yl)methyl)carbamate pentahydrochloride

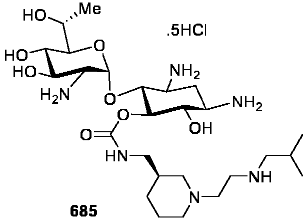

Starting from intermediate **38** (70.0 mg, 0.0887 mmol, 1 eq.), using general procedure C4, **685** was obtained as a white solid (52.0 mg, 77%). LCMS (ESI⁺): RT= 5.40 min, [M-5HCl+H]⁺=577.9 (Method 5).¹H NMR (400 MHz, D₂O): *δ* 5.48 (d, *J* = 4.0 Hz, 1H), 4.97 (t, *J* = 9.3 Hz, 1H), 4.30 (qd, *J* = 6.6, 2.4 Hz, 1H), 4.20 (t, *J = 9.6* Hz, 1H), 3.96 (dd, *J* = 10.6, 8.9 Hz, 1H), 3.85-3.78 (m, 2H), 3.75-3.67 (m, 1H), 3.66-3.54 (m, 6H), 3.54-3.44 (m, 3H), 3.32 (dd, *J* = 14.3, 6.9 Hz, 1H), 3.14 (dd, *J* = 14.3, 5.4 Hz, 1H), 3.09-2.97 (m, 3H), 2.93-2.80 (m, 1H), 2.57 (dt, *J* = 12.7, 4.3 Hz, 1H), 2.21-2.01 (m, 3H), 2.00-1.88 (m, 2H), 1.87-1.74 (m, 1H), 1.41-1.29 (m, 1H), 1.27 (d, *J* = 6.6 Hz, 3H), 1.04 (d, *J* = 6.7 Hz, 6H). All exchangeable protons are missing.

### Preparation of 686

### Synthetic scheme

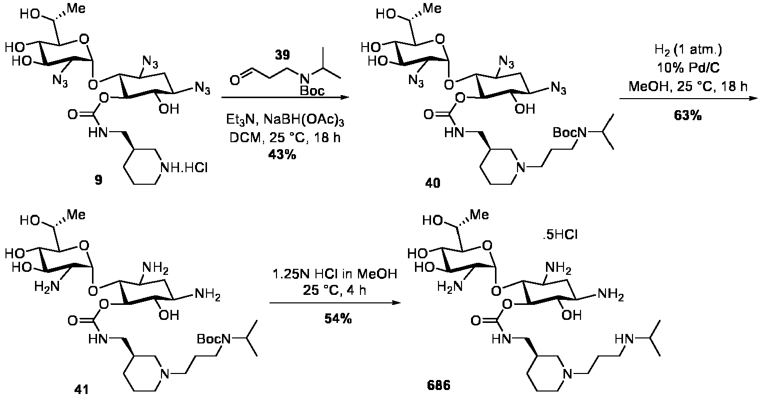

### Compound 40

### tert-butyl (3-((S)-3-((((((1R,2R,3S,5R,6S)-3,5-diazido-2-(((2S,3R,4R,5S,6R)-3-azido-4,5-dihydroxy-6-((R)-1-hydroxyethyl)tetrahydro-2H-pyran-2-yl)oxy)-6-hydroxycyclohexyl)oxy)carbonyl)amino)methyl)piperidin-1-yl)propyl)(isopropyl)carbamate or ??

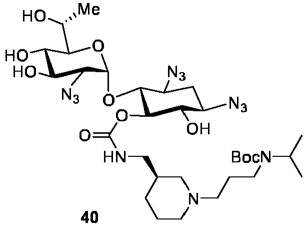

Intermediate **40** was prepared according to general procedure **E1,** starting from intermediate **9** (130 mg, 0.220 mmol, 1 eq.) and intermediate **39** (56.7 mg, 0.264 mmol, 1.2 eq.). The crude residue was purified by silica gel flash chromatography (DCM/MeOH= from 100:0 to 80:20) to afford intermediate **40** as a pale yellow solid (71.0 mg, 43%). LCMS (ESI⁺): RT= 8.25 min, [M+H]⁺=756.0 (Method 2).

### Compound 41

### tert-butyl (3-((S)-3-((((((1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl)oxy)carbonyl)amino)methyl)piperidin-1-yl)propyl)(isopropyl)carbamate

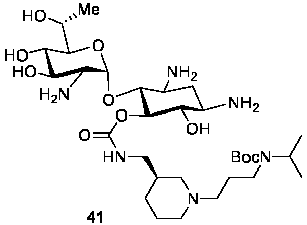

Starting from intermediate **40** (71.0 mg, 0.0941 mmol, 1 eq.), using general procedure **C2,** compound **41** was obtained as a white solid (40.0 mg, 63%). LCMS (ESI⁺): RT= 4.30 min, [M+H]⁺=678.0 (Method 5).
**686**

### (1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl (((S)-1-(3-(isopropylamino)propyl)piperidin-3-yl)methyl)carbamate pentahydrochloride

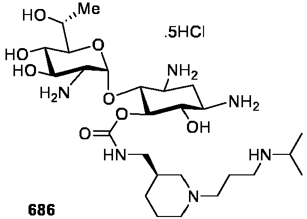

Starting from intermediate **41** (35.0 mg, 0.0517 mmol, 1 eq.), using general procedure **D1, 686** was obtained as a white solid (21.0 mg, 54%). LCMS (ESI⁺): RT= 5.55 min, [M-5HCl+H]⁺=577.8 (Method 5).¹H NMR (400 MHz, D₂O): *δ* 5.48 (d, *J* = 4.0 Hz, 1H), 4.97 (t, *J* = 9.3 Hz, 1H), 4.30 (qd, *J* = 6.6, 2.6 Hz, 1H), 4.20 (t, *J = 9.6* Hz, 1H), 3.96 (dd, *J* = 10.6, 8.8 Hz, 1H), 3.85-3.79 (m, 2H), 3.71 (ddd, *J* = 12.7, 10.0, 4.5 Hz, 1H), 3.67-3.55 (m, 2H), 3.52-3.43 (m, 4H), 3.34-3.22 (m, 3H), 3.20-3.10 (m, 3H), 2.99-2.89 (m, 1H), 2.83-2.71 (m, 1H), 2.57 (dt, *J* = 12.8, 4.3 Hz, 1H), 2.23-2.04 (m, 4H), 1.99-1.88 (m, 2H), 1.86-1.72 (m, 1H), 1.36 (d, *J =* 6.6 Hz, 6H), 1.34-1.29 (m, 1H), 1.27 (d, *J* = 6.6 Hz, 3H). All exchangeable protons are missing.

### Preparation of 687

### Synthetic scheme

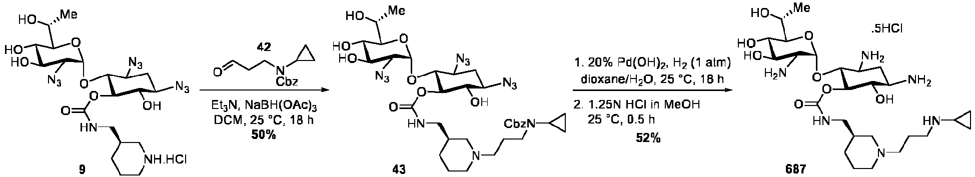

### Compound 43

### benzyl cyclopropyl(3-((S)-3-((((((1R,2R,3S,5R,6S)-3,5-diazido-2-(((2S,3R,4R,5S,6R)-3-azido-4,5-dihydroxy-6-((R)-1-hydroxyethyl)tetrahydro-2H-pyran-2-yl)oxy)-6-hydroxycyclohexyl)oxy)carbonyl)amino)methyl)piperidin-1-yl)propyl)carbamate

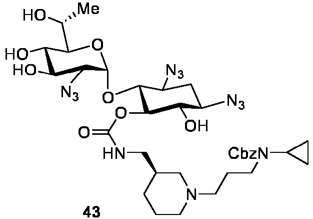

Intermediate **43** was prepared according to general procedure **E1,** starting from intermediate **9** (130 mg, 0.220 mmol, 1 eq.) and intermediate **42** (65.2 mg, 0.264 mmol, 1.2 eq.). The crude residue was purified by silica gel flash chromatography (DCM/MeOH= from 100:0 to 80:20) to afford intermediate **43** as a white solid (86 mg, 50%). LCMS (ESI⁺): RT= 8.29 min, [M+H]⁺=788.0 (Method 2).
**687**

### (1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl (((S)-1-(3-(cyclopropylamino)propyl)piperidin-3-yl)methyl)carbamate pentahydrochloride

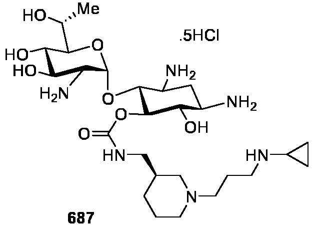

Starting from intermediate **43** (80.0 mg, 0.102 mmol, 1 eq.), using general procedure **C4, 687** was obtained as a white solid (40.0 mg, 52%). LCMS (ESI⁺): RT= 5.63 min, [M-5HCl+H]⁺=575.8 (Method 5).¹H NMR (400 MHz, D₂O): *δ* 5.48 (d, *J* = 4.0 Hz, 1H), 4.97 (t, *J* = 9.3 Hz, 1H), 4.30 (qd, *J* = 6.5, 2.4 Hz, 1H), 4.20 (t, *J* = 9.6 Hz, 1H), 3.96 (dd, *J* = 10.6, 8.8 Hz, 1H), 3.85-3.78 (m, 2H), 3.75-3.67 (m, 1H), 3.67-3.56 (m, 2H), 3.54-3.43 (m, 3H), 3.34-3.23 (m, 5H), 3.14 (dd, *J* = 14.2, 5.4 Hz, 1H), 3.00-2.88 (m, 1H), 2.84-2.73 (m, 2H), 2.57 (dt, *J* = 12.6, 4.2 Hz, 1H), 2.28-2.18 (m, 2H), 2.17-2.02 (m, 2H), 2.00-1.87 (m, 2H), 1.87-1.70 (m, 1H), 1.38-1.29 (m, 1H), 1.27 (d, *J* = 6.6 Hz, 3H), 1.01-0.88 (m, 4H). All exchangeable protons are missing.

### Preparation of 690

### Synthetic scheme

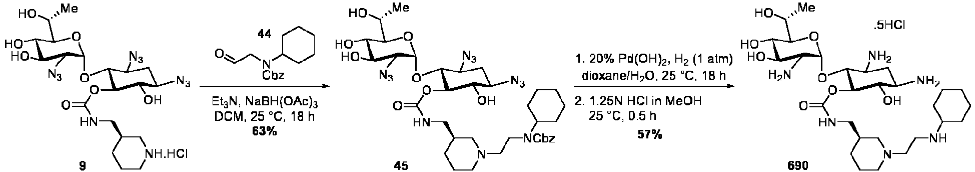

### Compound 45

### benzyl cyclohexyl(2-((S)-3-((((((1R,2R,3S,5R,6S)-3,5-diazido-2-(((2S,3R,4R,5S,6R)-3-azido-4,5-dihydroxy-6-((R)-1-hydroxyethyl)tetrahydro-2H-pyran-2-yl)oxy)-6-hydroxycyclohexyl)oxy)carbonyl)amino)methyl)piperidin-1-yl)ethyl)carbamate

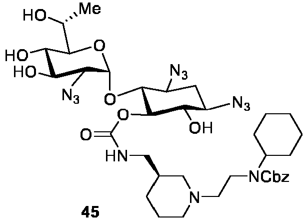

Intermediate **45** was prepared according to general procedure **E1,** starting from intermediate **9** (273 mg, 0.461 mmol, 1 eq.) and intermediate **44** (191 mg, 0.692 mmol, 1.5 eq.). The crude residue was purified by preparative HPLC (column: XBridge C18 (30x150 mm (5µm)); flow Rate: 43 mL/min; gradient: Water (+0.2% NH₄HCO₃)/Acetonitrile = from 55:45 to 40:60) to afford intermediate **45** as a white solid (236 mg, 63%). LCMS (ESI⁺): RT= 9.57 min, [M+H]⁺=815.9 (Method 2).
**690**

### (1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl (((S)-1-(2-(cyclohexylamino)ethyl)piperidin-3-yl)methyl)carbamate pentahydrochloride

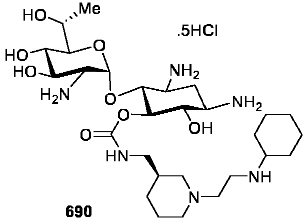

Starting from intermediate **45** (60.0 mg, 0.0736 mmol, 1 eq.), using general procedure **C4, 690** was obtained as a white solid (33.0 mg, 57%). LCMS (ESI⁺): RT= 5.25 min, [M-5HCl+H]⁺=603.9 (Method 5).¹H NMR (400 MHz, D₂O): *δ* 5.48 (d, *J* = 4.0 Hz, 1H), 4.97 (t, *J* = 9.3 Hz, 1H), 4.30 (qd, *J* = 6.4, 2.4 Hz, 1H), 4.20 (t, *J* = 9.5 Hz, 1H), 3.96 (dd, *J* = 10.6, 8.8 Hz, 1H), 3.85-3.78 (m, 2H), 3.75-3.67 (m, 1H), 3.66-3.44 (m, 10H), 3.31 (dd, *J* = 14.2, 7.0 Hz, 1H), 3.27-3.19 (m, 1H), 3.14 (dd, *J* = 14.2, 5.4 Hz, 1H), 3.09-2.98 (m, 1H), 2.93-2.81 (m, 1H), 2.57 (dt, *J* = 12.6, 4.3 Hz, 1H), 2.19-2.04 (m, 4H), 1.99-1.77 (m, 5H), 1.74-1.67 (m, 1H), 1.48-1.29 (m, 5H), 1.27 (d, *J* = 6.6 Hz, 3H). All exchangeable protons are missing.

### Preparation of 717

### Synthetic scheme

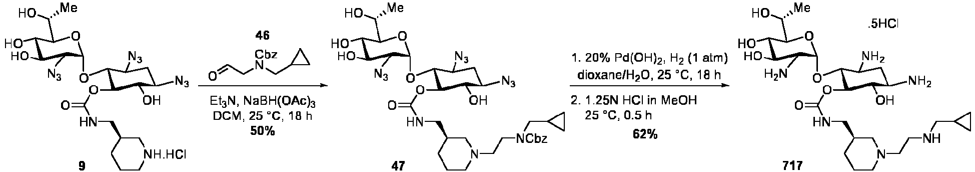

### Compound 47

### benzyl (cyclopropylmethyl)(2-((S)-3-((((((1R,2R,3S,5R,6S)-3,5-diazido-2-(((2S,3R,4R,5S,6R)-3-azido-4,5-dihydroxy-6-((R)-1-hydroxyethyl)tetrahydro-2H-pyran-2-yl)oxy)-6-hydroxycyclohexyl)oxy)carbonyl)amino)methyl)piperidin-1-yl)ethyl)carbamate

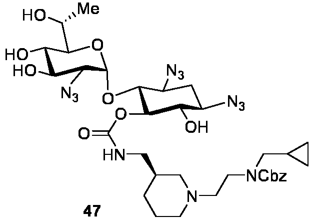

Intermediate **47** was prepared according to general procedure **E1,** starting from intermediate **9** (150 mg, 0.253 mmol, 1 eq.) and intermediate **46** (94.0 mg, 0.380 mmol, 1.5 eq.). The crude residue was purified by silica gel flash chromatography (DCM/MeOH= from 100:0 to 80:20) to afford intermediate **47** as a white solid (99.0 mg, 50%). LCMS (ESI⁺): RT= 8.93 min, [M+H]⁺=787.8 (Method 2).
**717**

### (1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl (((S)-1-(2-((cyclopropylmethyl)amino)ethyl)piperidin-3-yl)methyl)carbamate pentahydrochloride

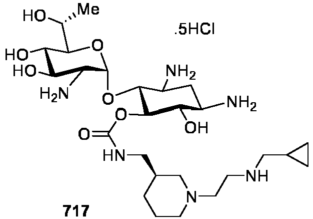

Starting from intermediate **47** (60.0 mg, 0.0763 mmol, 1 eq.), using general procedure **C4, 717** was obtained as a white solid (36.0 mg, 62%). LCMS (ESI⁺): RT= 5.53 min, [M-5HCl+H]⁺=575.7 (Method 5).¹H NMR (400 MHz, D₂O): *δ* 5.47 (d, *J* = 4.0 Hz, 1H), 4.97 (t, *J* = 9.3 Hz, 1H), 4.30 (qd, *J* = 6.4, 2.5 Hz, 1H), 4.19 (t, *J* = 9.5 Hz, 1H), 3.95 (dd, *J* = 10.3, 9.1 Hz, 1H), 3.85-3.77 (m, 2H), 3.74-3.66 (m, 1H), 3.66-3.50 (m, 7H), 3.50-3.43 (m, 2H), 3.31 (dd, *J* = 14.2, 7.0 Hz, 1H), 3.14 (dd, *J* = 14.3, 5.3 Hz, 1H), 3.07 (d, *J* = 7.6 Hz, 2H), 3.05-2.96 (m, 1H), 2.92-2.80 (m, 1H), 2.57 (dt, *J* = 12.5, 4.4 Hz, 1H), 2.21-2.03 (m, 2H), 1.99-1.87 (m, 2H), 1.87-1.73 (m, 1H), 1.41-1.30 (m, 1H), 1.27 (d, *J* = 6.5 Hz, 3H), 1.18-1.09 (m, 1H), 0.79-0.72 (m, 2H), 0.45-0.39 (m, 2H). All exchangeable protons are missing.

### Preparation of 832

### Synthetic scheme

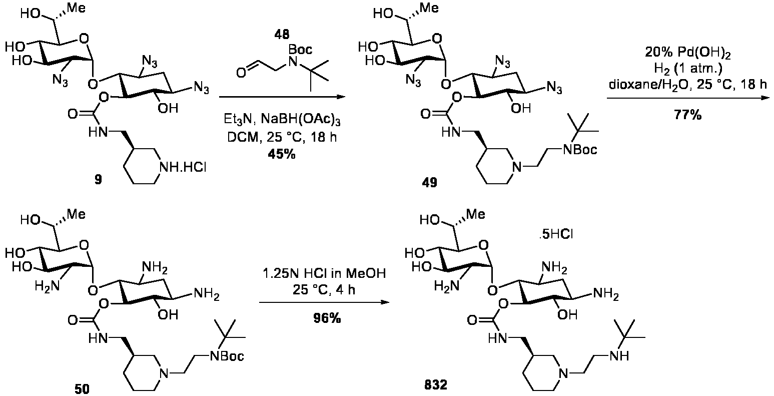

### Compound 49

### tert-butyl tert-butyl(2-((S)-3-((((((1R,2R,3S,5R,6S)-3,5-diazido-2-(((2S,3R,4R,5S,6R)-3-azido-4,5-dihydroxy-6-((R)-1-hydroxyethyl)tetrahydro-2H-pyran-2-yl)oxy)-6-hydroxycyclohexyl)oxy)carbonyl)amino)methyl)piperidin-1-yl)ethyl)carbamate

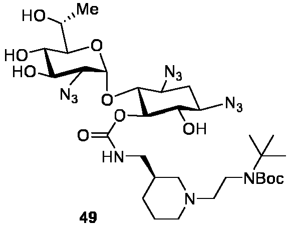

Intermediate **49** was prepared according to general procedure **E1,** starting from intermediate **9** (207 mg, 0.350 mmol, 1 eq.) and intermediate **48** (90 mg, 0.420 mmol, 1.2 eq.). The crude residue was purified by silica gel flash chromatography (DCM/MeOH= from 100:0 to 80:20) to afford intermediate **49** as a white solid (120 mg, 45%). LCMS (ESI⁺): RT= 9.04 min, [M+H]⁺=755.8 (Method 2).

### Compound 50

### tert-butyl tert-butyl(2-((S)-3-((((((1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl)oxy)carbonyl)amino)methyl)piperidin-1-yl)ethyl)carbamate

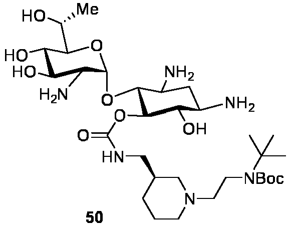

Starting from intermediate **49** (40.0 mg, 0.0530 mmol, 1 eq.), using general procedure **C1,** compound **50** was obtained as a white solid (28.0 mg, 77%). LCMS (ESI⁺): RT= 4.33 min, [M+H]⁺=677.7 (Method 5).
**832**

### (1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl (((S)-1-(2-(tert-butylamino)ethyl)piperidin-3-yl)methyl)carbamate pentahydrochloride

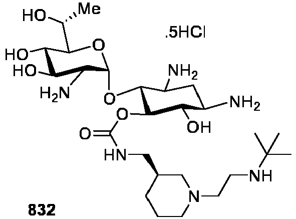

Starting from intermediate **50** (26.0 mg, 0.0384 mmol, 1 eq.), using general procedure **D1, 832** was obtained as a white solid (28.0 mg, 96%). LCMS (ESI⁺): RT= 5.57 min, [M-5HCl+H]⁺=577.7 (Method 5).¹H NMR (400 MHz, D₂O): *δ* 5.47 (d, *J =* 4.1 Hz, 1H), 4.97 (t, *J* = 9.3 Hz, 1H), 4.30 (qd, *J* = 6.6, 2.8 Hz, 1H), 4.19 (t, *J = 9.6* Hz, 1H), 3.95 (dd, *J* = 10.2, 9.2 Hz, 1H), 3.85-3.77 (m, 2H), 3.75-3.55 (m, 3H), 3.55-3.43 (m, 7H), 3.31 (dd, *J* = 14.3, 7.0 Hz, 1H), 3.14 (dd, *J* = 14.2, 5.3 Hz, 1H), 3.09-2.97 (m, 1H), 2.92-2.81 (m, 1H), 2.57 (dt, *J* = 12.3, 4.1 Hz, 1H), 2.19-2.04 (m, 2H), 1.99-1.87 (m, 2H), 1.87-1.74 (m, 1H), 1.44 (s, 9H), 1.39-1.30 (m, 1H), 1.27 (d, *J* = 6.5 Hz, 3H). All exchangeable protons are missing.

### Preparation of 511

### Synthetic scheme

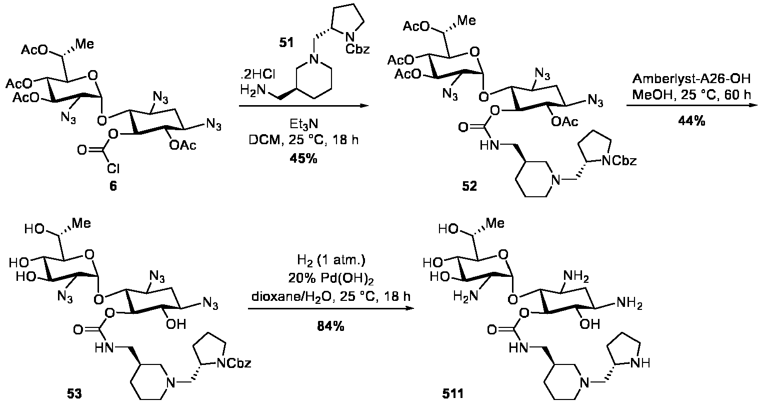

### Compound 52

### (2R,3S,4R,5R,6S)-6-(((1R,2S,3S,4R,6S)-3-acetoxy-4,6-diazido-2-(((((S)-1-(((S)-1-((benzyloxy)carbonyl)pyrrolidin-2-yl)methyl)piperidin-3-yl)methyl)carbamoyl)oxy)cyclohexyl)oxy)-2-((R)-1-acetoxyethyl)-5-azidotetrahydro-2H-pyran-3,4-diyl diacetate

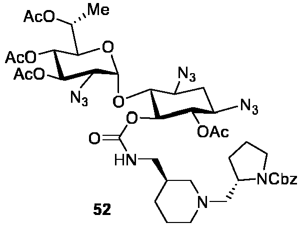

Intermediate **52** was prepared according to general procedure **A**, starting from intermediate **6** (610 mg, 0.944 mmol, 1 eq.) and intermediate **51** (764 mg, 1.89 mmol, 2 eq.). The crude residue was purified by silica gel flash chromatography (DCM/MeOH= from 100:0 to 92:8) to afford intermediate **52** as an off-white solid (404 mg, 45%). LCMS (ESI⁺): RT= 2.91 min, [M+H]⁺=941.6 (Method 1).

### Compound 53

### benzyl (S)-2-(((iS)-3-((((((1R,2R,3S,5R,6S)-3,5-diazido-2-(((2S,3R,4R,5S,6R)-3-azido-4,5-dihydroxy-6-((R)-1-hydroxyethyl)tetrahydro-2H-pyran-2-yl)oxy)-6-hydroxycyclohexyl)oxy)carbonyl)amino)methyl)piperidin-1-yl)methyl)pyrrolidine-1-carboxylate

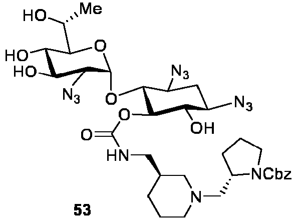

Intermediate **53** was prepared according to general procedure **B**, starting from intermediate **52** (404 mg, 0.429 mmol, 1 eq.). The crude residue was purified by preparative HPLC (column: XBridge C18 (30x150 mm (5µm)); flow Rate: 43 mL/min; gradient: Water (+0.2% NH₄HCO₃)/Acetonitrile = from 72:28 to 56:44) to afford intermediate **53** as a white solid (145 mg, 44%). LCMS (ESI⁺): RT= 8.26 min, [M+H]⁺=773.3 (Method 4).
**511**

### (1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl (((S)-1-(((S)-pyrrolidin-2-yl)methyl)piperidin-3-yl)methyl)carbamate

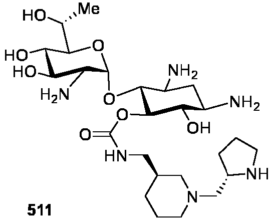

Starting from intermediate **53** (52.0 mg, 0.0673 mmol, 1 eq.), using general procedure **C1**, **511** was obtained as an off-white solid (31.7 mg, 84%). LCMS (ESI⁺): RT= 9.12 min, [M+H]⁺=561.3 (Method 5). ¹H NMR (400 MHz, D₂O): *δ* 5.03 (d, *J =* 3.7 Hz, 1H), 4.68 (t, *J* = 9.5 Hz, 1H), 4.16 (qd, *J* = 6.6, 2.6 Hz, 1H), 3.87 (dd, *J* = 10.1, 2.6 Hz, 1H), 3.50-3.37 (m, 3H), 3.32 (dd, *J* = 10.1, 9.0 Hz, 1H), 3.27 (t, *J* = 9.8 Hz, 1H), 3.10-2.98 (m, 3H), 2.98-2.87 (m, 4H), 2.82-2.72 (m, 1H), 2.67 (dd, *J* = 10.2, 3.6 Hz, 1H), 2.54 (d, *J* = 6.5 Hz, 2H), 2.08-1.95 (m, 3H), 1.90-1.68 (m, 6H), 1.60-1.38 (m, 2H), 1.28-1.15 (m, 4H), 1.05-0.92 (m, 1H). All exchangeable protons are missing.

### Preparation of 525

### Synthetic scheme

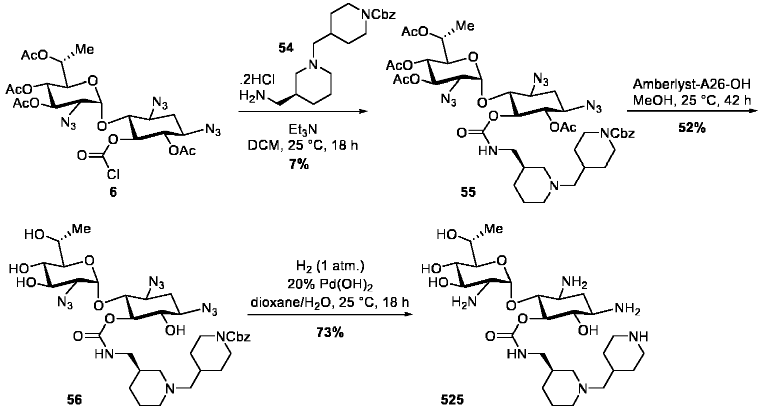

### Compound 55

### (2R,3S,4R,5R,6S)-6-(((1R,2S,3S,4R,6S)-3-acetoxy-4,6-diazido-2-(((((S)-1-((1-((benzyloxy)carbonyl)piperidin-4-yl)methyl)piperidin-3-yl)methyl)carbamoyl)oxy)cyclohexyl)oxy)-2-((R)-1-acetoxyethyl)-5-azidotetrahydro-2H-pyran-3,4-diyl diacetate

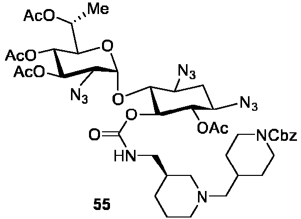

Intermediate **55** was prepared according to general procedure **A,** starting from intermediate **6** (1.18 g, 1.83 mmol, 1 eq.) and intermediate **54** (1.53 g, 3.65 mmol, 2 eq.). The crude residue was purified by silica gel flash chromatography (DCM/EtOAc= from 100:0 to 0:100 then EtOAc/MeOH= from 100:0 to 90:10) to afford intermediate **55** as a white solid (130 mg, 7%). LCMS (ESI⁺): RT= 2.90 min, [M+H]⁺=955.7 (Method 1).

### Compound 56

### benzyl 4-(((S)-3-((((((1R,2R,3S,5R,6S)-3,5-diazido-2-(((2S,3R,4R,5S,6R)-3-azido-4,5-dihydroxy-6-((R)-1-hydroxyethyl)tetrahydro-2H-pyran-2-yl)oxy)-6-hydroxycyclohexyl)oxy)carbonyl)amino)methyl)piperidin-1-yl)methyl)piperidine-1-carboxylate

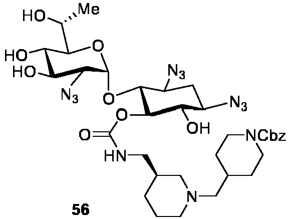

Intermediate **56** was prepared according to general procedure **B**, starting from intermediate **55** (130 mg, 0.136 mmol, 1 eq.). The crude residue was purified by preparative HPLC (column: XBridge C18 (30x150 mm (5µm)); flow Rate: 43 mL/min; gradient: Water (+0.2% NH₄HCO₃)/Acetonitrile = from 65:35 to 50:50) to afford intermediate **56** as a white solid (56.0 mg, 52%). LCMS (ESI⁺): RT= 7.68 min, [M+H]⁺=787.4 (Method 3).
**525**

### (1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl (((S)-1-(piperidin-4-ylmethyl)piperidin-3-yl)methyl)carbamate

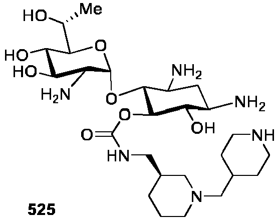

Starting from intermediate **56** (56.0 mg, 0.0712 mmol, 1 eq.), using general procedure **C1, 525** was obtained as a white solid (30.0 mg, 73%). LCMS (ESI⁺): RT= 8.99 min, [M+H]⁺=575.3 (Method 5). ¹H NMR (400 MHz, D₂O): *δ* 5.03 (d, *J* = 3.6 Hz, 1H), 4.68 (t, *J* = 9.7 Hz, 1H), 4.16 (qd, *J* = 6.7, 2.6 Hz, 1H), 3.86 (dd, *J* = 10.1, 2.6 Hz, 1H), 3.50-3.43 (m, 2H), 3.32 (dd, *J* = 10.1, 8.9 Hz, 1H), 3.27 (t, *J* = 9.8 Hz, 1H), 3.12 (d, *J* = 12.5 Hz, 2H), 3.08-2.97 (m, 2H), 2.96-2.84 (m, 3H), 2.81-2.73 (m, 1H), 2.73-2.63 (m, 3H), 2.25 (d, *J =* 6.5 Hz, 2H), 1.98 (dt, *J* = 13.1, 4.2 Hz, 1H), 1.94-1.63 (m, 8H), 1.58-1.46 (m, 1H), 1.27-1.12 (m, 6H), 1.02-0.90 (m, 1H). All exchangeable protons are missing.

### Preparation of 529

### Synthetic scheme

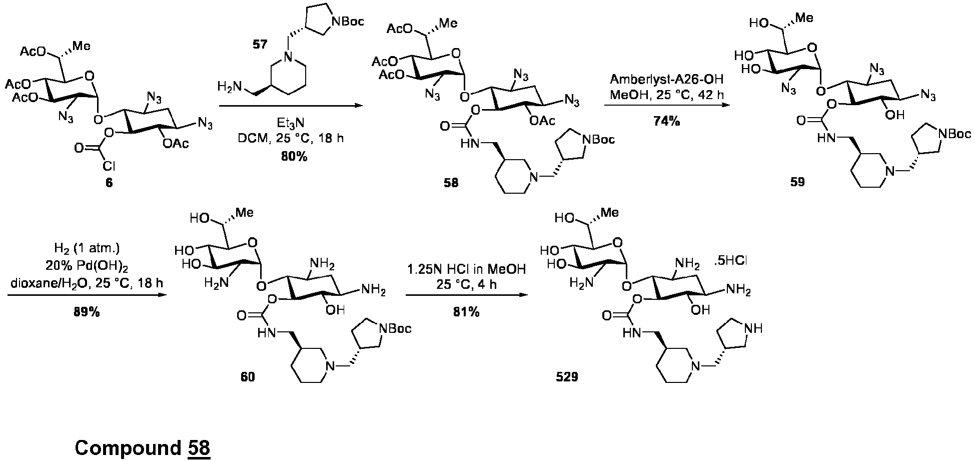

### (2R,3S,4R,5R,6S)-6-(((1R,2S,3S,4R,6S)-3-acetoxy-4,6-diazido-2-(((((S)-1-(((S)-1-(tert-butoxycarbonyl)pyrrolidin-3-yl)methyl)piperidin-3-yl)methyl)carbamoyl)oxy)cyclohexyl)oxy)-2-((R)-1-acetoxyethyl)-5-azidotetrahydro-2H-pyran-3,4-diyl diacetate

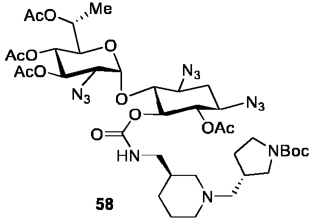

Intermediate **58** was prepared according to general procedure **A,** starting from intermediate **6** (3.10 g, 4.79 mmol, 1 eq.) and intermediate **57** (1.71 g, 5.75 mmol, 1.2 eq.). The crude residue was purified by silica gel flash chromatography (DCM/EtOAc= from 100:0 to 0:100 then EtOAc/MeOH= from 100:0 to 90:10) to afford intermediate **58** as a white solid (3.49 g, 80%). LCMS (ESI⁺): RT= 2.90 min, [M+H]⁺=907.6 (Method 1).

### Compound 59

### tert-butyl (S)-3-(((S)-3-((((((1R,2R,3S,5R,6S)-3,5-diazido-2-(((2S,3R,4R,5S,6R)-3-azido-4,5-dihydroxy-6-((R)-1-hydroxyethyl)tetrahydro-2H-pyran-2-yl)oxy)-6-hydroxycyclohexyl)oxy)carbonyl)amino)methyl)piperidin-1-yl)methyl)pyrrolidine-1-carboxylate

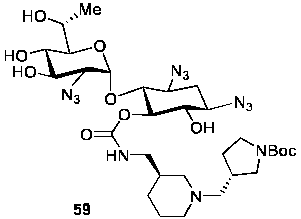

Intermediate **59** was prepared according to general procedure **B,** starting from intermediate **58** (3.49 g, 3.85 mmol, 1 eq.). The crude residue was purified by preparative HPLC (column: XBridge C18 (30x150 mm (5µm)); flow Rate: 43 mL/min; gradient: Water (+0.2% NH₄HCO₃)/Acetonitrile = from 65:35 to 50:50) to afford intermediate **59** as a white solid (2.09 g, 74%). LCMS (ESI⁺): RT= 9.22 min, [M+H]⁺=739.4 (Method 2).

### Compound 60

### tert-butyl (S)-3-(((S)-3-((((((1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl)oxy)carbonyl)amino)methyl)piperidin-1-yl)methyl)pyrrolidine-1-carboxylate

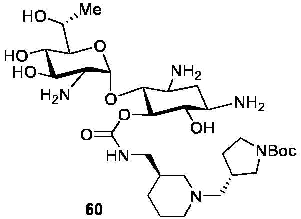

Starting from intermediate **59** (846 mg, 1.15 mmol, 1 eq.), using general procedure **C1,** intermediate **60** was obtained as an off-white solid (675 mg, 89%). LCMS (ESI⁺): RT= 4.52 min, [M+H]⁺=661.9 (Method 5).
**529**

### (1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl (((S)-1-(((R)-pyrrolidin-3-yl)methyl)piperidin-3-yl)methyl)carbamate pentahydrochloride

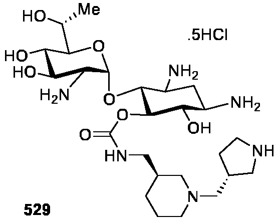

Starting from intermediate **60** (675 mg, 1.02 mmol, 1 eq.), using general procedure **D1, 529** was obtained as an off-white solid (617 mg, 81%). LCMS (ESI⁺): RT= 5.86 min, [M-5HCl+H]⁺=561.3 (Method 5). ¹H NMR (400 MHz, D₂O): *δ* 5.49 (d, *J* = 4.0 Hz, 1H), 4.97 (t, *J* = 9.3 Hz, 1H), 4.30 (qd, *J* = 6.5, 2.5 Hz, 1H), 4.22 (t, *J =* 9.6 Hz, 1H), 3.97 (dd, *J* = 10.6, 8.9 Hz, 1H), 3.86-3.79 (m, 2H), 3.77-3.58 (m, 4H), 3.58-3.44 (m, 4H), 3.43-3.27 (m, 4H), 3.17-3.04 (m, 2H), 3.02-2.88 (m, 2H), 2.83-2.71 (m, 1H), 2.58 (dt, *J* = 12.8, 4.3 Hz, 1H), 2.43 (dtd, *J* = 13.4, 7.1, 3.7 Hz, 1H), 2.23-2.02 (m, 2H), 2.01-1.75 (m, 4H), 1.39-1.29 (m, 1H), 1.27 (d, *J* = 6.6 Hz, 3H). All exchangeable protons are missing.

### Preparation of 534

### Synthetic scheme

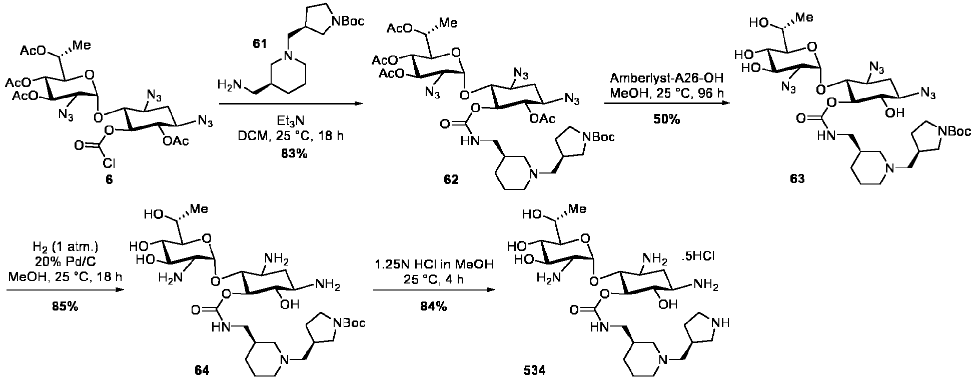

### Compound 62

### (2R,3S,4R,5R,6S)-6-(((1R,2S,3S,4R,6S)-3-acetoxy-4,6-diazido-2-(((((S)-1-(((R)-1-(tert-butoxycarbonyl)pyrrolidin-3-yl)methyl)piperidin-3-yl)methyl)carbamoyl)oxy)cyclohexyl)oxy)-2-((R)-1-acetoxyethyl)-5-azidotetrahydro-2H-pyran-3,4-diyl diacetate

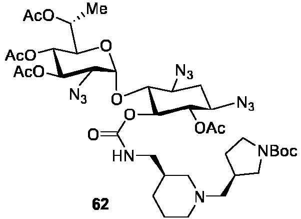

Intermediate **62** was prepared according to general procedure **A,** starting from intermediate **6** (443 mg, 0.686 mmol, 1 eq.) and intermediate **61** (306 g, 1.03 mmol, 1.5 eq.). The crude residue was purified by silica gel flash chromatography (DCM/EtOAc= from 100:0 to 0:100) to afford intermediate **62** as a white solid (514 mg, 83%). LCMS (ESI⁺): RT= 2.78 min, [M+H]⁺=907.7 (Method 1).

### Compound 63

### tert-butyl (R)-3-(((S)-3-((((((1R,2R,3S,5R,6S)-3,5-diazido-2-(((2S,3R,4R,5S,6R)-3-azido-4,5-dihydroxy-6-((R)-1-hydroxyethyl)tetrahydro-2H-pyran-2-yl)oxy)-6-hydroxycyclohexyl)oxy)carbonyl)amino)methyl)piperidin-1-yl)methyl)pyrrolidine-1-carboxylate

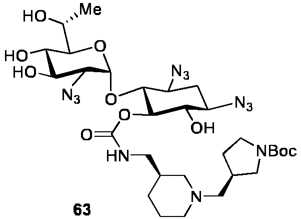

Intermediate **63** was prepared according to general procedure **B,** starting from intermediate **62** (514 mg, 0.567 mmol, 1 eq.). The crude residue was purified by preparative HPLC (column: XBridge C18 (30x150 mm (5µm)); flow Rate: 43 mL/min; gradient: Water (+0.2% NH₄HCO₃)/Acetonitrile = from 63:37 to 47:53) to afford intermediate **63** as a white solid (210 mg, 50%). LCMS (ESI⁺): RT= 9.22 min, [M+H]⁺=739.4 (Method 2).

### Compound 64

### tert-butyl (R)-3-(((S)-3-((((((1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl)oxy)carbonyl)amino)methyl)piperidin-1-yl)methyl)pyrrolidine-1-carboxylate

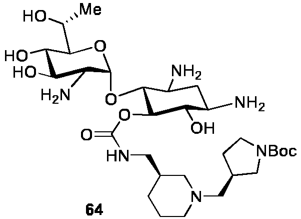

Starting from intermediate **63** (50.0 mg, 0.0677 mmol, 1 eq.), using general procedure **C2,** intermediate **64** was obtained as a white solid (38.0 mg, 85%). LCMS (ESI⁺): RT= 6.50 min, [M+H]⁺=661.4 (Method 4).
**534**

### (1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl (((S)-1-(((S)-pyrrolidin-3-yl)methyl)piperidin-3-yl)methyl)carbamate pentahydrochloride

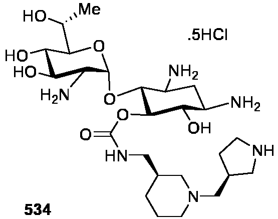

Starting from intermediate **64** (38.0 mg, 0.0575 mmol, 1 eq.), using general procedure **D1, 534** was obtained as a white solid (36 mg, 84%). LCMS (ESI⁺): RT= 9.03 min, [M-5HCl+H]⁺=561.3 (Method 5). ¹H NMR (400 MHz, D₂O): *δ* 5.45 (d, *J* = 4.0 Hz, 1H), 4.94 (t, *J* = 9.3 Hz, 1H), 4.28 (qd, *J* = 6.5, 2.5 Hz, 1H), 4.17 (t, *J =* 9.6 Hz, 1H), 3.93 (dd, *J* = 10.6, 8.9 Hz, 1H), 3.83-3.75 (m, 2H), 3.73-3.40 (m, 8H), 3.39-3.25 (m, 4H), 3.15-3.03 (m, 2H), 2.99-2.84 (m, 2H), 2.82-2.70 (m, 1H), 2.54 (dt, *J* = 12.7, 4.3 Hz, 1H), 2.40 (dtd, *J* = 14.0, 7.2, 3.6 Hz, 1H), 2.19-1.99 (m, 2H), 1.96-1.73 (m, 4H), 1.36-1.26 (m, 1H), 1.24 (d, *J* = 6.5 Hz, 3H). All exchangeable protons are missing.

### Preparation of 531

### Synthetic scheme

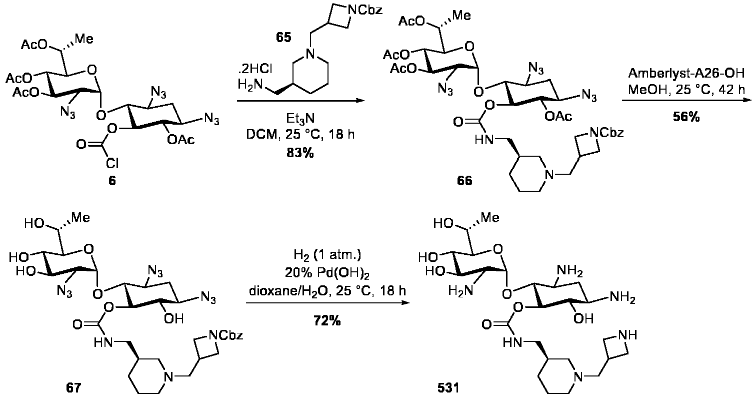

### Compound 66

### (2R,3S,4R,5R,6S)-6-(((1R,2S,3S,4R,6S)-3-acetoxy-4,6-diazido-2-(((((S)-1-((1-((benzyloxy)carbonyl)azetidin-3-yl)methyl)piperidin-3-yl)methyl)carbamoyl)oxy)cyclohexyl)oxy)-2-((R)-1-acetoxyethyl)-5-azidotetrahydro-2H-pyran-3,4-diyl diacetate

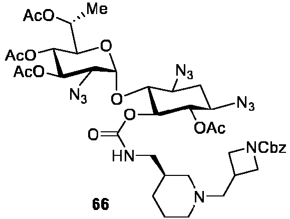

Intermediate **66** was prepared according to general procedure **A,** starting from intermediate **6** (333 mg, 0.515 mmol, 1 eq.) and intermediate **65** (278 mg, 0.876 mmol, 1.7 eq.). The crude residue was purified by silica gel flash chromatography (DCM/EtOAc= from 100:0 to 0:100) to afford intermediate **66** as a white solid (396 mg, 83%). LCMS (ESI⁺): RT= 2.83 min, [M+H]⁺=927.6 (Method 1).

### Compound 67

### benzyl 3-(((S)-3-((((((1R,2R,3S,5R,6S)-3,5-diazido-2-(((2S,3R,4R,5S,6R)-3-azido-4,5-dihydroxy-6-((R)-1-hydroxyethyl)tetrahydro-2H-pyran-2-yl)oxy)-6-hydroxycyclohexyl)oxy)carbonyl)amino)methyl)piperidin-1-yl)methyl)azetidine-1-carboxylate

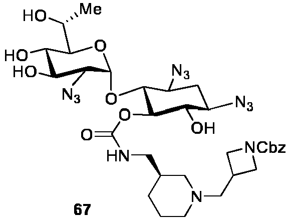

Intermediate **67** was prepared according to general procedure **B,** starting from intermediate **66** (396 mg, 0.427 mmol, 1 eq.). The crude residue was purified by preparative HPLC (column: XBridge C18 (30x150 mm (5µm)); flow Rate: 43 mL/min; gradient: Water (+0.2% NH₄HCO₃)/Acetonitrile = from 75:25 to 59:41) to afford intermediate **67** as a white solid (181 mg, 56%). LCMS (ESI⁺): RT= 9.02 min, [M+H]⁺=759.3 (Method 2).
**531**

### (1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl (((S)-1-(azetidin-3-ylmethyl)piperidin-3-yl)methyl)carbamate

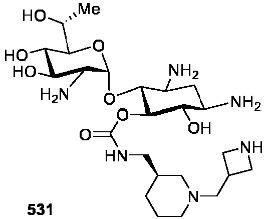

Starting from intermediate **67** (50.0 mg, 0.0659 mmol, 1 eq.), using general procedure **C1, 531** was obtained as a white solid (26.0 mg, 72%). LCMS (ESI⁺): RT= 9.15 min, [M+H]⁺=547.2 (Method 5). ¹H NMR (400 MHz, D₂O): *δ* 5.03 (d, *J =* 3.6 Hz, 1H), 4.68 (t, *J* = 9.5 Hz, 1H), 4.17 (qd, *J* = 6.6, 2.3 Hz, 1H), 3.87 (dd, *J* = 10.1, 2.6 Hz, 1H), 3.82 (t, *J* = 8.8 Hz, 2H), 3.59-3.53 (m, 2H), 3.50-3.42 (m, 2H), 3.33 (t, *J* = 9.5 Hz, 1H), 3.27 (t, *J* = 9.7 Hz, 1H), 3.12-2.87 (m, 4H), 2.82-2.73 (m, 3H), 2.70-2.62 (m, 3H), 2.04-1.93 (m, 2H), 1.83-1.66 (m, 4H), 1.56-1.42 (m, 1H), 1.27-1.15 (m, 4H), 1.02-0.89 (m, 1H). All exchangeable protons are missing.

### Preparation of 688

### Synthetic scheme

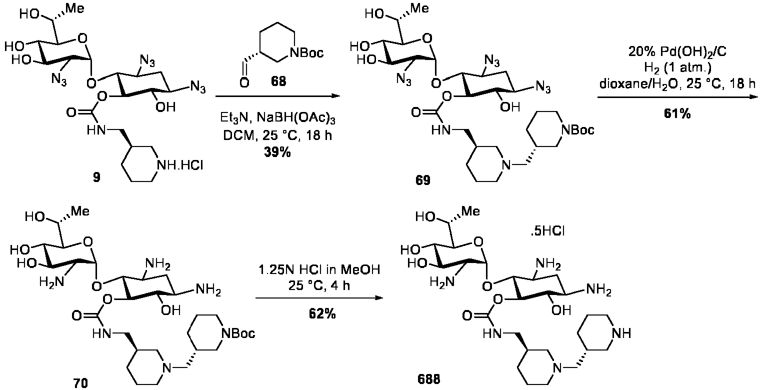

### Compound 69

### tert-butyl (S)-3-(((S)-3-((((((1R,2R,3S,5R,6S)-3,5-diazido-2-(((2S,3R,4R,5S,6R)-3-azido-4,5-dihydroxy-6-((R)-1-hydroxyethyl)tetrahydro-2H-pyran-2-yl)oxy)-6-hydroxycyclohexyl)oxy)carbonyl)amino)methyl)piperidin-1-yl)methyl)piperidine-1-carboxylate

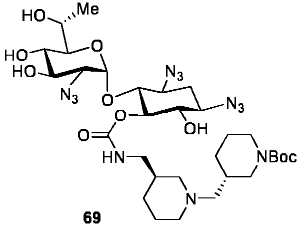

Intermediate **69** was prepared according to general procedure **E1,** starting from intermediate **9** (150 mg, 0.253 mmol, 1 eq.) and intermediate **68** (64.8 mg, 0.304 mmol, 1.2 eq.). The crude residue was purified by silica gel flash chromatography (DCM/MeOH= from 100:0 to 80:20) to afford intermediate **69** as a pale yellow solid (75 mg, 39%). LCMS (ESI⁺): RT= 8.75 min, [M+H]⁺=754.0 (Method 2).

### Compound 70

### tert-butyl (S)-3-(((S)-3-((((((1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl)oxy)carbonyl)amino)methyl)piperidin-1-yl)methyl)piperidine-1-carboxylate

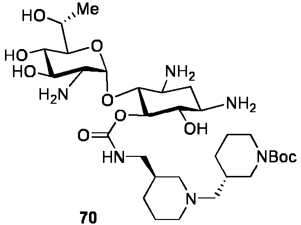

Starting from intermediate **69** (75.0 mg, 0.0996 mmol, 1 eq.), using general procedure **C1,** compound **70** was obtained as a white solid (41.0 mg, 61%). LCMS (ESI⁺): RT= 4.45 min, [M+H]⁺=675.9 (Method 5).
**688**

### (1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl (((S)-1-(((R)-piperidin-3-yl)methyl)piperidin-3-yl)methyl)carbamate pentahydrochloride

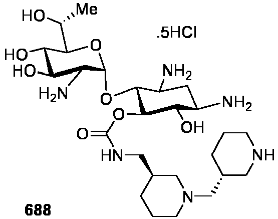

Starting from intermediate **70** (40.0 mg, 0.0593 mmol, 1 eq.), using general procedure **D1, 688** was obtained as a white solid (28.0 mg, 62%). LCMS (ESI⁺): RT= 5.76 min, [M-5HCl+H]⁺=575.8 (Method 5).¹H NMR (400 MHz, D₂O): *δ* 5.47 (d, *J* = 4.0 Hz, 1H), 4.96 (t, *J* = 9.3 Hz, 1H), 4.30 (qd, *J* = 6.5, 2.4 Hz, 1H), 4.19 (t, *J =* 9.5 Hz, 1H), 3.99-3.92 (m, 1H), 3.84-3.78 (m, 2H), 3.74-3.66 (m, 1H), 3.66-3.39 (m, 7H), 3.30 (dd, *J =* 14.1, 7.0 Hz, 1H), 3.21-3.11 (m, 3H), 3.03-2.93 (m, 2H), 2.85 (t, *J =* 12.3 Hz, 1H), 2.81-2.69 (m, 1H), 2.57 (dt, *J* = 12.7, 4.2 Hz, 1H), 2.50-2.37 (m, 1H), 2.21-2.10 (m, 1H), 2.10-2.01 (m, 3H), 1.99-1.88 (m, 2H), 1.87-1.76 (m, 2H), 1.48-1.40 (m, 1H), 1.40-1.29 (m, 1H), 1.27 (d, *J* = 6.5 Hz, 3H). All exchangeable protons are missing.

### Preparation of 692

### Synthetic scheme

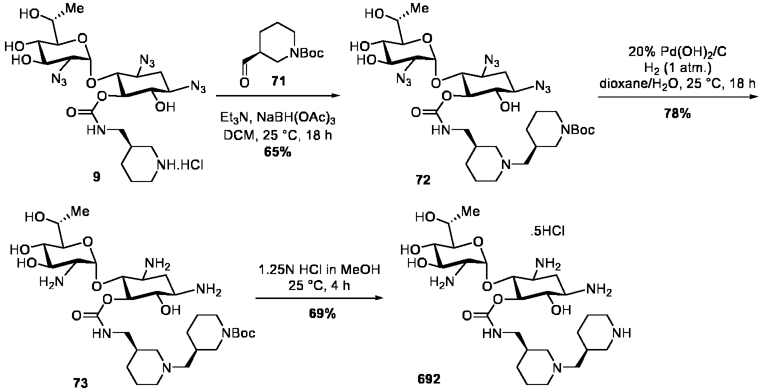

### Compound 72

### tert-butyl (R)-3-(((S)-3-((((((1R,2R,3S,5R,6S)-3,5-diazido-2-(((2S,3R,4R,5S,6R)-3-azido-4,5-dihydroxy-6-((R)-1-hydroxyethyl)tetrahydro-2H-pyran-2-yl)oxy)-6-hydroxycyclohexyl)oxy)carbonyl)amino)methyl)piperidin-1-yl)methyl)piperidine-1-carboxylate

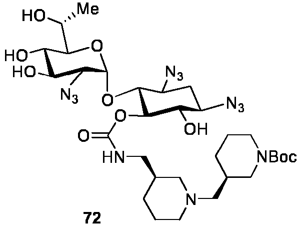

Intermediate **72** was prepared according to general procedure **E1,** starting from intermediate **9** (150 mg, 0.253 mmol, 1 eq.) and intermediate **71** (75.6 mg, 0.355 mmol, 1.4 eq.). The crude residue was purified by silica gel flash chromatography (DCM/MeOH= from 100:0 to 80:20) to afford intermediate **72** as a pale yellow solid (123 mg, 65%). LCMS (ESI⁺): RT= 8.68 min, [M+H]⁺=754.0 (Method 2).

### Compound 73

### tert-butyl (R)-3-(((S)-3-((((((1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl)oxy)carbonyl)amino)methyl)piperidin-1-yl)methyl)piperidine-1-carboxylate

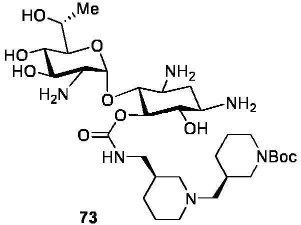

Starting from intermediate **72** (60.0 mg, 0.0797 mmol, 1 eq.), using general procedure C1, compound **73** was obtained as a white solid (41.8 mg, 78%). LCMS (ESI⁺): RT= 4.40 min, [M+H]⁺=676.0 (Method 5).
**692**

### (1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl (((S)-1-(((S)-piperidin-3-yl)methyl)piperidin-3-yl)methyl)carbamate pentahydrochloride

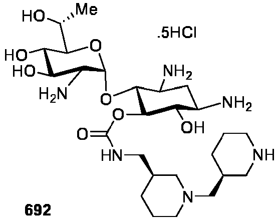

Starting from intermediate **73** (39.0 mg, 0.0578 mmol, 1 eq.), using general procedure **D1, 692** was obtained as a white solid (30.0 mg, 69%). LCMS (ESI⁺): RT= 5.75 min, [M-5HCl+H]⁺=575.8 (Method 5).¹H NMR (400 MHz, D₂O): *δ* 5.47 (d, *J =* 4.1 Hz, 1H), 4.97 (t, *J* = 9.3 Hz, 1H), 4.30 (qd, *J* = 6.6, 2.7 Hz, 1H), 4.20 (t, *J =* 9.5 Hz, 1H), 3.95 (dd, *J* = 10.6, 8.9 Hz, 1H), 3.85-3.78 (m, 2H), 3.75-3.55 (m, 3H), 3.54-3.42 (m, 5H), 3.31 (dd, *J =* 14.2, 6.8 Hz, 1H), 3.22-3.10 (m, 3H), 2.98 (td, *J* = 13.0, 3.1 Hz, 1H), 2.95-2.74 (m, 3H), 2.57 (dt, *J =* 12.7, 4.3 Hz, 1H), 2.49-2.37 (m, 1H), 2.24-2.11 (m, 1H), 2.11-1.99 (m, 3H), 2.00-1.87 (m, 2H), 1.87-1.73 (m, 2H), 1.49-1.39 (m, 1H), 1.38-1.29 (m, 1H), 1.27 (d, *J* = 6.5 Hz, 3H). All exchangeable protons are missing.

### Preparation of 617

### Synthetic scheme

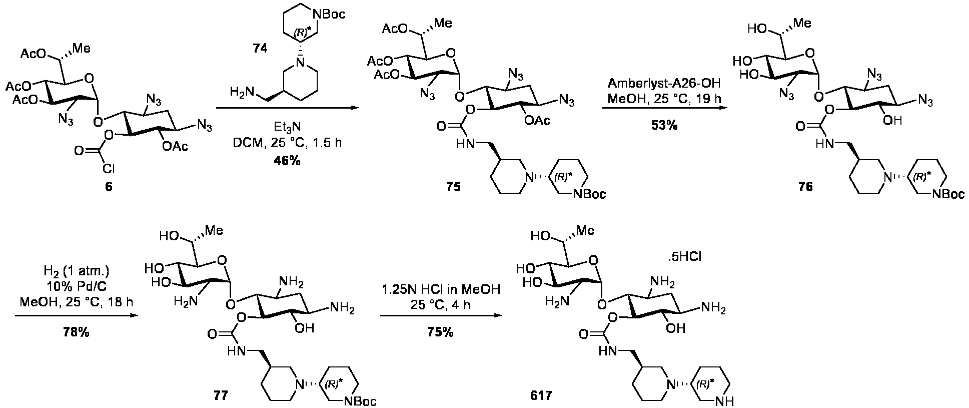

### Compound 75

### (2R,3S,4R,5R,6S)-6-(((1R,2S,3S,4R,6S)-3-acetoxy-4,6-diazido-2-(((((3S,3'R*)-1'-(tert-butoxycarbonyl)-[1,3'-bipiperidin]-3-yl)methyl)carbamoyl)oxy)cyclohexyl)oxy)-2-((R)-1-acetoxyethyl)-5-azidotetrahydro-2H-pyran-3,4-diyl diacetate

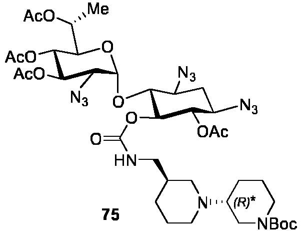

Intermediate **75** was prepared according to general procedure **A,** starting from intermediate **6** (387 mg, 0.599 mmol, 1 eq.) and intermediate **74** (214 mg, 0.719 mmol, 1.2 eq.). The crude residue was purified by silica gel flash chromatography (DCM/EtOAc= from 50:50 to 0:100) to afford intermediate **75** as a pale yellow solid (252 mg, 46%). LCMS (ESI⁺): RT= 2.25 min, [M+H]⁺=907.6 (Method 1).

### Compound 76

### tert-butyl (3S,3'R*)-3-((((((1R,2R,3S,5R,6S)-3,5-diazido-2-(((2S,3R,4R,5S,6R)-3-azido-4,5-dihydroxy-6-((R)-1-hydroxyethyl)tetrahydro-2H-pyran-2-yl)oxy)-6-hydroxycyclohexyl)oxy)carbonyl)amino)methyl)-[1,3'-bipiperidine]-1'-carboxylate

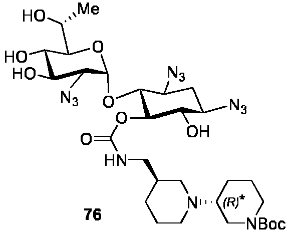

Intermediate **76** was prepared according to general procedure **B,** starting from intermediate **75** (252 mg, 0.278 mmol, 1 eq.). The crude residue was purified by preparative HPLC (column: XBridge C18 (30x150 mm (5µm)); flow Rate: 43 mL/min; gradient: Water (+0.2% NH₄HCO₃)/Acetonitrile = from 70:30 to 55:45) to afford intermediate **76** as a white solid (109 mg, 53%). LCMS (ESI⁺): RT= 8.25 min, [M+H]⁺=740.0 (Method 2).

### Compound 77

### tert-butyl (3S,3'R*)-3-((((((1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl)oxy)carbonyl)amino)methyl)-[1,3'-bipiperidine]-1'-carboxylate

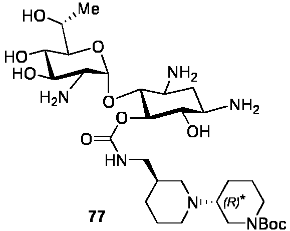

Starting from intermediate **76** (60.0 mg, 0.0812 mmol, 1 eq.), using general procedure **C2,** intermediate **77** was obtained as an off-white solid (42.0 mg, 78%). LCMS (ESI⁺): RT= 0.47 min, [M+H]⁺=661.9 (Method 1).
**617**

### (1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl (((3S,3'R*)-[1,3'-bipiperidin]-3-yl)methyl)carbamate pentahydrochloride

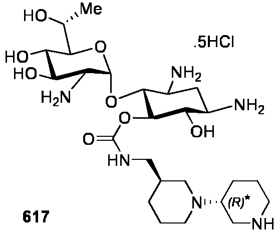

Starting from intermediate **77** (45.0 mg, 0.0681 mmol, 1 eq.), using general procedure **D1, 617** was obtained as a white solid (38.0 mg, 75%). LCMS (ESI⁺): RT= 5.83 min, [M-5HCl+H]⁺=561.8 (Method 5).¹H NMR (400 MHz, D₂O): *δ* 5.45 (d, *J* = 4.0 Hz, 1H), 4.95 (t, *J* = 9.3 Hz, 1H), 4.28 (qd, *J* = 6.3, 2.3 Hz, 1H), 4.18 (t, *J =* 9.6 Hz, 1H), 3.94 (t, *J* = 9.6 Hz, 1H), 3.86-3.75 (m, 3H), 3.73-3.61 (m, 2H), 3.61-3.39 (m, 6H), 3.34-3.24 (m, 2H), 3.15-3.04 (m, 2H), 3.03-2.94 (m, 1H), 2.94-2.84 (m, 1H), 2.54 (dt, *J* = 12.6, 4.3 Hz, 1H), 2.40-2.28 (m, 1H), 2.24-2.03 (m, 3H), 1.97-1.73 (m, 5H), 1.38-1.27 (m, 1H), 1.24 (d, *J* = 6.6 Hz, 3H). All exchangeable protons are missing.

### Preparation of 613

### Synthetic scheme

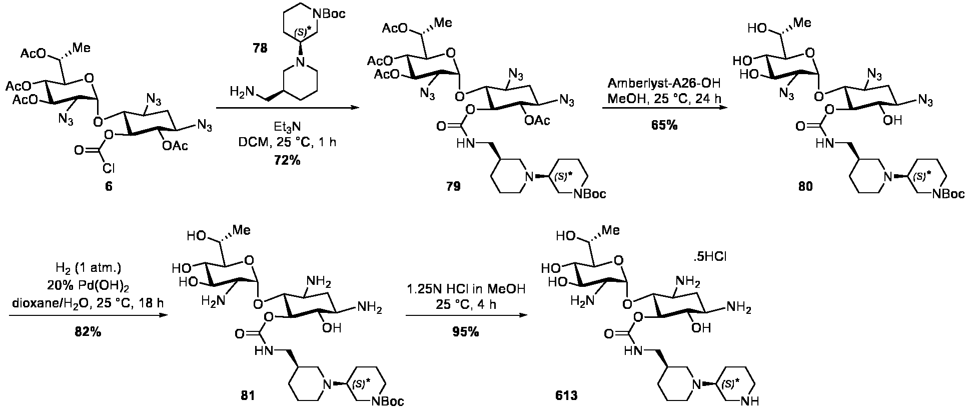

### Compound 79

### (2R,3S,4R,5R,6S)-6-(((1R,2S,3S,4R,6S)-3-acetoxy-4,6-diazido-2-(((((3S,3'S*)-1'-(tert-butoxycarbonyl)-[1,3'-bipiperidin]-3-yl)methyl)carbamoyl)oxy)cyclohexyl)oxy)-2-((R)-1-acetoxyethyl)-5-azidotetrahydro-2H-pyran-3,4-diyl diacetate

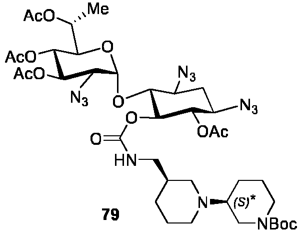

Intermediate **79** was prepared according to general procedure **A,** starting from intermediate **6** (4.46 g, 6.90 mmol, 1 eq.) and intermediate **78** (2.46 g, 8.28 mmol, 1.2 eq.). The crude residue was purified by silica gel flash chromatography (DCM/EtOAc= from 100:0 to 0:100) to afford intermediate **79** as a white solid (4.52 g, 72%). LCMS (ESI⁺): RT= 2.36 min, [M+H]⁺=907.7 (Method 1).

### Compound 80

### tert-butyl (3S,3'S*)-3-((((((1R,2R,3S,5R,6S)-3,5-diazido-2-(((2S,3R,4R,5S,6R)-3-azido-4,5-dihydroxy-6-((R)-1-hydroxyethyl)tetrahydro-2H-pyran-2-yl)oxy)-6-hydroxycyclohexyl)oxy)carbonyl)amino)methyl)-[1,3'-bipiperidine]-1'-carboxylate

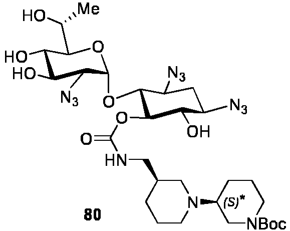

Intermediate **80** was prepared according to general procedure **B,** starting from intermediate **79** (4.78 g, 5.27 mmol, 1 eq.). The crude residue was purified by preparative HPLC (column: XBridge C18 (30x150 mm (5µm)); flow Rate: 43 mL/min; gradient: Water (+0.2% NH₄HCO₃)/Acetonitrile = from 70:30 to 55:45) to afford intermediate **80** as a white solid (2.51 g, 65%). LCMS (ESI⁺): RT= 8.37 min, [M+H]⁺=739.9 (Method 2).

### Compound 81

### tert-butyl (3S,3'S*)-3-((((((1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl)oxy)carbonyl)amino)methyl)-[1,3'-bipiperidine]-1'-carboxylate

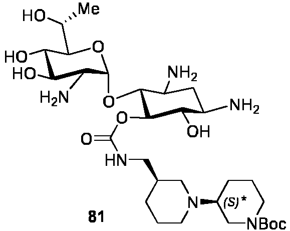

Starting from intermediate **80** (2.93 g, 3.97 mmol, 1 eq.), using general procedure **C1,** intermediate **81** was obtained as an off-white solid (2.14 g, 82%). LCMS (ESI⁺): RT= 4.50 min, [M+H]⁺=661.6 (Method 5).
**613**

### (1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl (((3S,3'S*)-[1,3'-bipiperidin]-3-yl)methyl)carbamate pentahydrochloride

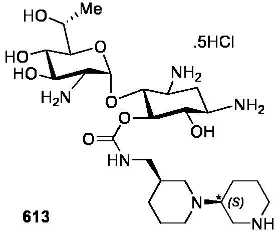

**613** was prepared according to general procedure **D1,** starting from intermediate **81** (2.14 g, 3.24 mmol, 1 eq.). The resulting solid was dissolved in H₂O (15 mL) and freeze-dried for 48 h to afford compound **613** as an off-white solid (2.28 g, 95%). LCMS (ESI⁺): RT= 5.92 min, [M-5HCl+H]⁺=561.6 (Method 5). ¹H NMR (400 MHz, D₂O): *δ* 5.49 (d, *J =* 4.0 Hz, 1H), 4.98 (t, *J* = 9.3 Hz, 1H), 4.30 (qd, *J* = 6.5, 2.5 Hz, 1H), 4.22 (dd, *J* = 10.0, 9.2 Hz, 1H), 3.98 (dd, *J* = 10.6, 8.8 Hz, 1H), 3.91-3.80 (m, 3H), 3.77-3.65 (m, 2H), 3.61-3.54 (m, 2H), 3.54-3.45 (m, 4H), 3.37-3.27 (m, 2H), 3.18-3.08 (m, 2H), 3.02 (td, *J* = 13.0, 3.4 Hz, 1H), 2.98-2.88 (m, 1H), 2.58 (dt, *J* = 12.7, 4.3 Hz, 1H), 2.40-2.32 (m, 1H), 2.26-2.06 (m, 3H), 2.00-1.78 (m, 5H), 1.41-1.30 (m, 1H), 1.27 (d, *J* = 6.6 Hz, 3H). All exchangeable protons are missing.

### Preparation of 614

### Synthetic scheme

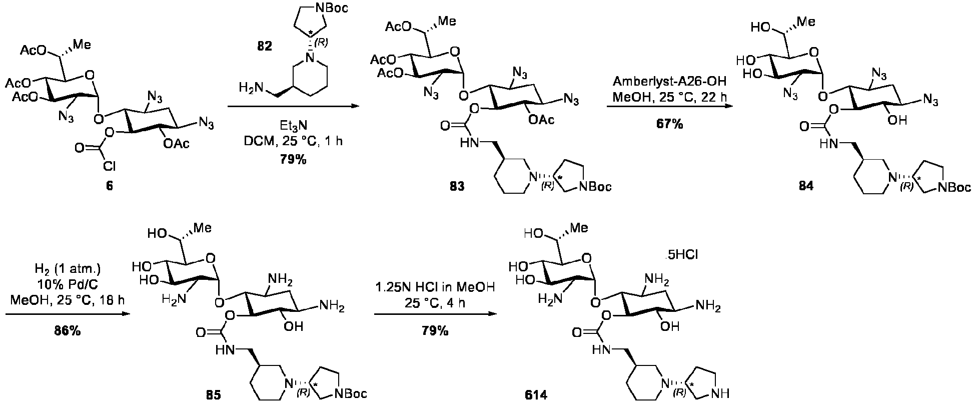

### Compound 83

### (2R,3S,4R,5R,6S)-6-(((1R,2S,3S,4R,6S)-3-acetoxy-4,6-diazido-2-(((((S)-1-((R*)-1-(tert-butoxycarbonyl)pyrrolidin-3-yl)piperidin-3-yl)methyl)carbamoyl)oxy)cyclohexyl)oxy)-2-((R)-1-acetoxyethyl)-5-azidotetrahydro-2H-pyran-3,4-diyl diacetate

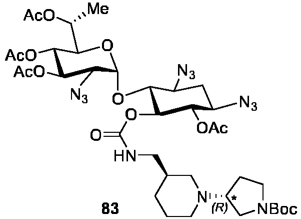

Intermediate **83** was prepared according to general procedure **A,** starting from intermediate **6** (300 mg, 0.464 mmol, 1 eq.) and intermediate **82** (197 mg, 0.697 mmol, 1.5 eq.). The crude residue was purified by silica gel flash chromatography (DCM/EtOAc= from 100:0 to 0:100) to afford intermediate **83** as a white solid (329 mg, 79%). LCMS (ESI⁺): RT= 2.06 min, [M+H]⁺=894.0 (Method 1).

### Compound 84

*tert-*butyl (*R**)-3-((*S*)-3-((((((*1R,2R,3S,5R,6S*)-3,5-diazido-2-(((*2S,3R,4R,5S,6R*)-3-azido-4,5-dihydroxy-6-((*R*)-1-hydroxyethyl)tetrahydro-2*H*-pyran-2-yl)oxy)-6-hydroxycyclohexyl)oxy)carbonyl)amino)methyl)piperidin-1-yl)pyrrolidine-1-carboxylate

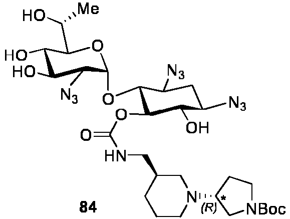

Intermediate **84** was prepared according to general procedure **B,** starting from intermediate **83** (329 mg, 0.369 mmol, 1 eq.). The crude residue was purified by preparative HPLC (column: XBridge C18 (30x150 mm (5µm)); flow Rate: 43 mL/min; gradient: Water (+0.2% NH₄HCO₃)/Acetonitrile = from 70:30 to 55:45) to afford intermediate **84** as a white solid (178 mg, 67%). LCMS (ESI⁺): RT= 7.83 min, [M+H]⁺=725.9 (Method 2).

### Compound 85

### tert-butyl (R*)-3-((S)-3-((((((1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl)oxy)carbonyl)amino)methyl)piperidin-1-yl)pyrrolidine-1-carboxylate

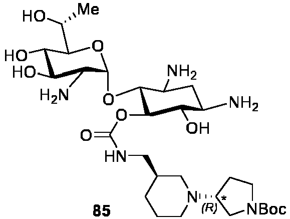

Starting from intermediate **84** (60.0 mg, 0.0828 mmol, 1 eq.), using general procedure **C2,** intermediate **85** was obtained as a white solid (46.0 mg, 86%). LCMS (ESI⁺): RT= 4.46 min, [M+H]⁺=648.0 (Method 5).
**614**

### (1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl (((S)-1-((R*)-pyrrolidin-3-yl)piperidin-3-yl)methyl)carbamate pentahydrochloride

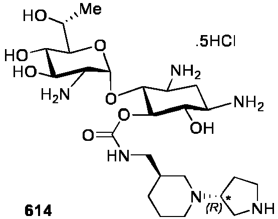

Starting from intermediate **85** (46.0 mg, 0.0711 mmol, 1 eq.), using general procedure **D1, 614** was obtained as a white solid (41.0 mg, 79%). LCMS (ESI⁺): RT= 5.92 min, [M-5HCl+H]⁺=547.7 (Method 5).¹H NMR (400 MHz, D₂O): *δ* 5.45 (d, *J* = 4.0 Hz, 1H), 4.94 (t, *J* = 9.2 Hz, 1H), 4.31-4.24 (m, 1H), 4.17 (t, *J* = 9.6 Hz, 1H), 4.12-4.05 (m, 1H), 3.97-3.88 (m, 2H), 3.82-3.75 (m, 2H), 3.72-3.57 (m, 3H), 3.57-3.39 (m, 6H), 3.30 (dd, *J* = 14.3, 7.0 Hz, 1H), 3.12 (dd, *J* = 14.2, 5.3 Hz, 1H), 3.08-2.96 (m, 1H), 2.88-2.75 (m, 1H), 2.71-2.61 (m, 1H), 2.54 (dt, *J* = 12.8, 4.3 Hz, 1H), 2.31-2.19 (m, 1H), 2.16-2.02 (m, 2H), 1.97-1.85 (m, 2H), 1.85-1.71 (m, 1H), 1.38-1.26 (m, 1H), 1.24 (d, *J* = 6.6 Hz, 3H). All exchangeable protons are missing.

### Preparation of 619

### Synthetic scheme

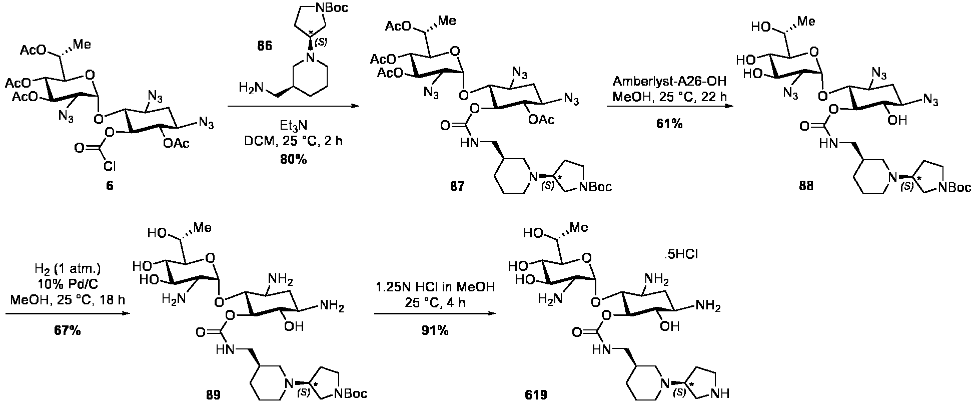

### Compound 87

### (2R,3S,4R,5R,6S)-6-(((1R,2S,3S,4R,6S)-3-acetoxy-4,6-diazido-2-(((((S)-1-((S*)-1-(tert-butoxycarbonyl)pyrrolidin-3-yl)piperidin-3-yl)methyl)carbamoyl)oxy)cyclohexyl)oxy)-2-((R)-1-acetoxyethyl)-5-azidotetrahydro-2H-pyran-3,4-diyl diacetate

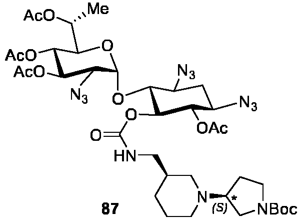

Intermediate **87** was prepared according to general procedure A, starting from intermediate **6** (364 mg, 0.563 mmol, 1 eq.) and intermediate **86** (240 mg, 0.845 mmol, 1.5 eq.). The crude residue was purified by silica gel flash chromatography (DCM/EtOAc= from 100:0 to 0:100) to afford intermediate **87** as a white solid (403 mg, 80%). LCMS (ESI⁺): RT= 2.04 min, [M+H]⁺=894.0 (Method 1).

### Compound 88

### tert-butyl (S*)-3-((S)-3-((((((1R,2R,3S,5R,6S)-3,5-diazido-2-(((2S,3R,4R,5S,6R)-3-azido-4,5-dihydroxy-6-((R)-1-hydroxyethyl)tetrahydro-2H-pyran-2-yl)oxy)-6-hydroxycyclohexyl)oxy)carbonyl)amino)methyl)piperidin-1-yl)pyrrolidine-1-carboxylate

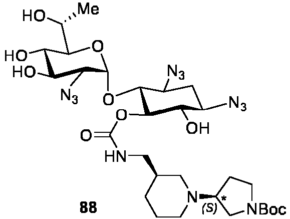

Intermediate **88** was prepared according to general procedure **B,** starting from intermediate **87** (402 mg, 0.450 mmol, 1 eq.). The crude residue was purified by preparative HPLC (column: XBridge C18 (30x150 mm (5µm)); flow Rate: 43 mL/min; gradient: Water (+0.2% NH₄HCO₃)/Acetonitrile = from 75:25 to 60:40) to afford intermediate **88** as a white solid (199 mg, 61%). LCMS (ESI⁺): RT= 7.93 min, [M+H]⁺=726.0 (Method 2).

### Compound 89

### tert-butyl (S*)-3-((S)-3-((((((1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl)oxy)carbonyl)amino)methyl)piperidin-1-yl)pyrrolidine-1-carboxylate

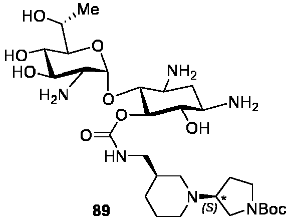

Starting from intermediate **88** (60.0 mg, 0.0828 mmol, 1 eq.), using general procedure **C2,** intermediate **89** was obtained as a white solid (35.8 mg, 67%). LCMS (ESI⁺): RT= 4.55 min, [M+H]⁺=647.9 (Method 5).
**619**

### (1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl (((S)-1-((S*)-pyrrolidin-3-yl)piperidin-3-yl)methyl)carbamate pentahydrochloride

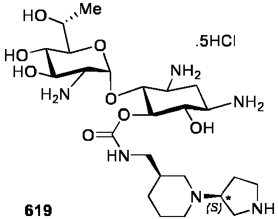

Starting from intermediate **89** (33.0 mg, 0.0510 mmol, 1 eq.), using general procedure **D1, 619** was obtained as a white solid (34.0 mg, 91%). LCMS (ESI⁺): RT= 5.98 min, [M-5HCl+H]⁺=547.7 (Method 5).¹H NMR (400 MHz, D₂O): *δ* 5.49 (d, *J =* 4.0 Hz, 1H), 4.97 (t, *J =* 9.3 Hz, 1H), 4.30 (qd, *J* = 6.5, 2.6 Hz, 1H), 4.25-4.11 (m, 2H), 4.01-3.93 (m, 2H), 3.86-3.79 (m, 2H), 3.76-3.63 (m, 3H), 3.63-3.57 (m, 1H), 3.56-3.42 (m, 5H), 3.33 (dd, *J* = 14.2, 6.9 Hz, 1H), 3.14 (dd, *J* = 14.2, 5.3 Hz, 1H), 3.11-3.01 (m, 1H), 2.99-2.87 (m, 1H), 2.69 (dtd, *J* = 14.2, 7.3, 3.6 Hz, 1H), 2.58 (dt, *J* = 12.7, 4.4 Hz, 1H), 2.36-2.24 (m, 1H), 2.21-2.07 (m, 2H), 2.00-1.89 (m, 2H), 1.89-1.76 (m, 1H), 1.43-1.30 (m, 1H), 1.27 (d, *J* = 6.6 Hz, 3H). All exchangeable protons are missing.

### Preparation of 615

### Synthetic scheme

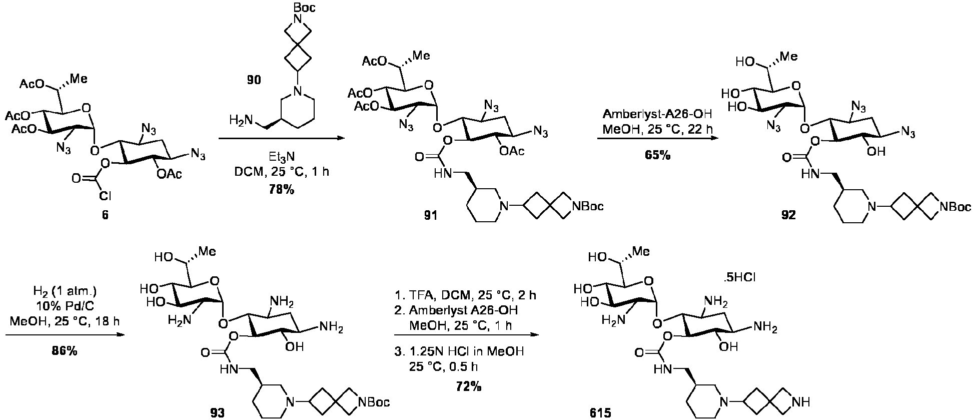

### Compound 91

### (2R,3S,4R,5R,6S)-6-(((1R,2S,3S,4R,6S)-3-acetoxy-4,6-diazido-2-(((((S)-1-(2-(tert-butoxycarbonyl)-2-azaspiro[3.3]heptan-6-yl)piperidin-3-yl)methyl)carbamoyl)oxy)cyclohexyl)oxy)-2-((R)-1-acetoxyethyl)-5-azidotetrahydro-2H-pyran-3,4-diyl diacetate

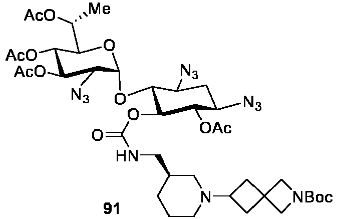

Intermediate **91** was prepared according to general procedure **A,** starting from intermediate **6** (300 mg, 0.464 mmol, 1 eq.) and intermediate **90** (216 mg, 0.697 mmol, 1.5 eq.). The crude residue was purified by silica gel flash chromatography (DCM/EtOAc= from 100:0 to 0:100) to afford intermediate **91** as a white solid (332 mg, 78%). LCMS (ESI⁺): RT= 2.13 min, [M+H]⁺=920.0 (Method 1).

### Compound 92

### tert-butyl 6-((S)-3-((((((1R,2R,3S,5R,6S)-3,5-diazido-2-(((2S,3R,4R,5S,6R)-3-azido-4,5-dihydroxy-6-((R)-1-hydroxyethyl)tetrahydro-2H-pyran-2-yl)oxy)-6-hydroxycyclohexyl)oxy)carbonyl)amino)methyl)piperidin-1-yl)-2-azaspiro[3.3]heptane-2-carboxylate

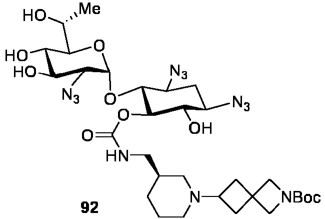

Intermediate **92** was prepared according to general procedure **B,** starting from intermediate **91** (332 mg, 0.361 mmol, 1 eq.). The crude residue was purified by preparative HPLC (column: XBridge C18 (30x150 mm (5µm)); flow Rate: 43 mL/min; gradient: Water (+0.2% NH₄HCO₃)/Acetonitrile = from 65:35 to 50:50) to afford intermediate **92** as a white solid (176 mg, 65%). LCMS (ESI⁺): RT= 8.08 min, [M+H]⁺=752.0 (Method 2).

### Compound 93

### tert-butyl 6-((S)-3-((((((1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl)oxy)carbonyl)amino)methyl)piperidin-1-yl)-2-azaspiro[3.3]heptane-2-carboxylate

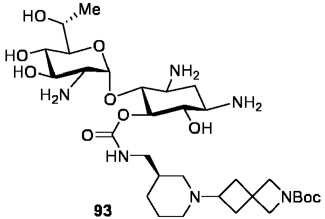

Starting from intermediate **92** (60.0 mg, 0.0799 mmol, 1 eq.), using general procedure **C2,** intermediate **93** was obtained as a white solid (46.0 mg, 86%). LCMS (ESI⁺): RT= 5.45 min, [M+H]⁺=673.9 (Method 5).
**615**

### (1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl (((S)-1-(2-azaspiro[3.3]heptan-6-yl)piperidin-3-yl)methyl)carbamate pentahydrochloride

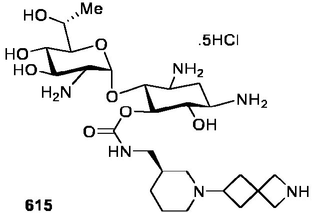

To a solution of intermediate **93** (46.0 mg, 0.0684 mmol, 1 eq.) in DCM (0.7 mL) was added TFA (102 µL, 1.37 mmol, 20 eq.) at room temperature. The reaction mixture was stirred at 25 °C for 2 h and then concentrated under reduced pressure. The resulting residue was dissolved in MeOH (0.6 mL) and Amberlyst A26(-OH) (~500 mg) was added at room temperature. The reaction mixture was stirred at 25 °C for 1 h, then filtered, rinsed with MeOH (3x2 mL) and the resulting filtrate was concentrated under reduced pressure. The obtained solid was dissolved in a 1.25N HCl solution in MeOH (10 eq.) and the resulting solution was stirred at 25 °C for 0.5 h. The reaction mixture was concentrated under reduced pressure. The resulting solid was dissolved in a minimum of MeOH (0.2 mL) and precipitated with Et₂O (3 mL). The resulting mixture was centrifugated (4000 rpm, 5 min) and the supernatant was removed. This procedure was repeated two times and the resulting solid was vacuum-dried at 40 °C for 2 h to afford **615** as a white solid (37.0 mg, 72%). LCMS (ESI⁺): RT= 5.73 min, [M-5HCl+H]⁺=573.8 (Method 5). ¹H NMR (400 MHz, D₂O): *δ* 5.44 (d, *J* = 4.0 Hz, 1H), 4.93 (t, *J* = 9.3 Hz, 1H), 4.31-4.21 (m, 3H), 4.20-4.12 (m, 3H), 3.92 (t, *J* = 9.7 Hz, 1H), 3.81-3.74 (m, 2H), 3.72-3.58 (m, 2H), 3.51-3.39 (m, 5H), 3.26 (dd, *J* = 14.5, 6.7 Hz, 1H), 3.09 (dd, *J* = 14.2, 5.1 Hz, 1H), 2.85-2.76 (m, 2H), 2.68 (t, *J* = 12.5 Hz, 1H), 2.58-2.46 (m, 4H), 2.09-1.83 (m, 4H), 1.77-1.61 (m, 1H), 1.32-1.25 (m, 1H), 1.24 (d, *J* = 6.5 Hz, 3H). All exchangeable protons are missing.

### Preparation of 972

### Synthetic scheme

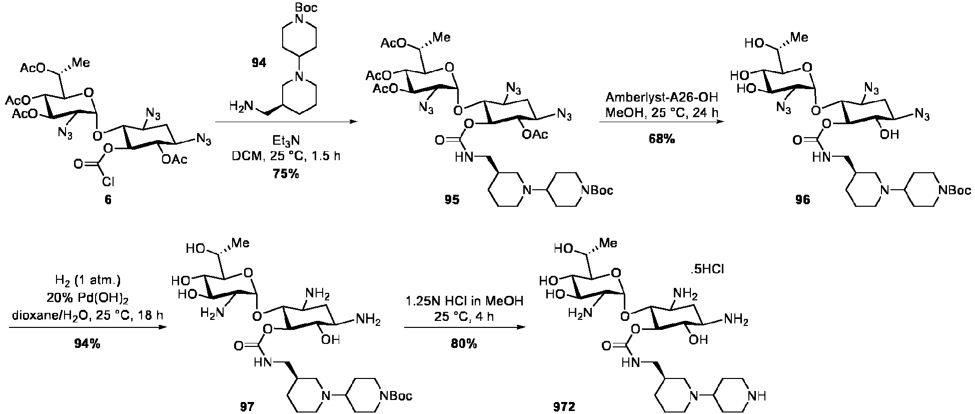

### Compound 95

### (2R,3S,4R,5R,6S)-6-(((1R,2S,3S,4R,6S)-3-acetoxy-4,6-diazido-2-(((((S)-1'-(tert-butoxycarbonyl)-[1,4'-bipiperidin]-3-yl)methyl)carbamoyl)oxy)cyclohexyl)oxy)-2-((R)-1-acetoxyethyl)-5-azidotetrahydro-2H-pyran-3,4-diyl diacetate

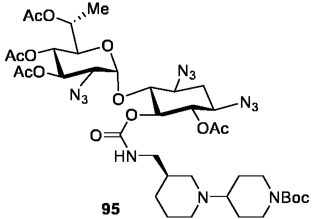

Intermediate **95** was prepared according to general procedure **A,** starting from intermediate **6** (651 mg, 1.01 mmol, 1 eq.) and intermediate **94** (359 mg, 1.21 mmol, 1.2 eq.). The crude residue was purified by silica gel flash chromatography (DCM/MeOH= from 100:0 to 95:5) to afford intermediate **95** as a white solid (690 mg, 75%). LCMS (ESI⁺): RT= 2.73 min, [M+H]⁺=907.6 (Method 1).

### Compound 96

### tert-butyl (S)-3-((((((1R,2R,3S,5R,6S)-3,5-diazido-2-(((2S,3R,4R,5S,6R)-3-azido-4,5-dihydroxy-6-((R)-1-hydroxyethyl)tetrahydro-2H-pyran-2-yl)oxy)-6-hydroxycyclohexyl)oxy)carbonyl)amino)methyl)-[1,4'-bipiperidine]-1'-carboxylate

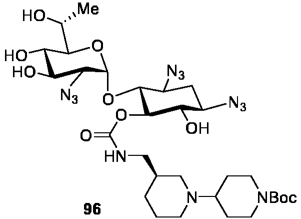

Intermediate **96** was prepared according to general procedure **B,** starting from intermediate **95** (690 mg, 0.761 mmol, 1 eq.). The crude residue was purified by preparative HPLC (column: XBridge C18 (30x150 mm (5µm)); flow Rate: 43 mL/min; gradient: Water (+0.2% NH₄HCO₃)/Acetonitrile = from 65:35 to 50:50) to afford intermediate **96** as a white solid (383 mg, 68%). LCMS (ESI⁺): RT= 7.98 min, [M+H]⁺=739.7 (Method 2).

### Compound 97

### tert-butyl (S)-3-((((((1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl)oxy)carbonyl)amino)methyl)-[1,4'-bipiperidine]-1'-carboxylate

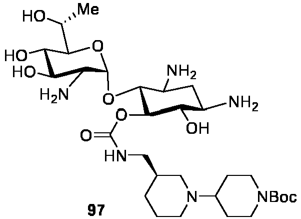

Starting from intermediate **96** (104 mg, 0.141 mmol, 1 eq.), using general procedure **C1,** intermediate **97** was obtained as an off-white solid (87.0 mg, 94%). LCMS (ESI⁺): RT= 5.39 min, [M+H]⁺=661.8 (Method 4).
**972**

### (1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl (((S)-[1,4'-bipiperidin]-3-yl)methyl)carbamate pentahydrochloride

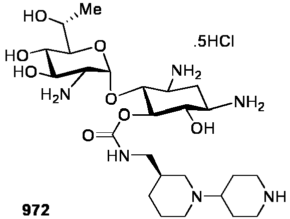

Starting from intermediate **97** (83.0 mg, 0.126 mmol, 1 eq.), using general procedure **D1, 972** was obtained as a white solid (75.0 mg, 80%). LCMS (ESI⁺): RT= 5.99 min, [M-5HCl+H]⁺=561.6 (Method 5).¹H NMR (400 MHz, D₂O): *δ* 5.48 (d, *J* = 4.0 Hz, 1H), 4.97 (t, *J* = 9.3 Hz, 1H), 4.30 (qd, *J* = 6.6, 2.6 Hz, 1H), 4.21 (dd, *J* = 10.0, 9.1 Hz, 1H), 3.96 (dd, *J* = 10.6, 8.9 Hz, 1H), 3.86-3.78 (m, 2H), 3.76-3.65 (m, 3H), 3.65-3.44 (m, 6H), 3.32 (dd, *J* = 14.8, 6.2 Hz, 1H), 3.21-3.10 (m, 3H), 3.10-3.00 (m, 1H), 2.92-2.81 (m, 1H), 2.57 (dt, *J* = 12.6, 4.3 Hz, 1H), 2.48-2.39 (m, 2H), 2.21-2.01 (m, 4H), 2.00-1.88 (m, 2H), 1.88-1.75 (m, 1H), 1.41-1.29 (m, 1H), 1.27 (d, *J* = 6.6 Hz, 3H). All exchangeable protons are missing.

### Preparation of 975

### Synthetic scheme

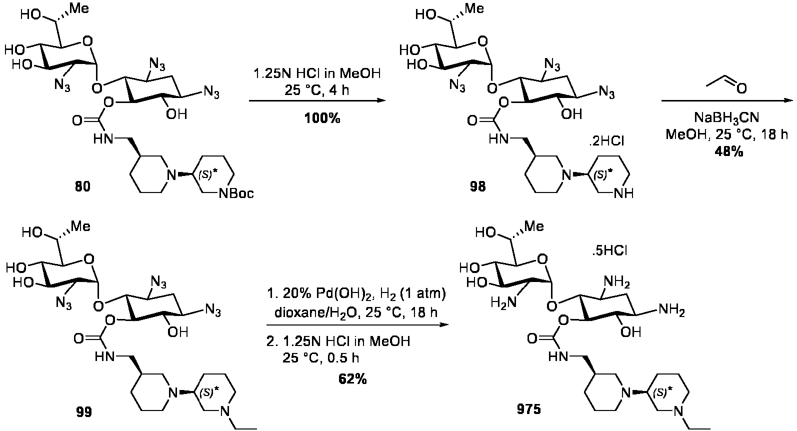

### Compound 98

### (1R,2R,3S,5R,6S)-3,5-diazido-2-(((2S,3R,4R,5S,6R)-3-azido-4,5-dihydroxy-6-((R)-1-hydroxyethyl)tetrahydro-2H-pyran-2-yl)oxy)-6-hydroxycyclohexyl (((3S,3'S*)-[1,3'-bipiperidin]-3-yl)methyl)carbamate dihydrochloride

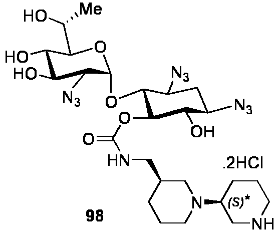

Starting from intermediate **80** (250 mg, 0.338 mmol, 1 eq.), using general procedure **D1,** compound **98** was obtained as a white solid (241 mg, 100%). LCMS (ESI⁺): RT= 5.94 min, [M-2HCl+H]⁺=639.6 (Method 2).

### Compound 99

### (1R,2R,3S,5R,6S)-3,5-diazido-2-(((2S,3R,4R,5S,6R)-3-azido-4,5-dihydroxy-6-((R)-1-hydroxyethyl)tetrahydro-2H-pyran-2-yl)oxy)-6-hydroxycyclohexyl (((3S,3'S*)-1'-ethyl-[1,3'-bipiperidin]-3-yl)methyl)carbamate

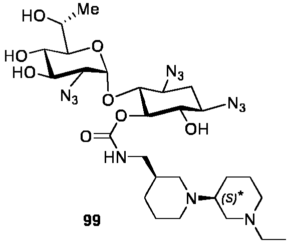

Intermediate **99** was prepared according to general procedure **E3,** starting from intermediate **98** (126 mg, 0.177 mmol, 1 eq.) and acetaldehyde (100 µL, 1.77 mmol, 10 eq.). The crude residue was purified by preparative HPLC (column: XBridge C18 (30x150 mm (5µm)); flow Rate: 43 mL/min; gradient: Water (+0.2% NH₄HCO₃)/Acetonitrile = from 90:10 to 75:25) to afford intermediate **99** as a white solid (57 mg, 48%). LCMS (ESI⁺): RT= 6.85 min, [M+H]⁺=667.9 (Method 2).
**975**

### (1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl (((3S,3'S*)-1'-ethyl-[1,3'-bipiperidin]-3-yl)methyl)carbamate pentahydrochloride

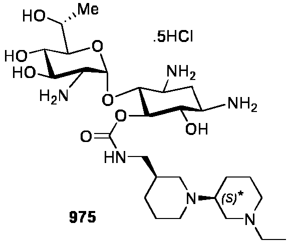

**975** was prepared according to general procedure **C4,** starting from intermediate **99** (50.0 mg, 0.0750 mmol, 1 eq.). The resulting solid was dissolved in H₂O (3 mL) and freeze-dried for 20 h to afford compound **975** as a white solid (36.0 mg, 62%). LCMS (ESI⁺): RT= 5.62 min, [M-5HCl+H]⁺=589.6 (Method 5). ¹H NMR (400 MHz, D₂O): *δ* 5.48 (d, *J* = 4.0 Hz, 1H), 4.97 (t, *J* = 9.3 Hz, 1H), 4.30 (qd, *J* = 6.6, 2.6 Hz, 1H), 4.21 (t, *J* = 9.6 Hz, 1H), 3.97 (dd, *J* = 10.6, 8.9 Hz, 1H), 3.94-3.88 (m, 1H), 3.86-3.79 (m, 2H), 3.76-3.63 (m, 3H), 3.61-3.45 (m, 5H), 3.39-3.27 (m, 4H), 3.19-3.09 (m, 2H), 3.05-2.90 (m, 2H), 2.57 (dt, *J* = 12.6, 4.3 Hz, 1H), 2.40-2.31 (m, 1H), 2.31-2.23 (m, 1H), 2.23-2.07 (m, 2H), 2.00-1.77 (m, 5H), 1.39 (t, *J* = 7.3 Hz, 3H), 1.36-1.29 (m, 1H), 1.27 (d, *J* = 6.5 Hz, 3H). All exchangeable protons are missing.

### Preparation of 597

### Synthetic scheme

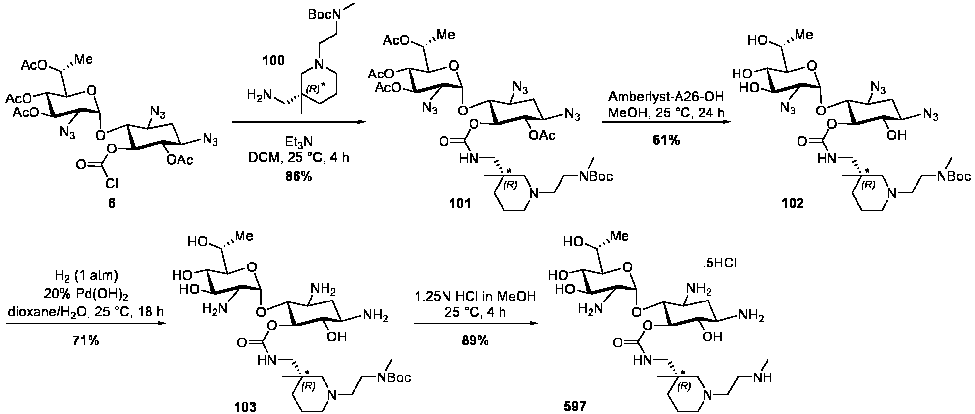

### Compound 101

### (2R,3S,4R,5R,6S)-6-(((1R,2S,3S,4R,6S)-3-acetoxy-4,6-diazido-2-(((((R*)-1-(2-((tert-butoxycarbonyl)(methyl)amino)ethyl)-3-methylpiperidin-3-yl)methyl)carbamoyl)oxy)cyclohexyl)oxy)-2-((R)-1-acetoxyethyl)-5-azidotetrahydro-2H-pyran-3,4-diyl diacetate

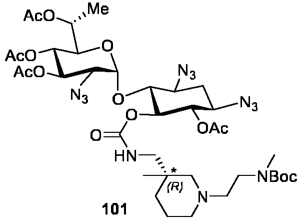

Intermediate **101** was prepared according to general procedure **A,** starting from intermediate **6** (5.04 g, 7.80 mmol, 1 eq.) and intermediate **100** (2.67 g, 9.36 mmol, 1.2 eq.). The crude residue was purified by silica gel flash chromatography (DCM/EtOAc= from 100:0 to 0:100) to afford intermediate **101** as a white solid (5.99 g, 86%). LCMS (ESI⁺): RT= 2.36 min, [M+H]⁺=895.6 (Method 1).

### Compound 102

### tert-butyl (2-((R*)-3-((((((1R,2R,3S,5R,6S)-3,5-diazido-2-(((2S,3R,4R,5S,6R)-3-azido-4,5-dihydroxy-6-((R)-1-hydroxyethyl)tetrahydro-2H-pyran-2-yl)oxy)-6-hydroxycyclohexyl)oxy)carbonyl)amino)methyl)-3-methylpiperidin-1-yl)ethyl)(methyl)carbamate

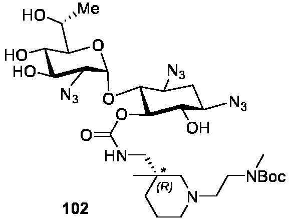

Intermediate **102** was prepared according to general procedure **B,** starting from intermediate **101** (5.99 g, 6.69 mmol, 1 eq.). The crude residue was purified by preparative HPLC (column: XBridge C18 (30x150 mm (5µm)); flow Rate: 43 mL/min; gradient: Water (+0.2% NH₄HCO₃)/Acetonitrile = from 65:35 to 50:50) to afford intermediate **102** as an off-white solid (2.96 g, 61%). LCMS (ESI⁺): RT= 8.92 min, [M+H]⁺=727.9 (Method 2).

### Compound 103

### tert-butyl (2-((R*)-3-((((((1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl)oxy)carbonyl)amino)methyl)-3-methylpiperidin-1-yl)ethyl)(methyl)carbamate

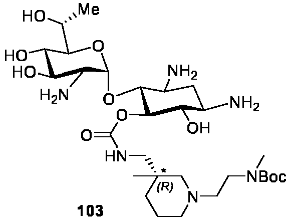

Starting from intermediate **102** (2.96 g, 4.07 mmol, 1 eq.), using general procedure **C1,** intermediate **103** was obtained as an off-white solid (1.87 g, 71%). LCMS (ESI⁺): RT= 4.41 min, [M+H]⁺=649.7 (Method 5).
**597**

### (1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl (((R*)-3-methyl-1-(2-(methylamino)ethyl)piperidin-3-yl)methyl)carbamate pentahydrochloride

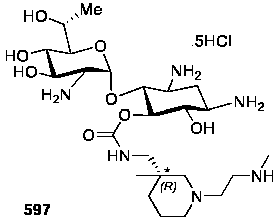

**597** was prepared according to general procedure **D1,** starting from intermediate **103** (1.87 g, 2.88 mmol, 1 eq.). The resulting solid was dissolved in H₂O (15 mL) and freeze-dried for 48 h to afford compound **597** as an off-white solid (1.87 g, 89%). LCMS (ESI⁺): RT= 5.86 min, [M-5HCl+H]⁺=549.6 (Method 5). ¹H NMR (400 MHz, D₂O): *δ* 5.51 (d, *J* = 4.0 Hz, 1H), 4.98 (t, *J* = 9.3 Hz, 1H), 4.31 (qd, *J* = 6.5, 2.6 Hz, 1H), 4.24 (t, *J =* 9.6 Hz, 1H), 3.99 (dd, *J* = 10.6, 8.8 Hz, 1H), 3.88-3.81 (m, 2H), 3.79-3.70 (m, 1H), 3.69-3.27 (m, 10H), 3.24-2.92 (m, 3H), 2.85 (s, 3H), 2.59 (dt, *J* = 12.7, 4.3 Hz, 1H), 2.07-1.90 (m, 3H), 1.82-1.46 (m, 2H), 1.28 (d, *J* = 6.5 Hz, 3H), 1.21-0.97 (m, 3H). All exchangeable protons are missing.

### Preparation of 577

### Synthetic scheme

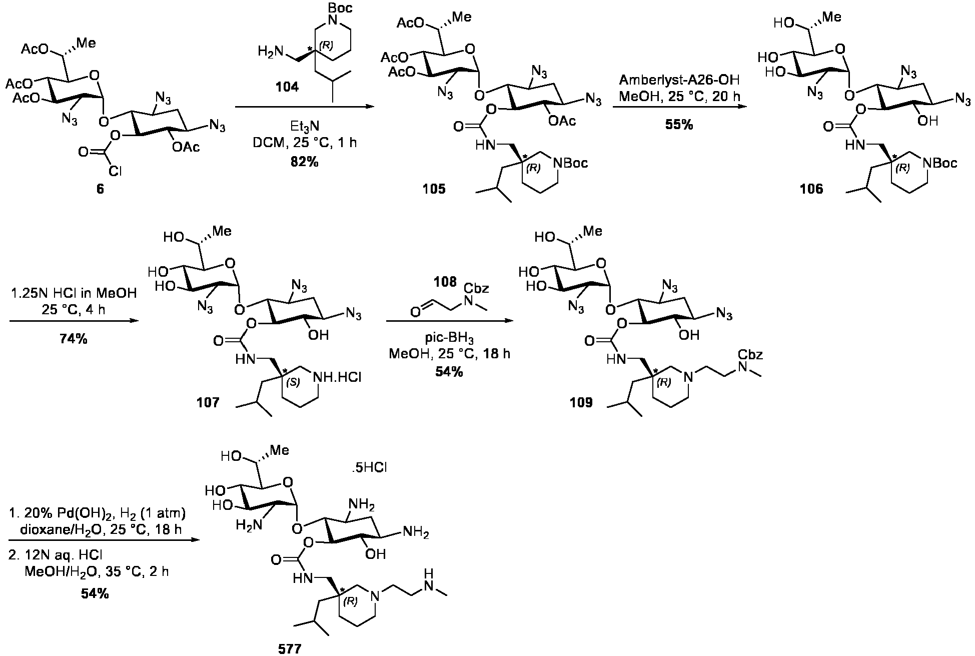

### Compound 105

### (2R,3S,4R,5R,6S)-6-(((1R,2S,3S,4R,6S)-3-acetoxy-4,6-diazido-2-(((((R*)-1-(tert-butoxycarbonyl)-3-isobutylpiperidin-3-yl)methyl)carbamoyl)oxy)cyclohexyl)oxy)-2-((R)-1-acetoxyethyl)-5-azidotetrahydro-2H-pyran-3,4-diyl diacetate

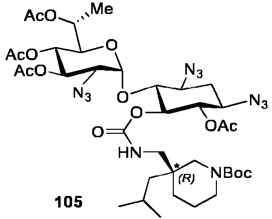

Intermediate **105** was prepared according to general procedure **A,** starting from intermediate **6** (400 mg, 0.619 mmol, 1 eq.) and intermediate **104** (201 mg, 0.743 mmol, 1.2 eq.). The crude residue was purified by silica gel flash chromatography (Cyclohexane/EtOAc= from 100:0 to 50:50) to afford intermediate **105** as a white solid (447 mg, 82%). LCMS (ESI⁺): RT= 3.72 min, [M-C₅H₈O₂+H]⁺=780.5 (Method 1).

### Compound 106

### tert-butyl (R*)-3-((((((1R,2R,3S,5R,6S)-3,5-diazido-2-(((2S,3R,4R,5S,6R)-3-azido-4,5-dihydroxy-6-((R)-1-hydroxyethyl)tetrahydro-2H-pyran-2-yl)oxy)-6-hydroxycyclohexyl)oxy)carbonyl)amino)methyl)-3-isobutylpiperidine-1-carboxylate

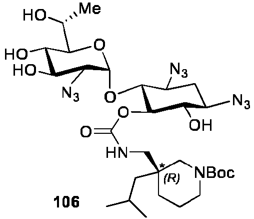

Intermediate **106** was prepared according to general procedure **B,** starting from intermediate **105** (447 mg, 0.508 mmol, 1 eq.). The crude residue was purified by preparative HPLC (column: XBridge C18 (30x150 mm (5µm)); flow Rate: 43 mL/min; gradient: Water (+0.2% NH₄HCO₃)/Acetonitrile = from 60:40 to 45:55) to afford intermediate **106** as a white solid (200 mg, 55%). LCMS (ESI⁺): RT= 10.3 min, [M-C₅H₈O₂+H]⁺=612.2 (Method 2).

### Compound 107

### (1R,2R,3S,5R,6S)-3,5-diazido-2-(((2S,3R,4R,5S,6R)-3-azido-4,5-dihydroxy-6-((R)-1-hydroxyethyl)tetrahydro-2H-pyran-2-yl)oxy)-6-hydroxycyclohexyl (((S*)-3-isobutylpiperidin-3-yl)methyl)carbamate hydrochloride

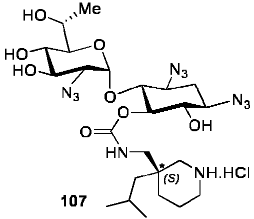

Starting from intermediate **106** (133 mg, 0.187 mmol, 1 eq.), using general procedure **D1,** intermediate **107** was obtained as a white solid (90.0 mg, 74%). LCMS (ESI⁺): RT= 1.70 min, [M-HCl+H]⁺=612.8 (Method 1).

### Compound 109

### benzyl (2-((R*)-3-((((((1R,2R,3S,5R,6S)-3,5-diazido-2-(((2S,3R,4R,5S,6R)-3-azido-4,5-dihydroxy-6-((R)-1-hydroxyethyl)tetrahydro-2H-pyran-2-yl)oxy)-6-hydroxycyclohexyl)oxy)carbonyl)amino)methyl)-3-isobutylpiperidin-1-yl)ethyl)(methyl)carbamate

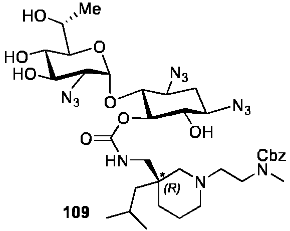

Intermediate **109** was prepared according to general procedure **E2,** starting from intermediate **107** (90.0 mg, 0.139 mmol, 1 eq.) and intermediate **108** (34.5 mg, 0.167 mmol, 1.2 eq.). The crude residue was purified by silica gel flash chromatography (DCM/MeOH= from 100:0 to 80:20) to afford intermediate **109** as a white solid (60.0 mg, 54%). LCMS (ESI⁺): RT= 1.98 min, [M+H]⁺=804.0 (Method 1).
**577**

### (1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl (((R*)-3-isobutyl-1-(2-(methylamino)ethyl)piperidin-3-yl)methyl)carbamate pentahydrochloride

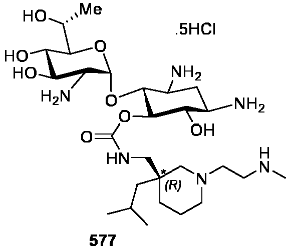

Starting from intermediate **109** (60.0 mg, 0.0747 mmol, 1 eq.), using general procedure **C3, 577** was obtained as a white solid (31.4 mg, 54%). LCMS (ESI⁺): RT= 5.59 min, [M-5HCl+H]⁺=591.6 (Method 5).¹H NMR (400 MHz, D₂O): *δ* 5.48 (d, *J* = 4.0 Hz, 1H), 4.97 (t, *J* = 9.2 Hz, 1H), 4.30 (q, *J* = 7.6 Hz, 1H), 4.22 (t, *J =* 9.6 Hz, 1H), 4.01-3.92 (m, 1H), 3.85-3.77 (m, 2H), 3.76-3.66 (m, 1H), 3.64-3.40 (m, 9H), 3.35-2.91 (m, 4H), 2.84 (s, 3H), 2.61-2.53 (m, 1H), 2.07-1.86 (m, 3H), 1.81-1.56 (m, 3H), 1.55-1.31 (m, 2H), 1.27 (d, *J* = 6.5 Hz, 3H), 0.98 (d, *J* = 6.4 Hz, 6H). All exchangeable protons are missing.

### Preparation of 720

### Synthetic scheme

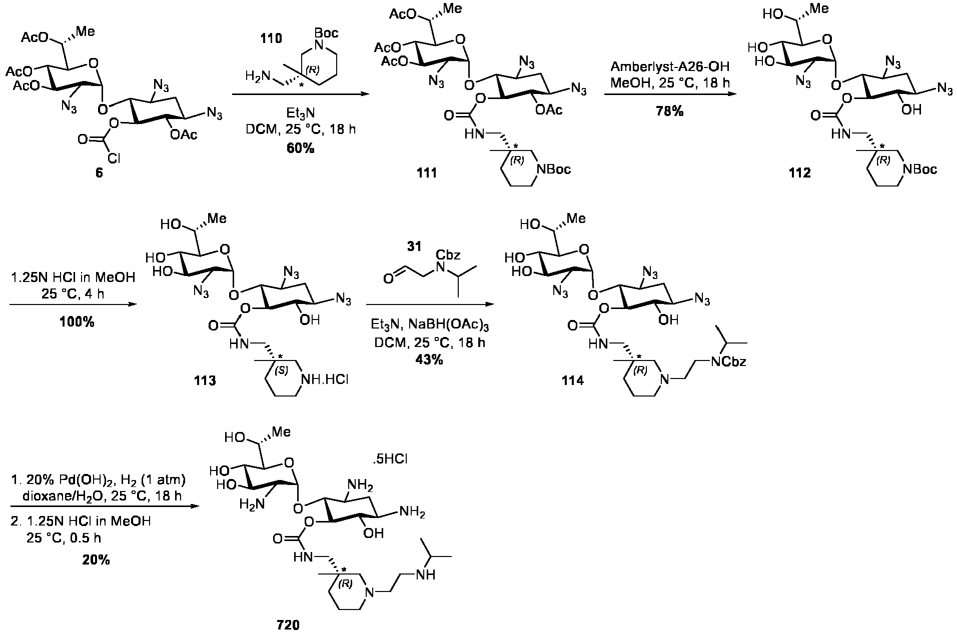

### Compound 111

### (2R,3S,4R,5R, 6S)-6-(((1R,2S,3S,4R,6S)-3-acetoxy-4,6-diazido-2-(((((R*)-1-(tert-butoxycarbonyl)-3-methylpiperidin-3-yl)methyl)carbamoyl)oxy)cyclohexyl)oxy)-2-((R)-1-acetoxyethyl)-5-azidotetrahydro-2H-pyran-3,4-diyl diacetate

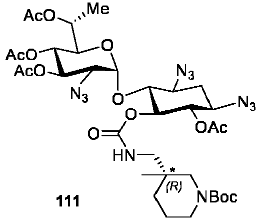

Intermediate **111** was prepared according to general procedure **A,** starting from intermediate **6** (1.37 g, 2.12 mmol, 1 eq.) and intermediate **110** (581 mg, 2.55 mmol, 1.2 eq.). The crude residue was purified by silica gel flash chromatography (DCM/EtOAc= from 100:0 to 50:50) to afford intermediate **111** as a white solid (1.06 g, 60%). LCMS (ESI⁺): RT= 10.9 min, [M+H]⁺=838.7 (Method 2).

### Compound 112

### tert-butyl (R*)-3-((((((1R,2R,3S,5R,6S)-3,5-diazido-2-(((2S,3R,4R,5S,6R)-3-azido-4,5-dihydroxy-6-((R)-1-hydroxyethyl)tetrahydro-2H-pyran-2-yl)oxy)-6-hydroxycyclohexyl)oxy)carbonyl)amino)methyl)-3-methylpiperidine-1-carboxylate

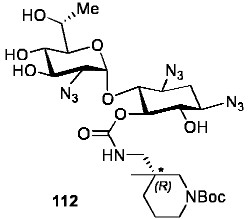

Intermediate **112** was prepared according to general procedure **B,** starting from intermediate **111** (1.05 g, 1.25 mmol, 1 eq.). The crude residue was purified by preparative HPLC (column: XBridge C18 (30x150 mm (5µm)); flow Rate: 43 mL/min; gradient: Water (+0.2% NH₄HCO₃)/Acetonitrile = from 60:40 to 45:55) to afford intermediate **112** as a white solid (655 mg, 78%). LCMS (ESI⁺): RT= 8.30 min, [M+H]⁺=670.6 (Method 2).

### Compound 113

### (1R,2R,3S,5R,6S)-3,5-diazido-2-(((2S,3R,4R,5S,6R)-3-azido-4,5-dihydroxy-6-((R)-1-hydroxyethyl)tetrahydro-2H-pyran-2-yl)oxy)-6-hydroxycyclohexyl (((S*)-3-methylpiperidin-3-yl)methyl)carbamate hydrochloride

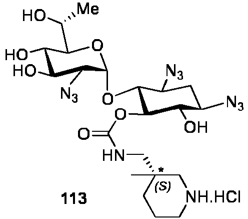

Starting from intermediate **112** (655 mg, 0.978 mmol, 1 eq.), using general procedure **D1,** intermediate **113** was obtained as a white solid (592 mg, 100%). LCMS (ESI⁺): RT= 5.52 min, [M-HCl+H]⁺=570.5 (Method 2).

### Compound 114

### benzyl (2-((R*)-3-((((((1R,2R,3S,5R,6S)-3,5-diazido-2-(((2S,3R,4R,5S,6R)-3-azido-4,5-dihydroxy-6-((R)-1-hydroxyethyl)tetrahydro-2H-pyran-2-yl)oxy)-6-hydroxycyclohexyl)oxy)carbonyl)amino)methyl)-3-methylpiperidin-1-yl)ethyl)(isopropyl)carbamate

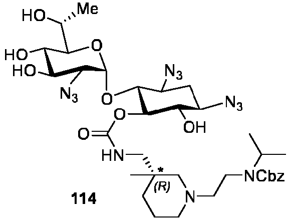

Intermediate **114** was prepared according to general procedure **E1,** starting from intermediate **113** (121 mg, 0.200 mmol, 1 eq.) and intermediate **31** (56.4 mg, 0.240 mmol, 1.2 eq.). The crude residue was purified by silica gel flash chromatography (DCM/MeOH= from 100:0 to 90:10) to afford impure compound **114** (88 mg). Impure **114** was then purified by preparative HPLC (column: XBridge C18 (30x150 mm (5µm)); flow Rate: 43 mL/min; gradient: Water (+0.2% NH₄HCO₃)/Acetonitrile = from 55:45 to 40:60) to afford pure intermediate **114** as a white solid (67.0 mg, 43%). LCMS (ESI⁺): RT= 9.47 min, [M+H]⁺=789.9 (Method 2).
**720**

### (1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl (((R*)-1-(2-(isopropylamino)ethyl)-3-methylpiperidin-3-yl)methyl)carbamate pentahydrochloride

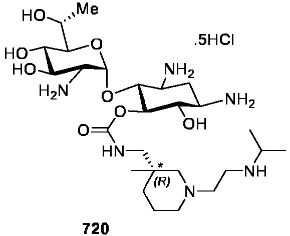

Starting from intermediate **114** (67.0 mg, 0.0849 mmol, 1 eq.), using general procedure **C4, 720** was obtained as a white solid (13.0 mg, 20%). LCMS (ESI⁺): RT= 5.77 min, [M-5HCl+H]⁺=577.6 (Method 5).¹H NMR (400 MHz, D₂O): *δ* 5.48 (d, *J* = 4.0 Hz, 1H), 4.97 (t, *J* = 9.3 Hz, 1H), 4.34-4.27 (m, 1H), 4.21 (t, *J = 9.6* Hz, 1H), 3.99-3.91 (m, 1H), 3.86-3.77 (m, 2H), 3.75-3.66 (m, 1H), 3.61-3.34 (m, 10H), 3.30-2.94 (m, 4H), 2.61-2.53 (m, 1H), 2.03-1.87 (m, 3H), 1.69-1.45 (m, 2H), 1.39 (d, *J* = 6.6 Hz, 6H), 1.27 (d, *J* = 6.6 Hz, 3H), 1.10 (s, 3H). All exchangeable protons are missing.

### Preparation of 834

### Synthetic scheme

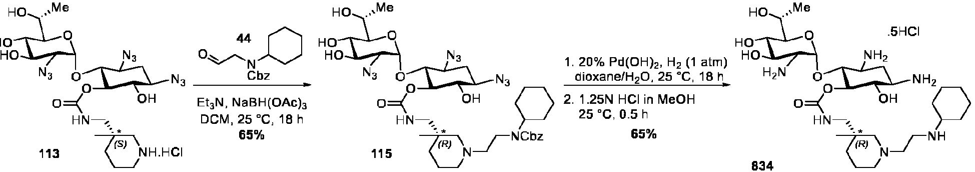

### Compound 115

### benzyl cyclohexyl(2-((R*)-3-((((((1R,2R,3S,5R,6S)-3,5-diazido-2-(((2S,3R,4R,5S,6R)-3-azido-4,5-dihydroxy-6-((R)-1-hydroxyethyl)tetrahydro-2H-pyran-2-yl)oxy)-6-hydroxycyclohexyl)oxy)carbonyl)amino)methyl)-3-methylpiperidin-1-yl)ethyl)carbamate

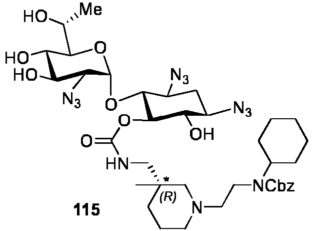

Intermediate **115** was prepared according to general procedure **E1,** starting from intermediate **113** (191 mg, 0.316 mmol, 1 eq.) and intermediate **44** (104 mg, 0.379 mmol, 1.2 eq.). The crude residue was purified by silica gel flash chromatography (DCM/MeOH= from 100:0 to 80:20) to afford intermediate **115** as a white solid (170 mg, 65%). LCMS (ESI⁺): RT= 10.5 min, [M+H]⁺=830.0 (Method 2).
**834**

### (1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl (((R*)-1-(2-(cyclohexylamino)ethyl)-3-methylpiperidin-3-yl)methyl)carbamate pentahydrochloride

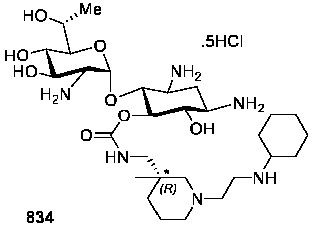

Starting from intermediate **115** (70.0 mg, 0.0844 mmol, 1 eq.), using general procedure **C4, 834** was obtained as a white solid (44.0 mg, 65%). LCMS (ESI⁺): RT= 5.27 min, [M-5HCl+H]⁺=617.7 (Method 5).¹H NMR (400 MHz, D₂O): *δ* 5.49 (d, *J* = 4.0 Hz, 1H), 4.97 (t, *J* = 9.2 Hz, 1H), 4.34-4.27 (m, 1H), 4.22 (t, *J = 9.5* Hz, 1H), 3.97 (t, *J =* 10.1 Hz, 1H), 3.86-3.78 (m, 2H), 3.76-3.67 (m, 1H), 3.67-3.38 (m, 9H), 3.29-2.85 (m, 5H), 2.63-2.51 (m, 1H), 2.19-2.06 (m, 2H), 2.05-1.82 (m, 5H), 1.76-1.47 (m, 3H), 1.47-1.33 (m, 4H), 1.27 (d, *J* = 6.6 Hz, 3H), 1.25-1.18 (m, 1H), 1.11 (s, 3H). All exchangeable protons are missing.

### Preparation of 835

### Synthetic scheme

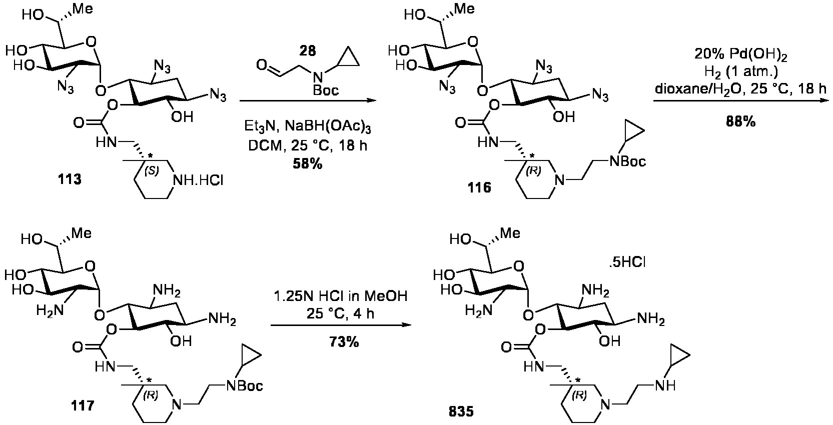

### Compound 116

### tert-butyl cyclopropyl(2-((R*)-3-((((((1R,2R,3S,5R,6S)-3,5-diazido-2-(((2S,3R,4R,5S,6R)-3-azido-4,5-dihydroxy-6-((R)-1-hydroxyethyl)tetrahydro-2H-pyran-2-yl)oxy)-6-hydroxycyclohexyl)oxy)carbonyl)amino)methyl)-3-methylpiperidin-1-yl)ethyl)carbamate

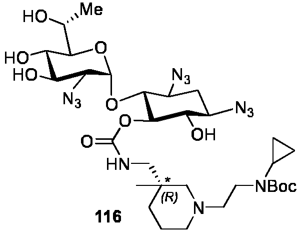

Intermediate **116** was prepared according to general procedure **E1,** starting from intermediate **113** (174 mg, 0.287 mmol, 1 eq.) and intermediate **28** (85.8 mg, 0.431 mmol, 1.5 eq.). The crude residue was purified by silica gel flash chromatography (DCM/MeOH= from 100:0 to 80:20) to afford intermediate **116** as a white solid (125 mg, 58%). LCMS (ESI⁺): RT= 9.51 min, [M+H]⁺=753.9 (Method 2).

### Compound 117

### tert-butyl cyclopropyl(2-((R*)-3-((((((1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl)oxy)carbonyl)amino)methyl)-3-methylpiperidin-1-yl)ethyl)carbamate

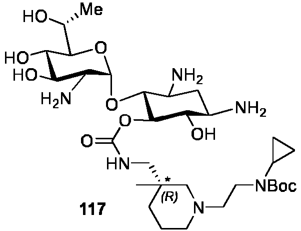

Starting from intermediate **116** (70.0 mg, 0.0930 mmol, 1 eq.), using general procedure **C1,** compound **117** was obtained as a white solid (55.0 mg, 88%). LCMS (ESI⁺): RT= 4.23 min, [M+H]⁺=675.9 (Method 5).
**835**

### (1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl (((R*)-1-(2-(cyclopropylamino)ethyl)-3-methylpiperidin-3-yl)methyl)carbamate pentahydrochloride

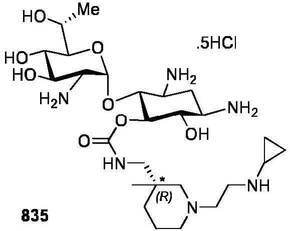

Starting from intermediate **117** (55 mg, 0.0815 mmol, 1 eq.), using general procedure **D1, 835** was obtained as a white solid (44.7 mg, 73%). LCMS (ESI⁺): RT= 5.69 min, [M-5HCl+H]⁺=575.6 (Method 5).¹H NMR (400 MHz, D₂O): *δ* 5.49 (d, *J =* 3.8 Hz, 1H), 4.97 (t, *J =* 9.2 Hz, 1H), 4.34-4.26 (m, 1H), 4.22 (t, *J =* 10.0 Hz, 1H), 3.97 (t, *J =* 8.9 Hz, 1H), 3.86-3.78 (m, 2H), 3.77-3.66 (m, 3H), 3.61-3.44 (m, 6H), 3.33-2.93 (m, 4H), 2.90-2.79 (m, 1H), 2.63-2.51 (m, 1H), 2.06-1.85 (m, 3H), 1.71-1.45 (m, 2H), 1.26 (d, *J =* 6.6 Hz, 3H), 1.23-1.17 (m, 1H), 1.11 (s, 3H), 1.02-0.93 (m, 4H). All exchangeable protons are missing.

### Preparation of 829 & 831

### Synthesis of key intermediates 120 & 121

### Synthetic scheme

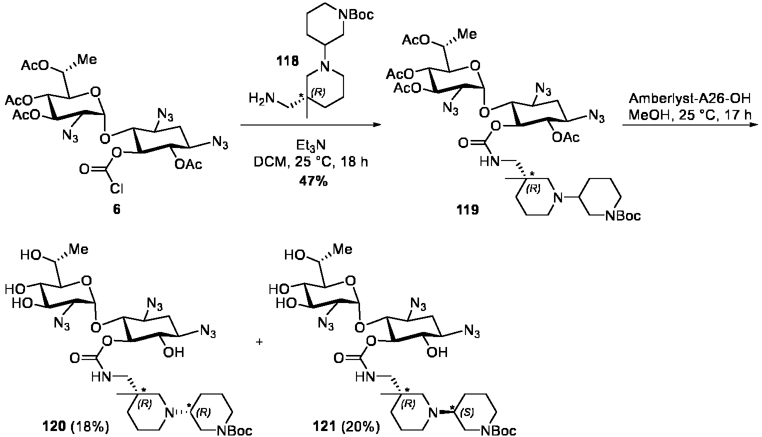

### Compound 119

### (2R,3S,4R,5R,6S)-6-(((1R,2S,3S,4R,6S)-3-acetoxy-4,6-diazido-2-(((((3R*)-1'-(tert-butoxycarbonyl)-3-methyl-[1,3'-bipiperidin]-3-yl)methyl)carbamoyl)oxy)cyclohexyl)oxy)-2-((R)-1-acetoxyethyl)-5-azidotetrahydro-2H-pyran-3,4-diyl diacetate

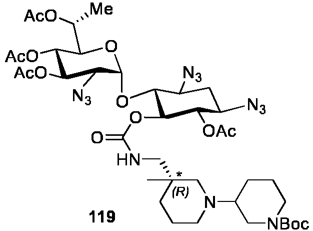

Intermediate **119** was prepared according to general procedure **A,** starting from intermediate **6** (431 mg, 0.668 mmol, 1 eq.) and intermediate **118** (250 mg, 0.802 mmol, 1.2 eq.). The crude residue was purified by silica gel flash chromatography (DCM/MeOH= from 100:0 to 90:10) to afford intermediate **119** as a mixture of diastereomers, as a white solid (286 mg, 47%). LCMS (ESI⁺): RT= 11.8 min, [M+H]⁺=922.0 (Method 2).

### Compound 120

### tert-butyl (3R*,3'R*)-3-((((((1R,2R,3S,5R,6S)-3,5-diazido-2-(((2S,3R,4R,5S,6R)-3-azido-4,5-dihydroxy-6-((R)-1-hydroxyethyl)tetrahydro-2H-pyran-2-yl)oxy)-6-hydroxycyclohexyl)oxy)carbonyl)amino)methyl)-3-methyl-[1,3'-bipiperidine]-1'-carboxylate

### & Compound 121

### tert-butyl (3R*,3'S*)-3-((((((1R,2R,3S,5R,6S)-3,5-diazido-2-(((2S,3R,4R,5S,6R)-3-azido-4,5-dihydroxy-6-((R)-1-hydroxyethyl)tetrahydro-2H-pyran-2-yl)oxy)-6-hydroxycyclohexyl)oxy)carbonyl)amino)methyl)-3-methyl-[1,3'-bipiperidine]-1'-carboxylate

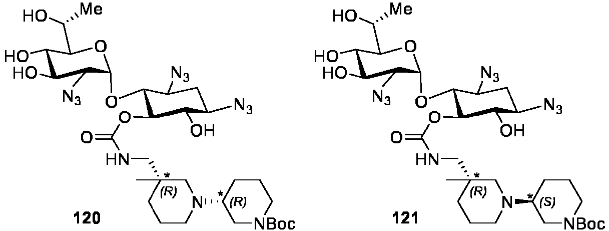

Intermediates **120** and **121** were prepared according to general procedure **B,** starting from intermediate **119** (286 mg, 0.311 mmol, 1 eq.). The crude residue was purified by preparative HPLC (column: XBridge C18 (30x150 mm (5µm)); flow Rate: 43 mL/min; gradient: Water (+0.2% NH₄HCO₃)/Acetonitrile = from 65:35 to 50:50) to afford intermediate **120** (42.5 mg, 18%) and intermediate **121** (47.2 mg, 20%) as white solids.

Compound **120:** LCMS (ESI⁺): RT= 9.40 min, [M+H]⁺=754.0 (Method 2).

Compound **121:** LCMS (ESI⁺): RT= 9.52 min, [M+H]⁺=753.9 (Method 2).

**Note:** The stereochemistry "*R**" of the *N*-Boc protected piperidine ring was arbitrarily assigned to the first eluted product by preparative HPLC purification and then the second eluted product was assigned "*S**".

### Preparation of 829

### Synthetic scheme

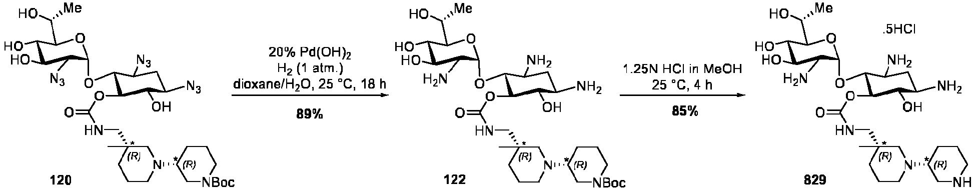

### Compound 122

### tert-butyl (3R*,3'R*)-3-((((((1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl)oxy)carbonyl)amino)methyl)-3-methyl-[1,3'-bipiperidine]-1'-carboxylate

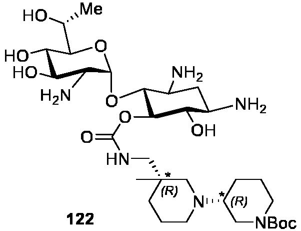

Starting from intermediate **120** (40.0 mg, 0.0531 mmol, 1 eq.), using general procedure **C1,** compound **122** was obtained as a white solid (32.0 mg, 89%). LCMS (ESI⁺): RT= 5.44 min, [M+H]⁺=675.7 (Method 4)
**829**

### (1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl (((3R*,3'R*)-3-methyl-[1,3'-bipiperidin]-3-yl)methyl)carbamate pentahydrochloride

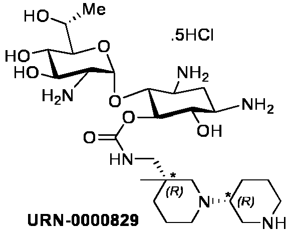

Starting from intermediate **122** (31.0 mg, 0.0459 mmol, 1 eq.), using general procedure **D1, 829** was obtained as a white solid (29.0 mg, 85%). LCMS (ESI⁺): RT= 5.86 min, [M-5HCl+H]⁺=575.6 (Method 5).¹H NMR (400 MHz, D₂O): *δ* 5.48 (d, *J* = 4.1 Hz, 1H), 4.97 (t, *J* = 9.3 Hz, 1H), 4.30 (qd, *J* = 6.5, 2.5 Hz, 1H), 4.21 (t, *J* = 9.6 Hz, 1H), 3.96 (dd, *J* = 10.6, 8.9 Hz, 1H), 3.93-3.88 (m, 1H), 3.86-3.77 (m, 2H), 3.75-3.67 (m, 1H), 3.67-3.60 (m, 1H), 3.56-3.35 (m, 6H), 3.33-3.14 (m, 3H), 3.14-2.95 (m, 3H), 2.57 (dt, *J* = 12.7, 4.3 Hz, 1H), 2.42-2.31 (m, 1H), 2.26-2.17 (m, 1H), 2.06-1.78 (m, 5H), 1.70-1.46 (m, 2H), 1.27 (d, *J =* 6.6 Hz, 3H), 1.12 (s, 3H). All exchangeable protons are missing.

### Preparation of 831

### Synthetic scheme

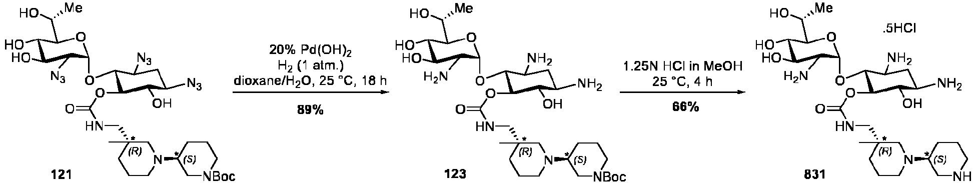

### Compound 123

### tert-butyl (3R*,3'R*)-3-((((((1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl)oxy)carbonyl)amino)methyl)-3-methyl-[1,3'-bipiperidine]-1'-carboxylate

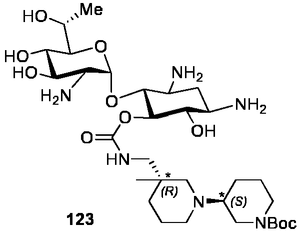

Starting from intermediate **121** (44.0 mg, 0.0584 mmol, 1 eq.), using general procedure **C1,** compound **123** was obtained as a white solid (35.0 mg, 89%). LCMS (ESI⁺): RT= 5.43 min, [M+H]⁺=675.7 (Method 4)
**831**

### (1R,2R,3S,5R,6S)-3,5-diamino-2-(((2S,3R,4R,5S,6R)-3-amino-4,5-dihydroxy-6-((R)-1-hydroxyethyl)tetrahydro-2H-pyran-2-yl)oxy)-6-hydroxycyclohexyl (((3R*,3'R*)-3-methyl-[1,3'-bipiperidin]-3-yl)methyl)carbamate pentahydrochloride

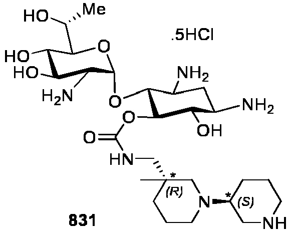

Starting from intermediate **123** (34.0 mg, 0.0504 mmol, 1 eq.), using general procedure **D1, 831** was obtained as a white solid (25.0 mg, 66%). LCMS (ESI⁺): RT= 5.85 min, [M-5HCl+H]⁺=575.6 (Method 5).¹H NMR (400 MHz, D₂O): *δ* 5.49 (d, *J* = 4.0 Hz, 1H), 4.98 (t, *J* = 9.2 Hz, 1H), 4.30 (qd, *J* = 6.5, 2.5 Hz, 1H), 4.22 (dd, *J* = 10.0, 9.2 Hz, 1H), 3.97 (dd, *J* = 10.6, 8.8 Hz, 1H), 3.92-3.85 (m, 1H), 3.85-3.79 (m, 2H), 3.76-3.68 (m, 1H), 3.68-3.63 (m, 1H), 3.55-3.44 (m, 5H), 3.37-3.28 (m, 2H), 3.27-2.97 (m, 5H), 2.57 (dt, *J* = 12.6, 4.3 Hz, 1H), 2.46-2.35 (m, 1H), 2.27-2.18 (m, 1H), 2.08-1.80 (m, 5H), 1.71-1.46 (m, 2H), 1.27 (d, *J* = 6.6 Hz, 3H), 1.11 (s, 3H). All exchangeable protons are missing.

### LCMS methods

### Method 1

LC-MS data was generated using a Waters 2695 e system: Waters PDA 2998 detector, Waters QDA detector (ESI), Sedere SEDEX 80 (light scattering detector).
LC-MS method: reverse phase HPLC analysis
Column Agilent: Cortecs C18, Length: 30 mm, Internal diameter: 3 mm, Particle size: 2.7 µm
Solvent A: Water with Formic Acid (0.1% V/V)
Solvent B: Acetonitrile
UV detection: 220 nm
Gradient table:

| Time (min) | Flow (mL/min) | %A | %B | Curve |
|---|---|---|---|---|
| 0 | 0.7 | 98 | 2 | initial |
| 2 | 0.7 | 0 | 100 | Linear |
| 2.5 | 0.7 | 0 | 100 | Linear |
| 2.6 | 0.7 | 98 | 2 | Linear |
| 5 | 0.7 | 98 | 2 | Linear |

### Method 2

LC-MS data was generated using a Waters 2695 e system: Waters PDA 2998 detector, Waters QDA detector (ESI), Sedere SEDEX 80 (light scattering detector).
LC/MS method: reverse phase HPLC analysis
Column Agilent: Poroshell, Length: 100 mm, Internal diameter: 4.6 mm, Particle size: 4 µm
Solvent A: Water with Ammonium Bicarbonate (0.2% V/W = 20 mM)
Solvent B: Acetonitrile
UV detection: 220 nm
ELSD detection (if required): T=45°C, Gain=5, Filter=2s
Gradient table:

| Time (min) | Flow (mL/min) | %A | %B | Curve |
|---|---|---|---|---|
| 0 | 1 | 98 | 2 | initial |
| 2 | 1 | 98 | 2 | 6 |
| 12 | 1 | 0 | 100 | 6 |
| 15.4 | 1 | 0 | 100 | 6 |
| 16.7 | 1 | 98 | 2 | 6 |
| 18.4 | 1 | 98 | 2 | 6 |

### Method 3

LC-MS data was generated using a Waters 2695 e system: Waters PDA 2998 detector, Waters QDA detector (ESI), Sedere SEDEX 80 (light scattering detector).
LC/MS method: reverse phase HPLC analysis
Column Agilent: Poroshell, Length: 100 mm, Internal diameter: 4.6 mm, Particle size: 4 µm
Solvent A: Water with Formic Acid (0.1% V/V)
Solvent B: Acetonitrile
UV detection: 220 nm
ELSD detection (if required): T=45°C, Gain=5, Filter=2s
Gradient table:

| Time (min) | Flow (mL/min) | %A | %B | Curve |
|---|---|---|---|---|
| 0 | 1 | 98 | 2 | initial |
| 2 | 1 | 98 | 2 | 6 |
| 12 | 1 | 0 | 100 | 6 |
| 15.4 | 1 | 0 | 100 | 6 |
| 16.7 | 1 | 98 | 2 | 6 |
| 18.4 | 1 | 98 | 2 | 6 |

### Method 4

LC-MS data was generated using a Waters 2695 e system: Waters PDA 2998 detector, Waters QDA detector (ESI), Sedere SEDEX 80 (light scattering detector).
LC/MS method: reverse phase HPLC analysis
Column Agilent Poroshell, Length: 100 mm, Internal diameter: 4.6 mm, Particle size: 4 µm Solvent A: Water with Trifluoroacetic Acid (0.1% V/V)
Solvent B: Acetonitrile
UV detection: 220 nm
ELSD detection (if required): T=45°C, Gain=5, Filter=2s
Gradient table:

| Time (min) | Flow (mL/min) | %A | %B | Curve |
|---|---|---|---|---|
| 0 | 1 | 98 | 2 | initial |
| 2 | 1 | 98 | 2 | 6 |
| 12 | 1 | 0 | 100 | 6 |
| 15.4 | 1 | 0 | 100 | 6 |
| 16.7 | 1 | 98 | 2 | 6 |
| 18.4 | 1 | 98 | 2 | 6 |

### Method 5

LC-MS data was generated using a Waters 2695 e system: Waters PDA 2998 detector, Waters QDA detector (ESI), Sedere SEDEX 80 (light scattering detector).
LC/MS method: reverse phase HPLC analysis
Column Agilent: Poroshell HILIC-Z, Length: 100 mm, Internal diameter: 4.6 mm, Particle size: 2.7 µm
Solvent A: Water with Trifluoroacetic Acid (0.1% V/V)
Solvent B: Acetonitrile
UV detection: 220 nm
ELSD detection: T=45°C, Gain=5, Filter=2s
Gradient table:

| Time (min) | Flow (mL/min) | %A | %B | Curve |
|---|---|---|---|---|
| 0 | 1 | 5 | 95 | initial |
| 1 | 1 | 5 | 95 | 6 |
| 9 | 1 | 50 | 50 | 6 |
| 11 | 1 | 50 | 50 | 6 |
| 11.2 | 1 | 5 | 95 | 6 |
| 15 | 1 | 5 | 95 | 6 |

Chiral SFC analysis and purification

### Diastereomers separation by chiral SFC

### Analytical conditions for diastereomers separation by chiral SFC

| **Cpd n°** | **Column details** | **Column T °C (°C)** | **Flow rate (mL/min)** | **Detector Wavelength (nm)** | **Injection volume (µL)** | **BPR (PSI)** | **Mobile Phase (isocratic conditions)** |
|---|---|---|---|---|---|---|---|
| **171** | Chiralpak IC-3 (4.6x100 mm) | 35 | 3.5 | 220-410 | 2 | 1500 | CO₂/(*i*-PrOH +0.3% *i*-PrNH₂), 70:30 |
| **172** | | | | | | | |
| **173** | Chiralpak AD-3 (4.6x100 mm) | | | | | | CO₂/(EtOH) +0.3% *i*-PrNH₂), 75:25 |
| **174** | | | | | | | |

### Preparative conditions for diastereomers separation by chiral SFC

| **Cpd n°** | **Column details** | **Column T °C (°C)** | **Flow rate (mL/min)** | **Detector Wavelength (nm)** | **Injection volume (µL)** | **BPR (bar)** | **Mobile Phase (isocratic conditions)** |
|---|---|---|---|---|---|---|---|
| **171** | | 35 | 160 | 220 | 1000 | 100 | CO₂/(*i*-PrOH |
| **172** | Chiralpak IG (30x250 mm) | | | | | | +0.3% *i*-PrNH₂), 70:30 |
| **173** | Chiralpak AD-H (30x250 mm) | | | 215 | | | CO₂/(EtOH) +0.3% *i*-PrNH₂), 75:25 |
| **174** | | | | | | | |

### Analytical data for separated diastereomers by chiral SFC

| **Cpd n°** | **Assigned Stereochemistry** | **Chiral SFC analysis conditions (mobile phase,** isocratic) | **Rt (min)** | **Chiral SFC purity (%)** |
|---|---|---|---|---|
| **171** | *R** | CO₂/(*i*-PrOH +0.3% *i*-PrNH₂), 70:30 | 1.39 | 100 |
| **172** | *S** | | 1.85 | 100 |
| **173** | *R** | CO₂/(EtOH) +0.3% *i*-PrNH₂), 75:25 | 1.08 | 100 |
| **174** | *S** | | 1.37 | 99.5 |

### Results.

The properties of the synthesized compounds were tested.

The *in vitro* readthrough activity of the compounds (expressed as pEC₅₀) using a mammalian coupled transcription / translation system was determined according to the protocols disclosed in the publication by Célia Floquet et al., Rescue of non-sense mutated p53 tumor suppress gene by aminoglycoside, Nucleic Acid Research, 2011, Vol. 39, N°8, pp 3350-3362.

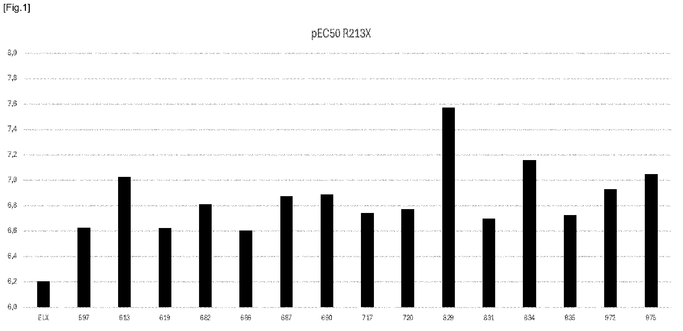

The results are presented in Figure 1 and in the Table below. ELX 02 is a reference compound (Exaluren, CAS N°= 1375073-93-0, commercially available). The results show that the compounds according to the present disclosure can have better readthrough activity than the reference compound.

| In_reg | plC50 prolif DMS114 |
|---|---|
| | <3 |
| | 3≥ x ≥3.5 |
| | >3.5 |
| 244 | <3 |
| 488 | <3 |
| 489 | <3 |
| 510 | 3≥ x ≥3.5 |
| 511 | <3 |
| 525 | 3≥ x ≥3.5 |
| 529 | 3≥ x ≥3.5 |
| 531 | <3 |
| 534 | <3 |
| 538 | 3≥ x ≥3.5 |
| 563 | <3 |
| 577 | <3 |
| 595 | >3.5 |
| 597 | 3≥ x ≥3.5 |
| 613 | >3.5 |
| 614 | <3 |
| 615 | >3.5 |
| 616 | >3.5 |
| 617 | 3≥ x ≥3.5 |
| 619 | >3.5 |
| 682- | >3.5 |
| 685- | 3≥ x ≥3.5 |
| 686- | >3.5 |
| 687- | >3.5 |
| 688- | 3≥ x ≥3.5 |
| 690- | >3.5 |
| 692- | <3 |
| 717- | >3.5 |
| 720- | >3.5 |
| 829 | >3.5 |
| 831- | >3.51 |
| 832- | >3.5 |
| 834 | >3.5 |
| 835- | >3.5 |
| 972- | >3.5 |
| 975- | >3.5 |

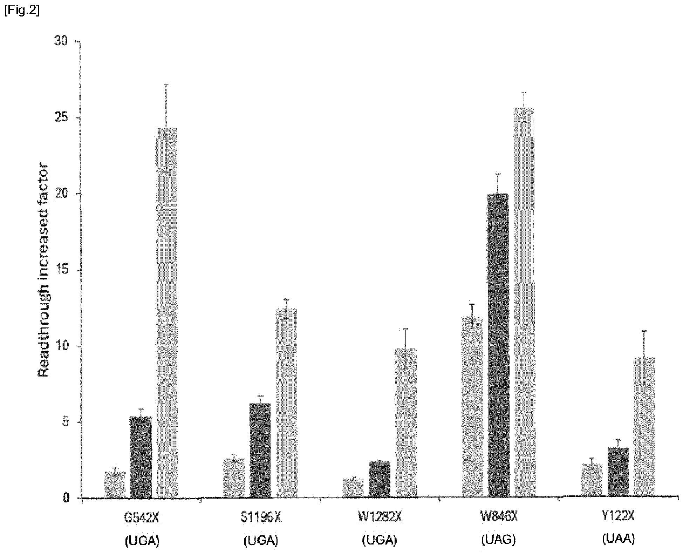

A stop codon readthrough assay for 5 premature stop codon found in CFTR was performed with compound 613, 829 and ELX-02 as a reference compound, according to the protocol described in Bidou L, Bugaud O, Merer G, Coupet M, Hatin I, Chirkin E, Karri S, Demais S, Frangois P, Cintrat JC, Namy O. 2-Guanidino-quinazoline promotes the readthrough of nonsense mutations underlying human genetic diseases. Proc Natl Acad Sci U S A. 2022 Aug 30;119(35):e2122004119. doi: 10.1073/pnas.2122004119. Epub 2022 Aug 22. PMID: 35994666; PMCID: PMC9436315. The results are presented in Fig. 2. Each value is the median of at least four independent assays. For each stop codon, the left bar represents the reference compound ELX-02, the middle bar represents compound 613, and the right bar represents compound 829. The increase factor is calculated in comparison to untreated cells set to 1. The results show that the compounds according to the disclosure have improved readthrough activites.

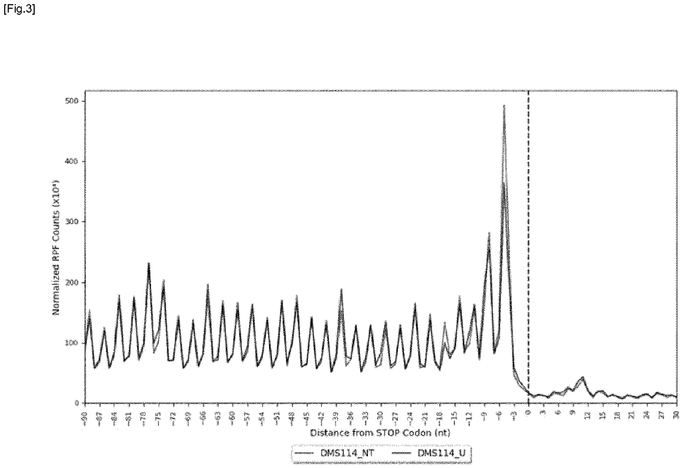

A ribosome profiling study was performed in HeLa cells treated for 24 h with 50 µm 829 (grey), DMSO (black) as a negative control., according to the protocol described in Bidou L, Bugaud O, Merer G, Coupet M, Hatin I, Chirkin E, Karri S, Demais S, Frangois P, Cintrat JC, Namy O. 2-Guanidino-quinazoline promotes the readthrough of nonsense mutations underlying human genetic diseases. Proc Natl Acad Sci U S A. 2022 Aug 30;119(35):e2122004119. doi: 10.1073/pnas.2122004119. Epub 2022 Aug 22. PMID: 35994666; PMCID: PMC9436315. The results are presented in Fig. 3. Each individual transcript was aligned from the stop codon, and normalized ribosome protected fragments (RPFs) are indicated by the first nucleotide at the A-site. The dashed line indicates the first position of the stop codon. Figure 3 shows that no readthrough of the natural stop codons induced by 829 can be observed (on the right of the dashed line).

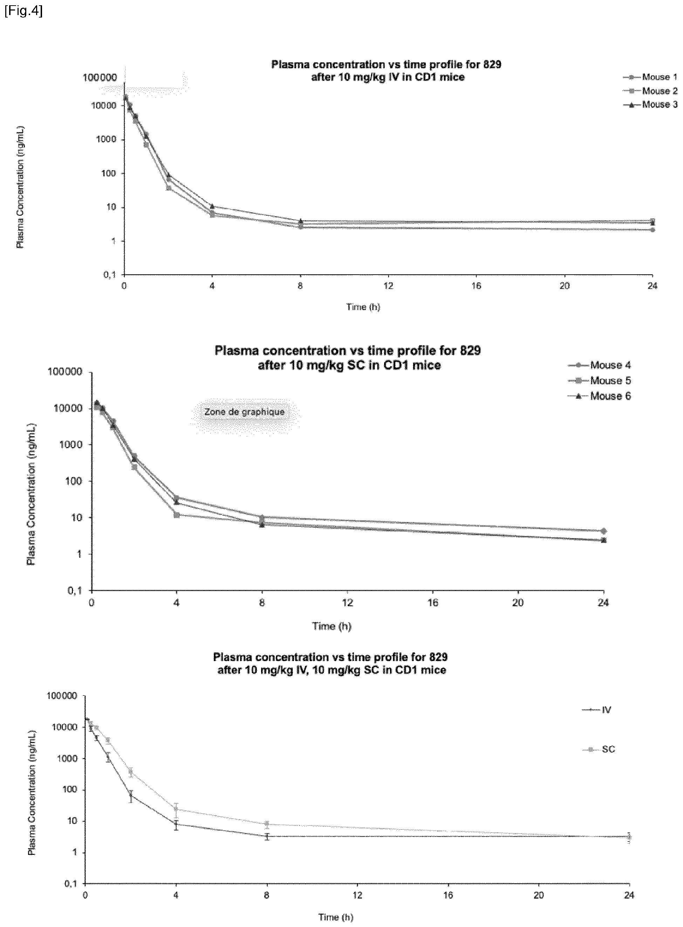

Pharmacokinetics studies were performed on compound 829 following a single intravenous (IV) or subcutaneous (SC) administration to CD1 mice (standard protocol). The results are presented in Figure 4. T_{1/2} (SC) = 7.8 ± 1.1 h, T_{1/2} (IV) = 4.2 ± 0.7 h. In comparison, ELX-02 has a T_{1/2} (SC) = 4.1 ± 0.8 h.

The results show that compounds according to the disclosure comprising a piperidine ring have improved readthrough activities.

Moreover, several compounds were tested and showed a low toxicity.

## Claims

1. A compound of formula (I): 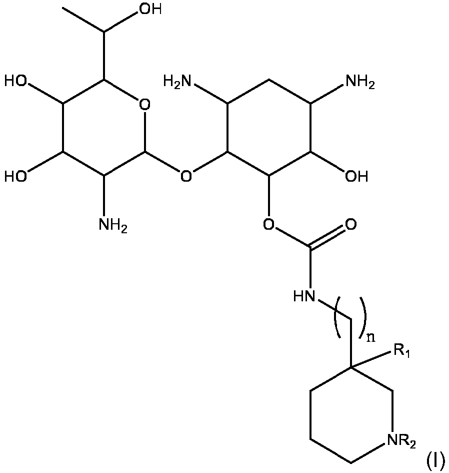 Wherein
R1 is H or an alkyl
R2 is selected from alkyl, heteroalkyl, cycloalkyl, heterocyclyl, aryl, heteroaryl and any combinations thereof, n is an integer from 1 to 3, preferably n is 1 or 2
R', R" and R‴ are independently selected from H and alkyl,
and pharmaceutically acceptable salts thereof.

2. Compound according to claim 1, wherein R2 is -R4-NH-R5,
R4 is an alkylene, and
R5 is selected from H, alkyl, heteroalkyl, cycloalkyl, heterocyclyl, aryl, heteroaryl and any combinations thereof.

3. Compound according to claim 1 or 2, wherein the compound is of formula (II) 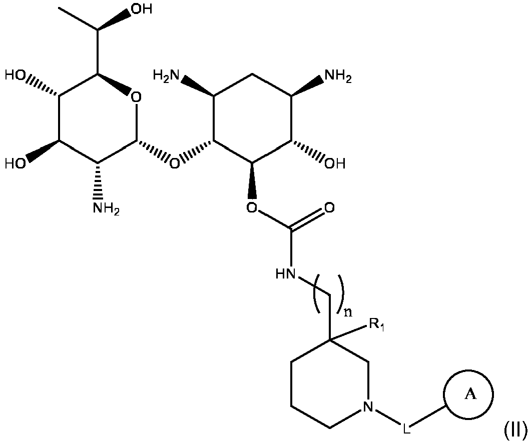 Wherein
L is a bond or an alkylene,
R1 is H or CH₃,
Ring A is selected from cycloalkyl, and heterocyclyl.

4. Compound according to any claim 3, wherein the compound is of formula (III)

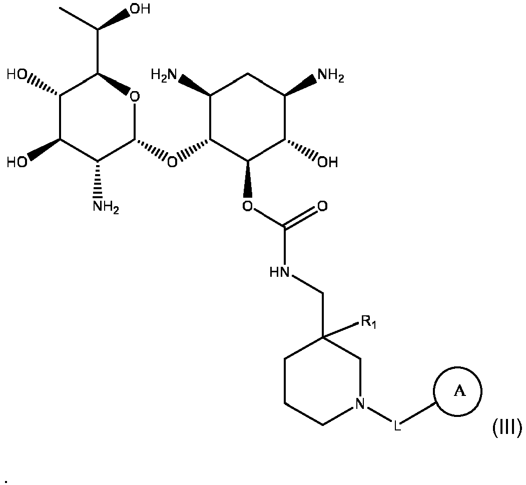

5. Compound according to claim 1, wherein the compound is selected from 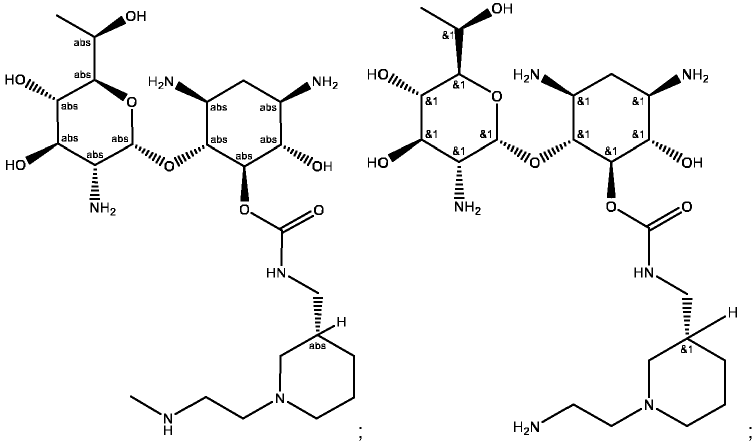 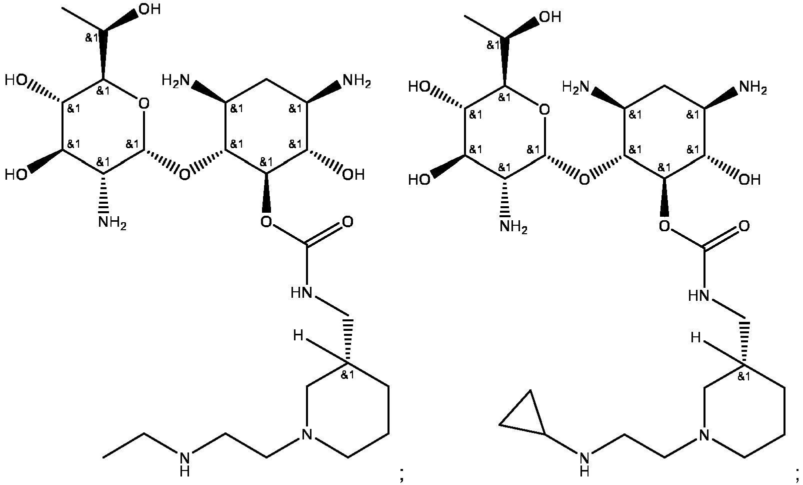 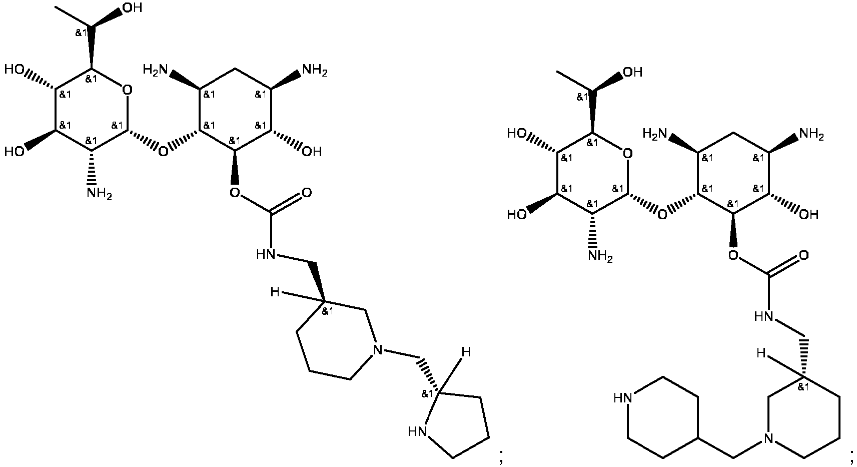 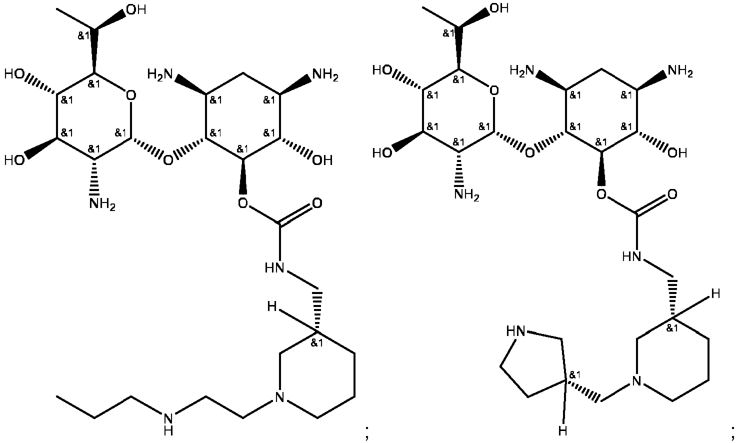 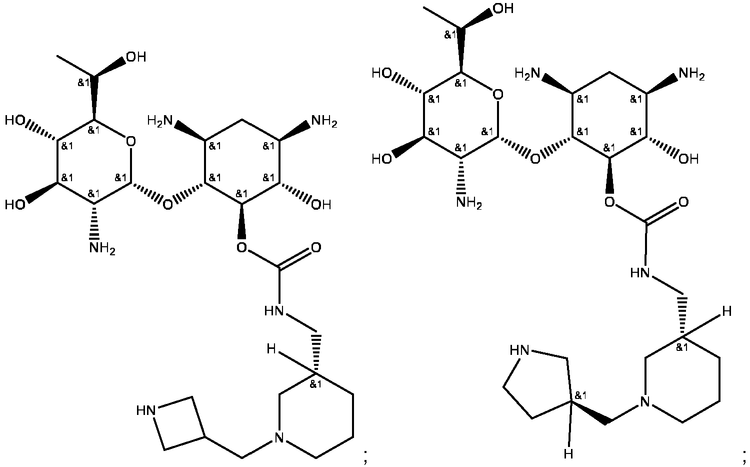 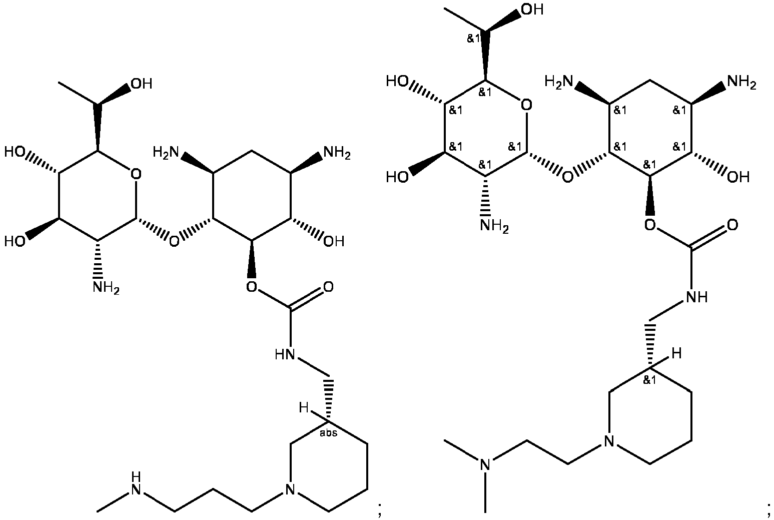 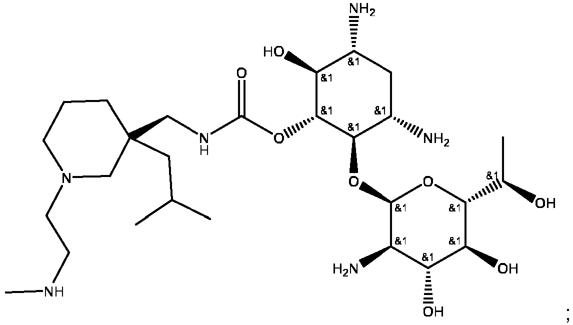 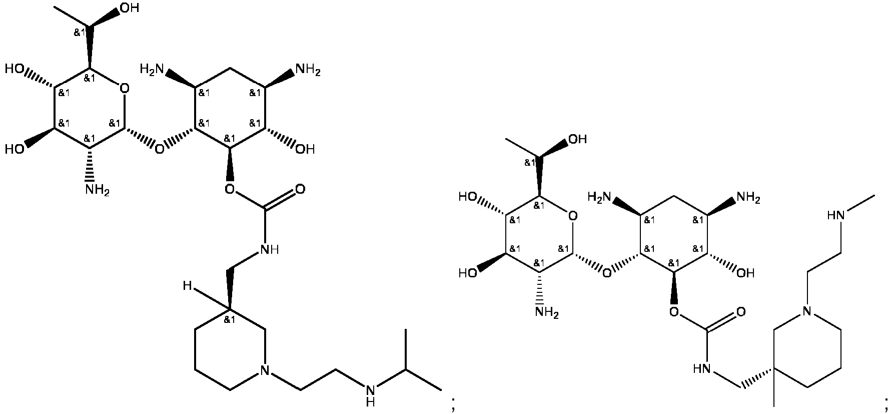 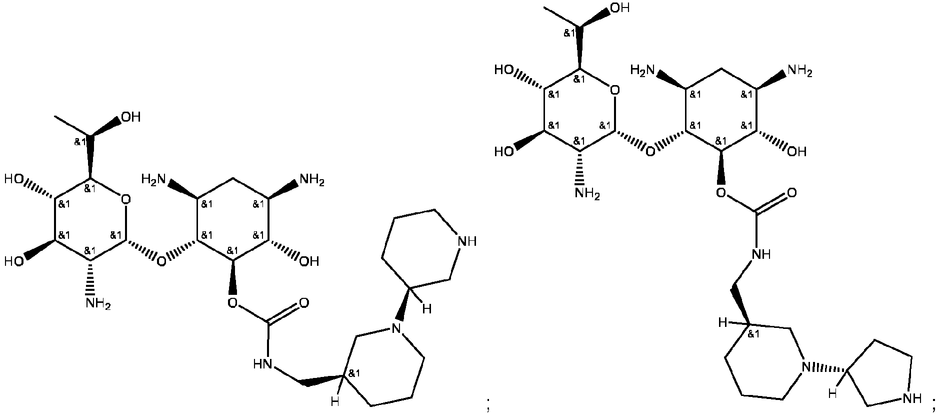 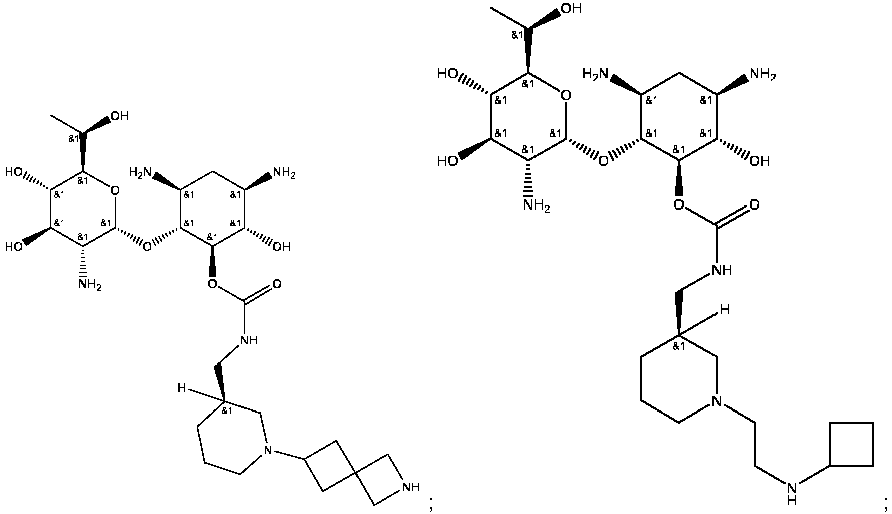 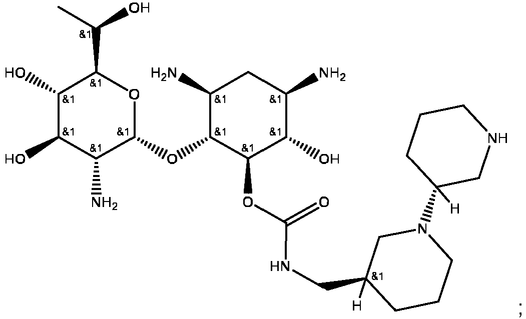 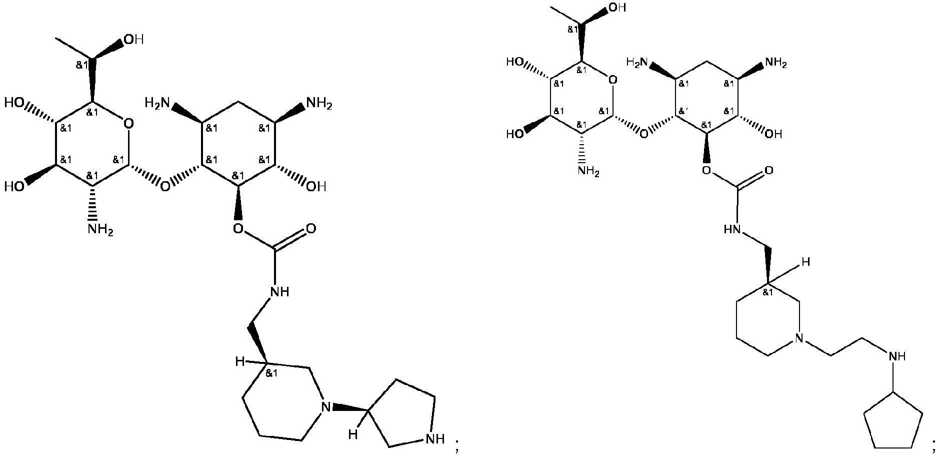 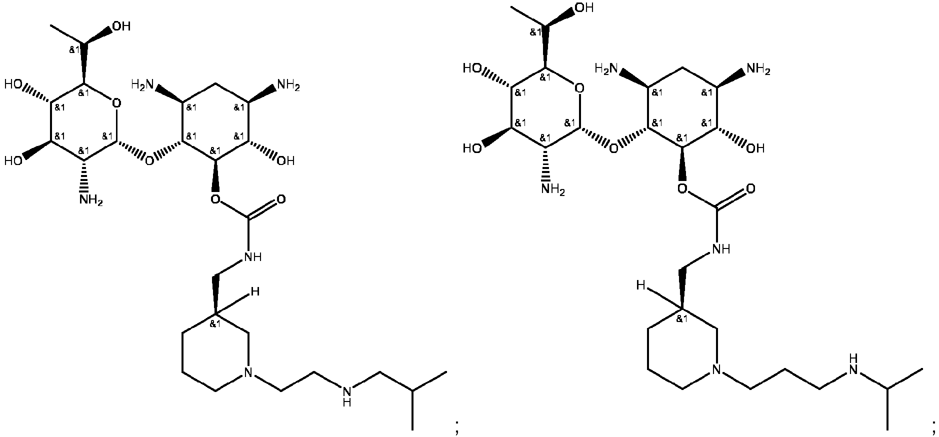 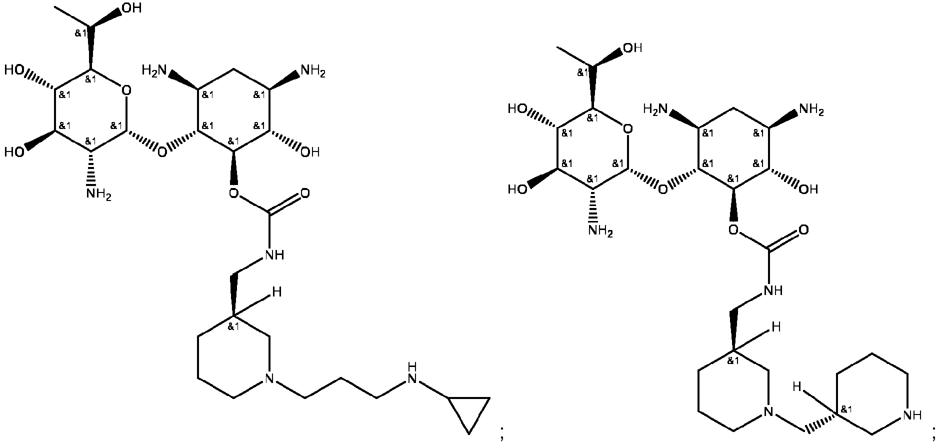 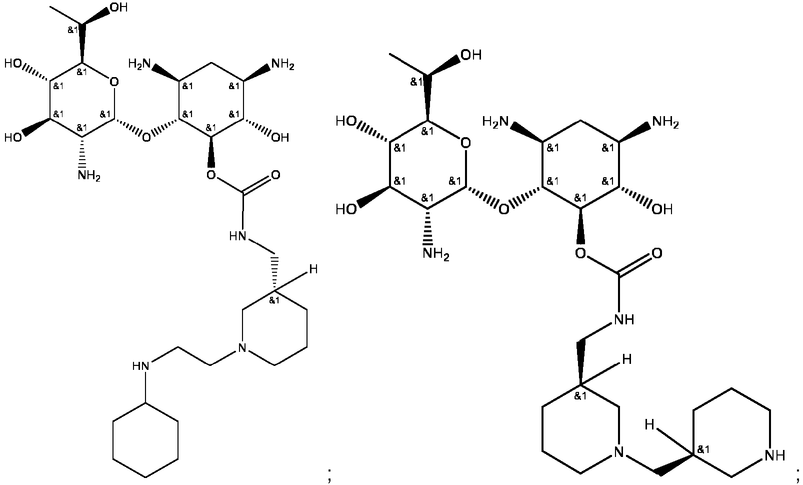 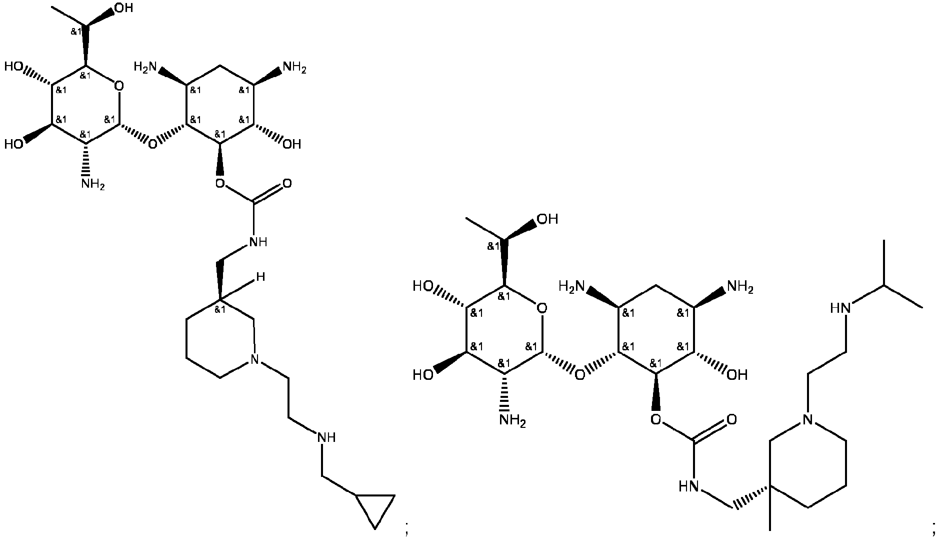 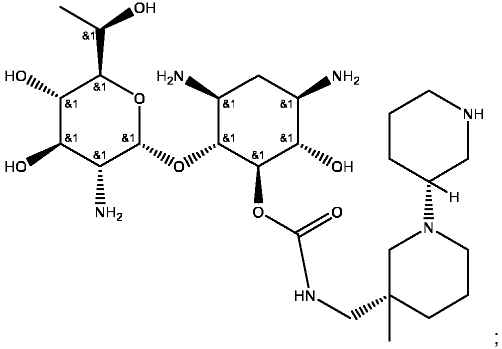 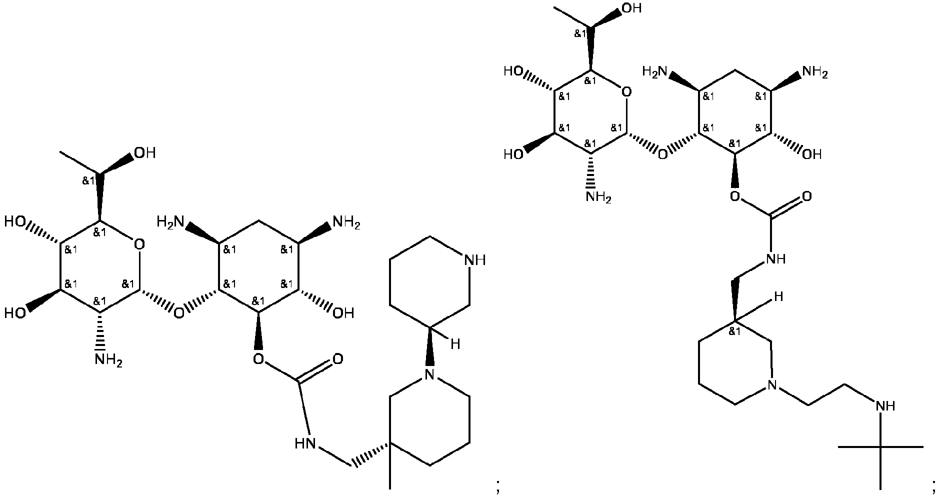 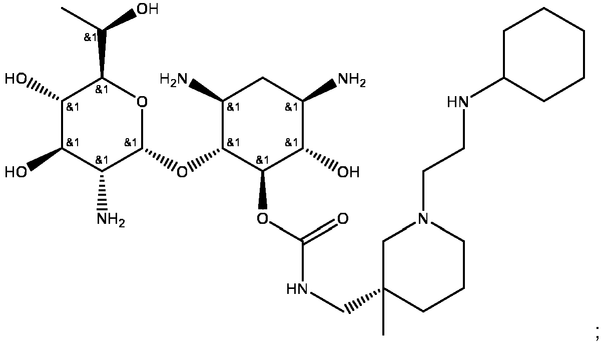 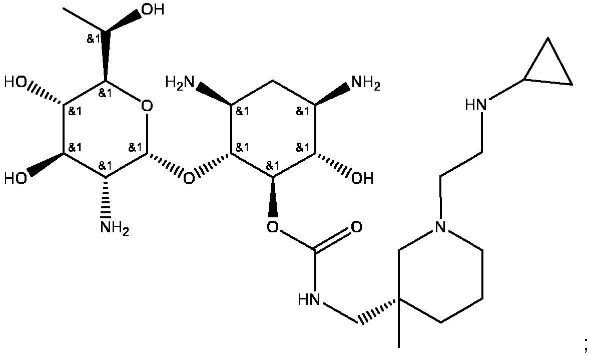 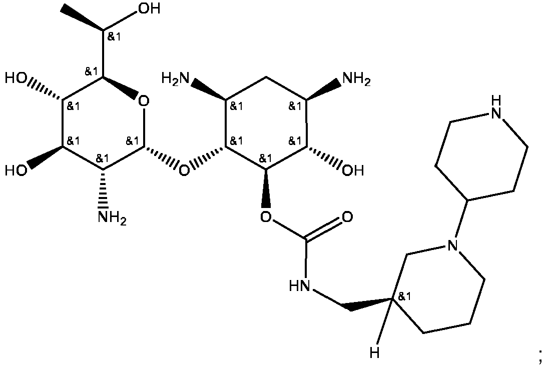 and

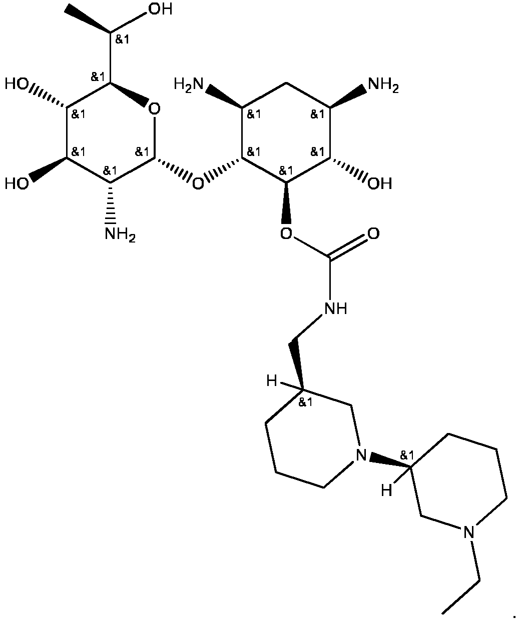

6. Compound according to claim 1, wherein the compound is

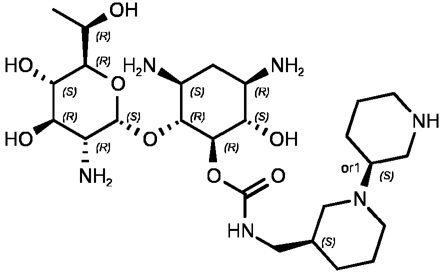

7. Pharmaceutical composition comprising a compound according to any of claims 1 to 6 and a pharmaceutically acceptable carrier.

8. Compound according to any of claims 1 to 6, for use as a drug.

9. Compound according to claim 8, for use in the treatment of genetic disorders.

10. Compound for use according to claim 9, wherein the genetic disorder is associated with a premature stop codon mutation and/or protein truncation phenotype.

11. Compound for use according to claim 9, wherein the genetic disorder is selected from the group consisting of monogenic diseases including among many others cystic fibrosis (CF), Duchenne muscular dystrophy (DMD), Becker muscular dystrophy (BMD), Congenital muscular dystrophy (CMD), Louis-Bar syndrome / ataxia-telangiectasia (AT), mucopolysaccharidoses such as Hurler syndrome, hemophilia A, hemophilia B, Usher syndrome, Tay-Sachs, Neurofibromatosis type 1 (NF1), Factor VII deficiency, Familial atrial fibrillation, McArdle disease, Nephropathic cystinosis, Polycystic kidney disease, Alport syndrome, Rett syndrome, Spinal muscular atrophy (SMA), various forms of epidermolysis bullosa, Hailey-Hailey disease, Dravet syndrome, X-linked nephrogenic diabetes insipidus (XNDI), X-linked retinitis pigmentosa and cancers caused by a nonsense mutation affecting a tumor suppressor gene such as *TP53, APC, PTEN* or *BRCA2.*
